# EUROPEAN PATENT SPECIFICATION

(11) **EP 3 429 526 B1**
(45) Date of publication and mention of the grant of the patent: **18.12.2019**
(21) Application number: 17714096.9
(22) Date of filing: 15.03.2017
(51) Int. Cl.: A61F 13/15

(54) **METHOD AND APPARATUS FOR MANUFACTURING AN ABSORBENT ARTICLE INCLUDING AN ULTRA SHORT PULSE LASER SOURCE**
VERFAHREN UND VORRICHTUNG ZUR HERSTELLUNG EINES SAUGFÄHIGEN ARTIKELS MIT EINER ULTRAKURZIMPULS-LASERQUELLE
PROCÉDÉ ET APPAREIL DE FABRICATION D'UN ARTICLE ABSORBANT COMPRENANT UNE SOURCE LASER À IMPULSIONS ULTRACOURTES

(30) Priority: 15.03.2016 US 201662308275 P
(43) Date of publication of application: 23.01.2019
(73) Proprietor: The Procter & Gamble Company, Cincinnati, OH 45202 (US)
(72) Inventor: EIMANN, Klaus, 97828 Marktheidenfeld (DE); SCHNEIDER, Uwe, Cincinnati Ohio 45202 (US); WALSH, Bradley, Edward, Cincinnati Ohio 45202 (US)
(74) Representative: P&G Patent Germany
(86) International application number: PCT/US2017/022392
(87) International publication number: WO 2017/160900

(56) References cited:
- WO-A1-2015/064486
- JP-A- 2015 009 504
- US-A- 5 017 423
- US-A- 5 817 271
- US-A1- 2014 110 037

## Description

### FIELD

The present disclosure relates to apparatuses and methods for manufacturing absorbent articles, and more particularly, methods and apparatuses for manufacturing absorbent articles using an ultra short pulse laser source to impart a line of weakness into a substrate.

### BACKGROUND

Along an assembly line, various types of articles, such as for example, diapers and other absorbent articles, may be assembled by adding components to and otherwise modifying an advancing, continuous web of material. For example, in some processes, advancing webs of material are combined with other advancing webs of material. In other examples, individual components created from advancing webs of material are combined with advancing webs of material, which in turn, are then combined with other advancing webs of material. Webs of material and component parts used to manufacture diapers may include: backsheets, topsheet, absorbent cores, front and/or back ears, fastener components, and various types of elastic webs and components such as leg elastics, barrier leg cuff elastics, and waist elastics. Once the desired component parts are assembled, the advancing web(s) and component parts are subjected to a final knife cut to separate the web(s) into discrete diapers or other absorbent articles. The discrete diapers or absorbent articles may also then be folded and packaged.

Various methods and apparatuses may be used for attaching different components to the advancing web and/or otherwise modify the advancing web. For example, some production operations are configured to construct elastic laminates including elastics bonded with the one or more substrates advancing in a machine direction. The operations may be further configured to cut and/or otherwise deactivate discrete lengths of the elastics. In some operations, a substrate, such as an elastic laminate, may advance through a cutting station that cuts the elastic in the advancing laminate. However, some current configurations have certain drawbacks. For example, some present cutting apparatuses may cause unintended damage to the elastic laminate, such as by severing the substrate while cutting the elastic. In addition, the blades on some current cutting apparatuses may be susceptible to wear after relatively short operating periods. Such blade wear may manifest itself in inconsistent elastic cutting. Further, a blade may be re-sharpened only a certain number of times before the cutting device, as a whole, needs to be replaced, and there are relatively high costs associated with maintaining worn cutting devices and ultimately replacing the cutting device. Further, a blade is manufactured with a certain shape and that shape remains unchangeable. For products which different shaped cut edges or different sized cut edges, multiple blades would need to be produced. Thus, it may be relatively expensive to maintain and replace cutting devices.

Due to the types of lasers commercially available that can operate at the relatively high speeds necessary to manufacture products commercially, the cut edge of the substrate has been relatively unacceptable to consumers. For example, the laser cut produces a relatively rough cut edge, and the substrate itself may develop a smell due to the residue and heat generated by the laser beam as it cuts the substrate. Thus, due to the relatively poor quality edge of the substrate produced, lasers have been largely unusable in relatively high speed manufacturing processes for consumer products.

Consequently, it would be beneficial to provide methods and apparatuses that are configured to produce a consumer acceptable substrate edge and that are configured to consistently and accurately remove trim from the advancing substrates. It would also be beneficial to provide methods and apparatuses that are not susceptible to blade wear and can be readily adapted for different configurations of edges.

### SUMMARY

The present disclosure relates to methods and apparatuses for assembling absorbent articles, and more particularly, methods and apparatuses for using an ultra short pulse laser source impart one or more lines of weakness into a substrate. In some embodiments, a method for separating a component of an absorbent article includes: advancing a substrate in a machine direction; providing an ultra-short pulse laser source; emitting a laser beam with the ultra-short pulse laser source; directing the laser beam at a portion of the substrate; imparting a line of weakness into a portion of the substrate using the laser beam, wherein the laser beam is pulsed at a frequency from about 100 kHz to about 100 MHz, wherein the laser beam has a pulse duration from about 5 femtoseconds to about 10 picoseconds, and wherein the laser beam has a level of peak energy from about 20 µJ to 875 µJ; forming a heat modified zone along the line of weakness; and separating the substrate along the line of weakness to form a separation edge; wherein the heat modified zone comprises a maximum width that is less than about 200 microns, wherein the maximum width is measured from the separation edge in a direction perpendicular to the separation edge toward a central region of the substrate.

In some embodiments, a method for separating a component of an absorbent article includes: advancing a nonwoven substrate in a machine direction; providing an ultra-short pulse laser source; emitting a laser beam with the ultra-short pulse laser source; directing the laser beam at a portion of the nonwoven substrate; imparting a line of weakness into a portion of the nonwoven substrate using the laser beam, wherein the laser beam is pulsed at a frequency from about 100 kHz to about 100 MHz, wherein the laser beam has a pulse duration from about 5 femtoseconds to about 10 picoseconds, and wherein the laser beam has a level of peak energy from about 20 µJ to 875 µJ; forming a heat modified zone along the line of weakness; and separating the substrate along the line of weakness to form a separation edge.

In some embodiments, a method for separating a component of an absorbent article includes: advancing a film substrate in a machine direction; providing an ultra-short pulse laser source; emitting a laser beam with the ultra-short pulse laser source; directing the laser beam at a portion of the film substrate; imparting a line of weakness into the film substrate using the laser beam, wherein the laser beam is pulsed at a frequency from about 100 kHz to about 100 MHz, wherein the laser beam has a pulse duration from about 5 femtoseconds to about 10 picoseconds, and wherein the laser beam has a level of peak energy from about 20 µJ to 875 µJ; forming a heat modified zone along the line of weakness; and separating the film along the line of weakness to form a separation edge.

In some embodiments, a method for separating a component of an absorbent article includes: advancing a nonwoven substrate in a machine direction, wherein the nonwoven substrate has a compressed caliper; providing an ultra-short pulse laser; emitting a laser beam with the ultra-short pulse laser; directing the laser beam at a portion of the nonwoven substrate; imparting a line of weakness into the nonwoven substrate using the ultra-short pulse laser, wherein the ultra-short pulse laser is pulsed at a frequency from about 100 kHz to about 100 MHz, wherein the ultra-short pulse laser comprises a pulse duration from about 5 femtoseconds to 10 picoseconds, and wherein the ultra-short pulse laser comprises a level of peak energy of from about 20 µJ to about 875 µJ; forming a heat modified zone along the line of weakness; and separating the nonwoven substrate along the line of weakness, wherein the heat modified zone comprises a cluster of laser affected fibers, wherein the cluster of laser affected fibers comprise a maximum linear length, and wherein the maximum linear length is less than 200 µm.

In some embodiments, a method for separating a component of an absorbent article includes: transferring a substrate onto an outer circumferential surface of a process member, wherein the substrate comprises one or more fibers, wherein each fibers includes a fiber diameter; rotating the process member about its longitudinal axis of rotation; advancing the substrate to an ultra-short pulse laser; imparting a line of weakness into the substrate using the ultra-short pulse laser, wherein the ultra-short pulse laser is pulsed at a frequency from about 100 kHz to about 100 MHz, wherein the ultra-short pulse laser comprises a pulse duration from about 5 femtoseconds to about 10 picoseconds, and wherein the ultra-short pulse laser comprises a level of peak energy of from about 20 µJ to about 875 µJ; forming a heat modified zone along the line of weakness; and separating the substrate along the line of weakness to form a separation edge, wherein the heat modified zone comprises one or more accumulation bulbs, wherein each of the one or more accumulation bulbs have an accumulation bulb diameter, and wherein the heat modified zone comprises less than three clusters.

In some embodiments, a method for separating a component of an absorbent article includes: advancing a substrate around a portion of a first guide roller, wherein the substrate comprises a first substrate layer and a second substrate layer, wherein the substrate has a first surface and a second surface; advancing the substrate around a portion of a second guide roller, wherein an unsupported portion of the substrate is suspended between the first guide roller and the second guide roller; directing a laser beam emitted by an ultra short pulse laser source at the first surface of the substrate, wherein the laser beam acts on the unsupported portion of the substrate; imparting a line of weakness into the substrate, wherein the laser beam is pulsed at a frequency from about 100 kHz to about 100 MHz, wherein the laser beam has a pulse duration from about 5 femtoseconds to about 10 picoseconds, and wherein the laser beam has a level of peak energy from about 20 µJ to 875 µJ; forming a heat modified zone along the line of weakness; and separating the substrate along the line of weakness to form a separation edge.

In some embodiments, a method for separating a component of an absorbent article includes: advancing a substrate around a portion of a first guide roller, wherein the substrate comprises a first substrate layer and a second substrate layer, wherein the substrate has a first surface and a second surface; advancing the substrate around a portion of a second guide roller, wherein an unsupported portion of the substrate is suspended between the first guide roller and the second guide roller; directing a first laser beam emitted by a first ultra short pulse laser source at the first surface of the substrate, wherein the first laser beam acts on the unsupported portion of the substrate; directing a second laser beam emitted by a second ultra short pulse laser source at the second surface of the substrate, wherein the second laser beam acts on the unsupported portion of the substrate; imparting a line of weakness into the substrate, wherein the first laser beam and the second laser beam are pulsed at a frequency from about 100 kHz to about 100 MHz, wherein the first and second laser beams have a pulse duration from about 5 femtoseconds to about 10 picoseconds, and wherein the first and second laser beams have a level of peak energy from about 20 µJ to 875 µJ; forming a heat modified zone along the line of weakness; and separating the substrate along the line of weakness to form a separation edge.

In some embodiments, a consumer product includes a nonwoven substrate. The nonwoven substrate may include a first nonwoven layer and a second nonwoven layer in a facing relationship. The nonwoven substrate may also include a separation edge and a heat modified zone. The heat modified zone has a maximum width that is less than about 200 microns. The width is measured from the separation edge in a direction perpendicular to the separation edge toward a central region of the nonwoven substrate.

In some embodiments, a consumer product includes a nonwoven substrate. The nonwoven substrate may include a first nonwoven layer and a second nonwoven layer in a facing relationship. The nonwoven substrate also includes a separation edge. The separation edge has a Free Fiber End value greater than 1.

In some embodiments, a consumer product includes a nonwoven substrate. The nonwoven substrate may include a first nonwoven layer and a second nonwoven layer in a facing relationship. The nonwoven substrate may also include a separation edge and a heat modified zone. The heat modified zone includes less than three clusters per centimeter and one or more accumulation bulbs.

In some embodiments, a consumer product includes a nonwoven substrate. The nonwoven substrate may include a first nonwoven layer and a second nonwoven layer in a facing relationship. The nonwoven substrate may also include a separation edge and a heat modified zone. The heat modified zone includes a cluster and an accumulation bulb, and the cluster has a maximum linear length less than about 200 µm.

In some embodiments, a consumer product includes a film substrate. The film substrate may include a separation edge and a heat modified zone. The film substrate comprises an edge length and a linear length, wherein the ratio of the edge length to linear length is less than 1.

In some embodiments, a consumer product includes a substrate. The substrate includes a first nonwoven layer and a film layer in facing relationship. The substrate includes a separation edge and a heat modified zone. The heat modified zone comprises a width that is less than about 200 microns. The width is measured from the separation edge in a direction perpendicular to the separation edge toward a central region of the nonwoven substrate.

### BRIEF DESCRIPTION OF THE DRAWINGS

Figure 1 is a perspective view of a diaper pant;
Figure 2 is a partially cut away plan view of the diaper pant shown in Figure 1;
Figure 3A is a cross-sectional view of the diaper pant of Figure 2 taken along line 3A-3A;
Figure 3B is a cross-sectional view of the diaper pant of Figure 2 taken along line 3B-3B;
Figure 4 is a partially cut away plan view of an absorbent article;
Figure 5 is a partially cut away plan view of an absorbent article;
Figure 6 is a partially cut away plan view of an absorbent article;
Figure 7 is an energy v. time graph for an ultra short pulse laser;
Figure 8 is an energy v. time graph for a CO₂ laser;
Figure 9A is a photograph of a portion of an edge of a substrate formed, at least in part, by a CO₂ laser source;
Figure 9B is the photograph of Figure 9A including dimensions;
Figure 9C is a photograph of a portion of an edge of a substrate formed, at least in part, by a CO₂ laser source;
Figure 9D is the photograph of Figure 9C including dimensions;
Figure 9E is a photograph of a portion of an edge of a substrate formed, at least in part, by a CO₂ laser source;
Figure 9F is a portion of the photograph of Figure 9E;
Figure 9G is the photograph of Figure 9F including dimensions;
Figure 9H is a photograph of a portion of an edge of a substrate formed, at least in part, by a CO₂ laser source;
Figure 9I is a portion of the photograph of Figure 9I;
Figure 9J is the photograph of Figure 9I including dimensions;
Figure 10A is a photograph of a portion of an edge of a substrate formed by an ultra short pulse laser source;
Figure 10B is a portion of the photograph of Figure 10B;
Figure 10C is the photograph of Figure 10B including dimensions;
Figure 10D is a photograph of a portion of an edge of a substrate formed by an ultra short pulse laser source;
Figure 10E is a portion of the photograph of Figure 10D;
Figure 10F is the photograph of Figure 10F including dimensions;
Figure 10G is a photograph of a portion of an edge of a substrate formed by an ultra short pulse laser source;
Figure 10H is a portion of the photograph of Figure 10G;
Figure 10I is the photograph of Figure 10G including dimensions;
Figure 10J is a photograph of a portion of a substrate including a line of weakness formed by an ultra short pulse laser source;
Figure 10K is a portion of the photograph of Figure 10J including dimensions;
Figure 10L is a portion of the photograph of Figure 10J including dimensions;
Figure 10M is a photograph of a portion of a substrate including a line of weakness formed by an ultra short pulse laser source;
Figure 10N is a portion of the photograph of Figure 10M including dimensions;
Figure 11 is a schematic representation of an apparatus that imparts a separation edge into a substrate in accordance with one non-limiting embodiment of the present disclosure;
Figure 12 is a schematic representation of an apparatus that imparts a separation edge into a substrate in accordance with one non-limiting embodiment of the present disclosure;
Figure 13 is a schematic representation of an apparatus that imparts a separation edge into a substrate in accordance with one non-limiting embodiment of the present disclosure;
Figure 14A is a schematic representation of an apparatus that imparts a separation edge into a substrate in accordance with one non-limiting embodiment of the present disclosure;
Figure 14B is a schematic representation of an apparatus that imparts a separation edge into a substrate in accordance with one non-limiting embodiment of the present disclosure;
Figure 15A is a schematic representation of an apparatus that imparts a separation edge into a substrate in accordance with one non-limiting embodiment of the present disclosure;
Figure 15B is a schematic representation of an apparatus that imparts a separation edge into a substrate in accordance with one non-limiting embodiment of the present disclosure;
Figure 16A is a schematic representation of an apparatus that imparts a separation edge into a substrate in accordance with one non-limiting embodiment of the present disclosure;
Figure 16B is a schematic representation of an apparatus that imparts a separation edge into a substrate in accordance with one non-limiting embodiment of the present disclosure;
Figure 17A is a perspective view of a substrate including a first layer and a second layer in accordance with one non-limiting embodiment of the present disclosure;
Figure 17B is a perspective view of a substrate including a first layer, a second layer, and a third layer in accordance with one non-limiting embodiment of the present disclosure;
Figure 18A is an end view of a laser beam acting on a substrate in accordance with one non-limiting embodiment of the present disclosure;
Figure 18B is an end view of a first laser beam and a second laser beam acting on a substrate in accordance with one non-limiting embodiment of the present disclosure;
Figure 18C is a perspective view of a substrate including a line of weakness and a separation edge in accordance with one non-limiting embodiment of the present disclosure;
Figure 19A is a top view of a belt assembly in accordance with one non-limiting embodiment of the present disclosure;
Figure 19B is a top view of a belt assembly in accordance with one non-limiting embodiment of the present disclosure;
Figure 20A is an end view of an outer circumferential surface of a roller or a process member in accordance with one non-limiting embodiment of the present disclosure;
Figure 20B is a partial end view of a substrate disposed on outer circumferential surface of a roller or a process member in accordance with one non-limiting embodiment of the present disclosure;
Figure 21A is a top view of a belt assembly including a discrete line of weakness in accordance with one non-limiting embodiment of the present disclosure;
Figure 21B is a top view of a belt assembly including a continuous line of weakness in accordance with one non-limiting embodiment of the present disclosure;
Figure 21C is an end view of a belt assembly traversing by a first and second laser source in accordance with one non-limiting embodiment of the present disclosure;
Figure 21D is an end view of a belt assembly traversing by a first, second, third, and fourth laser source in accordance with one non-limiting embodiment of the present disclosure;
Figure 22A is an end view of a belt assembly traversing by a first, second, third, and fourth laser source in accordance with one non-limiting embodiment of the present disclosure;
Figure 22B is a schematic representation of an end view of a mask positioned between a laser source and a portion of the belt assembly in accordance with one non-limiting embodiment of the present disclosure;
Figure 22C is a top view of a belt assembly including a discrete line of weakness and a gap in accordance with one non-limiting embodiment of the present disclosure;
Figure 23 is a schematic representation of an apparatus that imparts a line of weakness into a substrate in accordance with one non-limiting embodiment of the present disclosure;
Figure 24A is a top view of a belt assembly including a discrete separation edge in accordance with one non-limiting embodiment of the present disclosure;
Figure 24B is a top view of a belt assembly including a continuous separation edge in accordance with one non-limiting embodiment of the present disclosure;
Figure 25A is a top view of a discrete component in a first orientation in accordance with one non-limiting embodiment of the present disclosure;
Figure 25B is a top view of a discrete component in a second orientation in accordance with one non-limiting embodiment of the present disclosure;
Figure 25C is a top view of a belt assembly including a discrete component in accordance with one non-limiting embodiment of the present disclosure;
Figure 26 is a schematic representation of an apparatus that imparts a line of weakness into a substrate in accordance with one non-limiting embodiment of the present disclosure;
Figure 27 is a schematic representation of an apparatus that imparts a line of weakness into a substrate in accordance with one non-limiting embodiment of the present disclosure; and
Figure 28 is a schematic representation of an apparatus that imparts a line of weakness into a substrate in accordance with one non-limiting embodiment of the present disclosure.

### DETAILED DESCRIPTION

The following term explanations may be useful in understanding the present disclosure.

"Absorbent article" is used herein to refer to consumer products whose primary function is to absorb and retain soils and wastes. "Diaper" is used herein to refer to an absorbent article generally worn by infants and incontinent persons about the lower torso. The term "disposable" is used herein to describe absorbent articles which generally are not intended to be laundered or otherwise restored or reused as an absorbent article (e.g., they are intended to be discarded after an initial use and may also be configured to be recycled, composted or otherwise disposed of in an environmentally compatible manner).

An "elastic," "elastomer" or "elastomeric" refers to materials exhibiting elastic properties, which include any material that upon application of a force to its relaxed, initial length can stretch or elongate to an elongated length more than 10% greater than its initial length and will substantially recover back to about its initial length upon release of the applied force.

The term "extensible" as used herein refers to any material that upon application of a biasing force, can stretch to an elongated length of at least about 110% of its relaxed, original length (i.e. can stretch to 10%), without rupture or breakage, and upon release of the applied force, shows little recovery, less than about 40% of its elongation.

The terms "activating", "activation" or "mechanical activation" refer to the process of making a substrate, or an elastomeric laminate more extensible than it was prior to the process.

"Live stretch" includes stretching elastic and bonding the stretched elastic to a substrate. After bonding, the stretched elastic is released causing it to contract, resulting in a "corrugated" substrate. The corrugated substrate can stretch as the corrugated portion is pulled to about the point that the substrate reaches at least one original flat dimension. However, if the substrate is also elastic, then the substrate can stretch beyond the relaxed length of the substrate prior to bonding with the elastic. The elastic is stretched at least 25% of its relaxed length when it is bonded to the substrate.

As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.

"Longitudinal" means a direction running substantially perpendicular from a waist edge to a longitudinally opposing waist edge of an absorbent article when the article is in a flat out, uncontracted state, or from a waist edge to the bottom of the crotch, i.e. the fold line, in a bifolded article. Directions within 45 degrees of the longitudinal direction are considered to be "longitudinal." "Lateral" refers to a direction running from a longitudinally extending side edge to a laterally opposing longitudinally extending side edge of an article and generally at a right angle to the longitudinal direction. Directions within 45 degrees of the lateral direction are considered to be "lateral."

The term "substrate" is used herein to describe a material which is primarily two-dimensional (i.e. in an XY plane) and whose thickness (in a Z direction) is relatively small (i.e. 1/10 or less) in comparison to its length (in an X direction) and width (in a Y direction). Non-limiting examples of substrates include a web, layer or layers or fibrous materials, nonwovens, films, and foils such as polymeric films or metallic foils. These materials may be used alone or may comprise two or more layers laminated together. As such, a web is a substrate.

The term "nonwoven" means a porous, fibrous material made from continuous (long) filaments (fibers) and/or discontinuous (short) filaments (fibers) by processes such as, for example, spunbonding, meltblowing, airlaying, carding, coforming, hydroentangling, and the like. These processes may also be combined. Filaments may have aspect ratio (length to diameter) greater than about 10. Filaments can be mono-component or multi-components. Filaments can be laid and bonded by any means to form web except weaving and knitting. Nonwovens do not have a woven or knitted filament pattern. Nonwovens may be liquid permeable or impermeable.

The term "film" means a sheet-like material wherein the length and width of the material far exceed the thickness of the material (e.g., 10x, 50x, or even 1000x or more). Films are typically liquid impermeable but may be configured to be breathable. Typically, films have a thickness of about 0.5 mm or less. Films may be formed by any suitable method in the art, for example, by extruding molten thermoplastic and/or elastomeric polymers through a slit die and subsequently cooling the extruded sheet. Other non-limiting examples for making film forms include casting, blowing, solution casting, calendering, and formation from aqueous or, nonaqueous cast dispersions. Films may be a mono-layer film, and/or co-extruded with other materials to form a multi-layer film.

The term "machine direction" (MD) is used herein to refer to the direction of material flow through a process. In addition, relative placement and movement of material can be described as flowing in the machine direction through a process from upstream in the process to downstream in the process.

The term "cross direction" (CD) is used herein to refer to a direction that is generally perpendicular to the machine direction.

The term "pant" (also referred to as "training pant", "pre-closed diaper", "diaper pant", "pant diaper", and "pull-on diaper") refers herein to disposable absorbent articles having a continuous perimeter waist opening and continuous perimeter leg openings designed for infant or adult wearers. A pant can be configured with a continuous or closed waist opening and at least one continuous, closed, leg opening prior to the article being applied to the wearer. A pant can be preformed by various techniques including, but not limited to, joining together portions of the article using any refastenable and/or permanent closure member (e.g., seams, heat bonds, pressure welds, adhesives, cohesive bonds, mechanical fasteners, etc.). A pant can be preformed anywhere along the circumference of the article in the waist region (e.g., side fastened or seamed, front waist fastened or seamed, rear waist fastened or seamed.

"Pre-fastened" refers herein to pant diapers manufactured and provided to consumers in a configuration wherein the front waist region and the back waist region are fastened or connected to each other as packaged, prior to being applied to the wearer. As such pant diapers may have a continuous perimeter waist opening and continuous perimeter leg openings designed for infant or adult wearers. As discussed in more detail below, a diaper pant can be preformed by various techniques including, but not limited to, joining together portions of the diaper using refastenable and/or permanent closure members (e.g., seams, heat bonds, pressure welds, adhesives, cohesive bonds, mechanical fasteners, etc.). In addition, pant diapers can be preformed anywhere along the circumference of the waist region (e.g., side fastened or connected, front waist fastened or connected, rear waist fastened or connected).

The term "taped diaper" refers to disposable absorbent articles having an initial front waist region and an initial rear waist region that are not fastened, pre-fastened, or connected to each other as packaged, prior to being applied to the wearer. A taped diaper may be folded about its lateral central axis with the interior of one waist region in surface to surface contact with the interior of the opposing waist region without fastening or joining the waist regions together. Example taped diapers disclosed in various suitable configurations are disclosed in U.S. Patent Nos. 5,167,897; 5,360,420; 5,599,335; 5,643,588; 5,674,216; 5,702,551; 5,968,025; 6,107,537; 6,118,041; 6,153,209; 6,410,129; 6,426,444; 6,586,652; 6,627,787; 6,617,016; 6,825,393; and 6,861,571.

The present disclosure relates to methods and apparatuses for assembling absorbent articles, and more particularly, methods and apparatuses for using a laser source to impart a line of weakness into one or more portions of a substrate that may be used as a component of an absorbent article.

To help provide additional context to the subsequent discussion of the process embodiments, the following provides a general description of absorbent articles in the form of diapers, feminine hygiene articles, and other consumer products that may be assembled in accordance with the methods and apparatuses disclosed herein. Although the methods and apparatuses herein are discussed below in the context of manufacturing absorbent articles, it is to be appreciated that the assembly methods and apparatuses herein may be configured to manufacture various types of substrates.

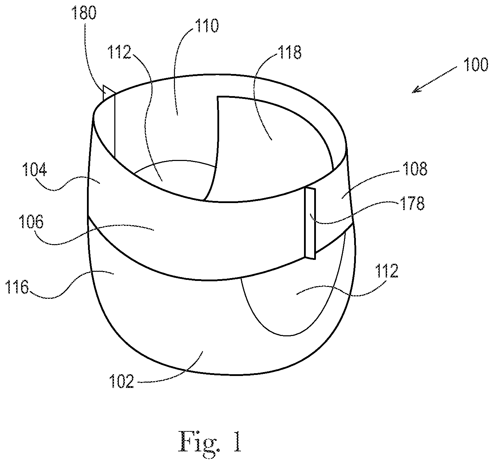

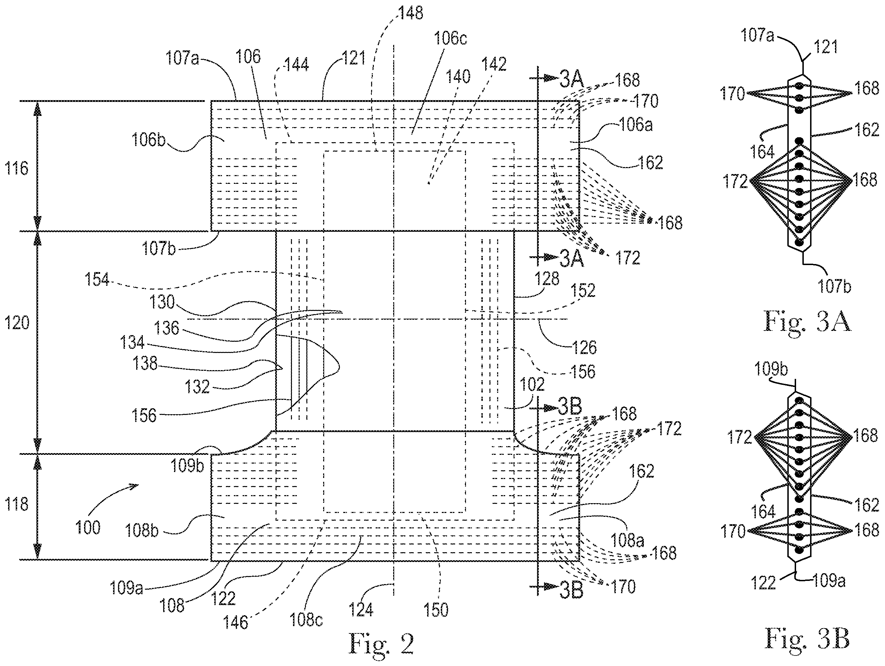

Figures 1, 2, 4, 5 and 6 illustrate an example of an absorbent article 100, such as a diaper, that may be assembled with the methods and apparatuses discussed herein. In particular, Figure 1 shows a perspective view of an absorbent article 100 in a pre-fastened configuration, and Figure 2 shows a plan view of the absorbent article 100 with the portion of the diaper that faces away from a wearer oriented towards the viewer. The absorbent article 100 shown in Figures 1 and 2 includes a chassis 102 and a ring-like elastic belt 104. As discussed below in more detail, a first belt 106 and a second belt 108, which are both elastic, are connected together to form the ring-like elastic belt 104.

With continued reference to Figure 2, the chassis 102 includes a first waist region 116, a second waist region 118, and a crotch region 120 disposed intermediate the first and second waist regions. The first waist region 116 may be configured as a front waist region, and the second waist region 118 may be configured as back waist region. In some embodiments, the length of each of the front waist region, back waist region, and crotch region 120 may be 1/3 of the length of the absorbent article 100. The diaper 100 may also include a laterally extending front waist edge 121 in the front waist region 116 and a longitudinally opposing and laterally extending back waist edge 122 in the back waist region 118. To provide a frame of reference for the present discussion, the absorbent article 100 and chassis 102 is shown with a longitudinal axis 124 and a lateral axis 126. In some embodiments, the longitudinal axis 124 may extend through the front waist edge 121 and through the back waist edge 122. And the lateral axis 126 may extend through a first longitudinal or right side edge 128 and through a midpoint of a second longitudinal or left side edge 130 of the chassis 102.

As shown in Figures 1, 2, 4, 5, and 6 the absorbent article 100 may include an inner, body facing surface 132, and an outer, garment facing surface 134. The chassis 102 may include a backsheet 136 and a topsheet 138. The chassis 102 may also include an absorbent assembly 140 including an absorbent core 142 that may be disposed between a portion of the topsheet 138 and the backsheet 136. As discussed in more detail below, the absorbent article 100 may also include other features, such as a waistband, leg elastics, and/or leg cuffs to enhance the fit around the legs of the wearer.

The periphery of the chassis 102 may be defined by the first longitudinal side edge 128, a second longitudinal side edge 130; a first laterally extending end edge 144 disposed in the first waist region 116; and a second laterally extending end edge 146 disposed in the second waist region 118. Both side edges 128 and 130 extend longitudinally between the first end edge 144 and the second end edge 146. When the absorbent article 100 is worn on the lower torso of a wearer, the front waist edge 121 and the back waist edge 122 of the chassis 102 may encircle a portion of the waist of the wearer. At the same time, the chassis side edges 128 and 130 may encircle at least a portion of the legs of the wearer. Moreover, the crotch region 120 may be generally positioned between the legs of the wearer with the absorbent core 142 extending from the front waist region 116 through the crotch region 120 to the back waist region 118. The chassis 102 may have opposing longitudinal edges that are oriented generally parallel to the longitudinal centerline 124. However, for better fit, longitudinal edges 128, 130 may be curved or angled to produce, for example, an "hourglass" shape diaper when viewed in a plan view, such as disclosed in U.S. Patent No. 8,939,957 and U.S. Patent Publication No. 2012/0277702.

It is also to be appreciated that a portion or the whole of the absorbent article 100 may also be made laterally extensible. The additional extensibility may help allow the absorbent article 100 to conform to the body of a wearer during movement by the wearer. The additional extensibility may also help, for example, allow the diaper 100, including a chassis 102 having a particular size before extension, to extend in the front waist region 116, the back waist region 118, or both waist regions of the diaper 100 and/or chassis 102 to provide additional body coverage for wearers of differing size, i.e., to tailor the diaper to an individual wearer. Such extension of the waist region or regions may give the absorbent article a generally hourglass shape, so long as the crotch region is extended to a relatively lesser degree than the waist region or regions, and may impart a tailored appearance to the article when it is worn.

As previously mentioned, the diaper 100 may include a backsheet 136. The backsheet 136 may also define the outer surface 134 of the chassis 102. The backsheet 136 may be impervious or at least partially impervious to fluids (e.g., menses, urine, and/or runny feces) and may be manufactured from a thin plastic film, although other flexible liquid impervious materials may also be used. The backsheet 136 may prevent the exudates absorbed and contained in the absorbent core from wetting articles that contact the diaper 100, such as bedsheets, pajamas, and undergarments. The backsheet 136 may also include a woven or nonwoven material, polymeric films such as thermoplastic films of polyethylene or polypropylene, and/or a multi-layer or composite materials comprising a film and a nonwoven material (e.g., having an inner film layer and an outer nonwoven layer). The backsheet may also include an elastomeric film. An example backsheet 136 may be a polyethylene film having a thickness of from about 0.012 mm (0.5 mils) to about 0.051 mm (2.0 mils). Exemplary polyethylene films are manufactured by Clopay Corporation of Cincinnati, Ohio, under the designation BR-120 and BR-121 and by Tredegar Film Products of Terre Haute, Ind., under the designation XP-39385. The backsheet 136 may also be embossed and/or matte-finished to provide a more clothlike appearance. Further, the backsheet 136 may permit vapors to escape from the absorbent core (i.e., the backsheet is breathable) while still preventing exudates from passing through the backsheet 136. The size of the backsheet 136 may be dictated by the size of the absorbent core 142 and/or particular configuration or size of the diaper 100.

In one embodiment, an adhesive may be applied to the garment-facing exterior of the backsheet for the purpose of holding the absorbent article in place by adhering to the wearer's underwear. Such adhesive may be especially desirable for use with adult incontinence and feminine hygiene type absorbent articles.

Also described above, the absorbent article 100 may include a topsheet 138. The topsheet 138 may also define all or part of the inner surface 132 of the chassis 102. The topsheet 138 may be compliant, soft feeling, and non-irritating to the wearer's skin. It may be elastically stretchable in one or two directions. Further, the topsheet 138 may be liquid pervious, permitting liquids (e.g., menses, urine, and/or runny feces) to penetrate through its thickness. A topsheet 138 may be manufactured from a wide range of materials such as woven and nonwoven materials; apertured or hydroformed thermoplastic films; apertured nonwovens, porous foams; reticulated foams; reticulated thermoplastic films; and thermoplastic scrims. Woven and nonwoven materials may comprise natural fibers such as wood or cotton fibers; synthetic fibers such as polyester, polypropylene, or polyethylene fibers; or combinations thereof. If the topsheet 138 includes fibers, the fibers may be spunbond, carded, wet-laid, meltblown, hydroentangled, or otherwise processed as is known in the art.

Topsheets 138 may be selected from high loft nonwoven topsheets, apertured film topsheets, and apertured nonwoven topsheets. Apertured film topsheets may be pervious to bodily exudates, yet substantially non-absorbent, and have a reduced tendency to allow fluids to pass back through and rewet the wearer's skin. Exemplary apertured films may include those described in U.S. Patent Nos. 5,628,097; 5,916,661; 6,545,197; and 6,107,539.

In some embodiments, the topsheet may comprise graphics such that depth perception is created as described in U.S. Pat. No. 7,163,528.

The absorbent article 100 may also include an absorbent assembly 140 that is joined to the chassis 102. As shown in Figures 2, 4, 5, and 6 the absorbent assembly 140 may have a laterally extending front edge 148 in the front waist region 116 and may have a longitudinally opposing and laterally extending back edge 150 in the back waist region 118. The absorbent assembly may have a longitudinally extending right side edge 152 and may have a laterally opposing and longitudinally extending left side edge 154, both absorbent assembly side edges 152 and 154 may extend longitudinally between the front edge 148 and the back edge 150. The absorbent assembly 140 may additionally include one or more absorbent cores 142 or absorbent core layers. The absorbent core 142 may be at least partially disposed between the topsheet 138 and the backsheet 136 and may be formed in various sizes and shapes that are compatible with the diaper. Exemplary absorbent structures for use as the absorbent core of the present disclosure are described in U.S. Patent Nos. 4,610,678; 4,673,402; 4,888,231; and 4,834,735.

Some absorbent core embodiments may comprise fluid storage cores that contain reduced amounts of cellulosic airfelt material. For instance, such cores may comprise less than about 40%, 30%, 20%, 10%, 5%, or even 1% of cellulosic airfelt material. Such a core may comprise primarily absorbent gelling material in amounts of at least about 60%, 70%, 80%, 85%, 90%, 95%, or even about 100%, where the remainder of the core may comprise a microfiber glue (if applicable). Such cores, microfiber glues, and absorbent gelling materials are described in U.S. Patent Nos. 5,599,335; 5,562,646; 5,669,894; and 6,790,798 as well as U.S. Patent Publication Nos. 2004/0158212 and 2004/0097895.

The absorbent article 100 may also include elasticized leg cuffs 156. It is to be appreciated that the leg cuffs 156 may be and are sometimes also referred to as leg bands, side flaps, barrier cuffs, elastic cuffs, or gasketing cuffs. The elasticized leg cuffs 156 may be configured in various ways to help reduce the leakage of body exudates in the leg regions. For example, in some embodiments, a gasketing leg cuff 160 may be positioned adjacent to the side edge 130, 128 of the chassis 102 and a barrier leg cuff 158 may be positioned between a gasketing leg cuff 160 and the longitudinal axis 124 of the absorbent article 100. Example leg cuffs 156 may include those described in U.S. Patent Nos. 3,860,003; 4,909,803; 4,695,278; 4,795,454; 4,704,115; 4,909,803; U.S. Patent Publication No. 2009/0312730 A1; and U.S. Patent Publication No. 2013/0255865 A1.

As mentioned above, diaper pants may be manufactured with a ring-like elastic belt 104 and provided to consumers in a configuration wherein the front waist region 116 and the back waist region 118 are connected to each other as packaged, prior to being applied to the wearer. As such, the absorbent article may have a continuous perimeter waist opening 110 and continuous perimeter leg openings 112 such as shown in Figure 1. As previously mentioned, the ring-like elastic belt 104 is defined by a first elastic belt 106 connected with a second elastic belt 108. As shown in Figure 2, the first elastic belt 106 defines first and second opposing end regions 106a, 106b and a central region 106c, and the second elastic 108 belt defines first and second opposing end regions 108a, 108b and a central region 108c.

The central region 106c of the first elastic belt is connected with the first waist region 116 of the chassis 102, and the central region 108c of the second elastic belt 108 is connected with the second waist region 118 of the chassis 102. As shown in Figure 1, the first end region 106a of the first elastic belt 106 is connected with the first end region 108a of the second elastic belt 108 at first side seam 178, and the second end region 106b of the first elastic belt 106 is connected with the second end region 108b of the second elastic belt 108 at second side seam 180 to define the ring-like elastic belt 104 as well as the waist opening 110 and leg openings 112.

As shown in Figures 2, 3A, and 3B, the first elastic belt 106 also defines an outer lateral edge 107a and an inner lateral edge 107b, and the second elastic belt 108 defines an outer lateral edge 109a and an inner lateral edge 109b. The outer lateral edges 107a, 109a may also define the front waist edge 121 and the laterally extending back waist edge 122. The first elastic belt and the second elastic belt may also each include an outer, garment facing layer 162 and an inner, wearer facing layer 164. It is to be appreciated that the first elastic belt 106 and the second elastic belt 108 may comprise the same materials and/or may have the same structure. In some embodiments, the first elastic belt 106 and the second elastic belt may comprise different materials and/or may have different structures. It should also be appreciated that the first elastic belt 106 and the second elastic belt 108 may be constructed from various materials. For example, the first and second belts may be manufactured from materials such as plastic films; apertured plastic films; discrete strands; woven or nonwoven webs of natural materials (e.g., wood or cotton fibers), synthetic fibers (e.g., polyolefins, polyamides, polyester, polyethylene, or polypropylene fibers) or a combination of natural and/or synthetic fibers; or coated woven or nonwoven webs. In some embodiments, the first and second elastic belts may include a nonwoven web of synthetic fibers, and may include a stretchable nonwoven. In other embodiments, the first and second elastic belts may include an inner hydrophobic, non-stretchable nonwoven material and an outer hydrophobic, non-stretchable nonwoven material.

The first and second elastic belts 106, 108 may also each include belt elastic material interposed between the outer layer 162 and the inner layer 164. The belt elastic material may include one or more elastic elements such as strands, ribbons, or panels extending along the lengths of the elastic belts. As shown in Figures 2, 3A, and 3B, the belt elastic material may include a plurality of elastic strands 168 that may be referred to herein as outer, waist elastics 170 and inner, waist elastics 172.

As shown in Figure 2, the outer, waist elastics 170 extend continuously laterally between the first and second opposing end regions 106a, 106b and across the central region 106c of the first elastic belt 106 and between the first and second opposing end regions 108a, 108b and across the central region 108c of the second elastic belt 108. In some embodiments, some elastic strands 168 may be configured with discontinuities in areas. For example, as shown in Figure 2, the inner, waist elastics 172 extend intermittently along the first and second elastic belts 106, 108. More particularly, the inner, waist elastics 172 extend along the first and second opposing end regions 106a, 106b and partially across the central region 106c of the first elastic belt 106. The inner, waist elastics 172 also extend along the first and second opposing end regions 108a, 108b and partially across the central region 108c of the second elastic belt 108. As such, the inner, waist elastics 172 do not extend across the entirety of the central regions 106c, 108c of the first and second elastic belts 106, 108. Thus, some elastic strands 168 may not extend continuously through regions of the first and second elastic belts 106, 108 where the first and second elastic belts 106, 108 overlap the absorbent assembly 140. In some embodiments, some elastic strands 168 may partially extend into regions of the first and second elastic belts 106, 108 where the first and second elastic belts 106, 108 overlap the absorbent assembly 140. In some embodiments, some elastic strands 168 may not extend into any region of the first and second elastic belts 106, 108 where the first and second elastic belts 106, 108 overlap the absorbent assembly 140. It is to be appreciated that the first and/or second elastic belts 106, 108 may be configured with various configurations of discontinuities in the outer, waist elastics 170 and/or the inner, waist elastic elastics 172.

In some embodiments, the elastic strands 168 may be disposed at a constant interval in the longitudinal direction. In other embodiments, the elastic strands 168 may be disposed at different intervals in the longitudinal direction. As discussed in more detail below, the belt elastic strands 168, in a stretched condition, may be interposed and joined between the uncontracted outer layer and the uncontracted inner layer. When the belt elastic material is relaxed, the belt elastic material returns to an unstretched condition and contracts the outer layer and the inner layer. The belt elastic material may provide a desired variation of contraction force in the area of the ring-like elastic belt. It is to be appreciated that the chassis 102 and elastic belts 106, 108 may be configured in different ways other than as depicted in Figure 2.

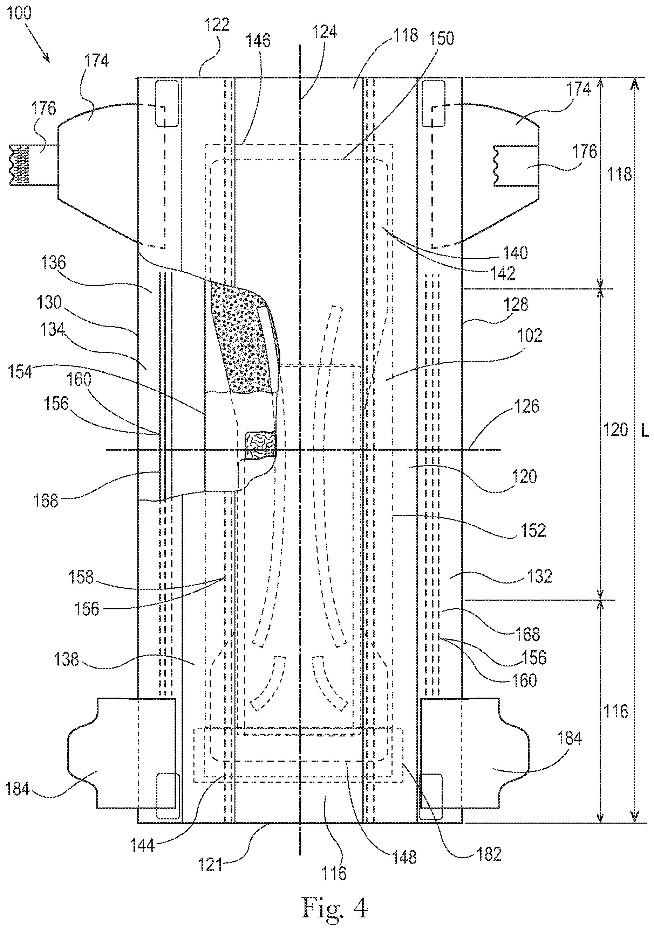

In some embodiments, as illustrated in Figure 4, the absorbent article 100 may comprise front ears 184 and back ears 174. The front ears 184 and the back ears 174 may be an integral part of the chassis 102. For example, the front ears 184 and the back ears 174 may be formed from the topsheet 138 and/or the backsheet 136. Alternatively, the front ears 184 and the back ears 174 may be attached to the backsheet 136 and/or the topsheet 138. The front ears 184 and the back ears 174 may be extensible to facilitate attachment on the landing zone 182 and to maintain placement around the waist of the wearer. The back ears 174 may comprise a tab member 176. The tab member 176 may be attached to a portion of the back ears 174 to facilitate attachment to the landing zone 182.

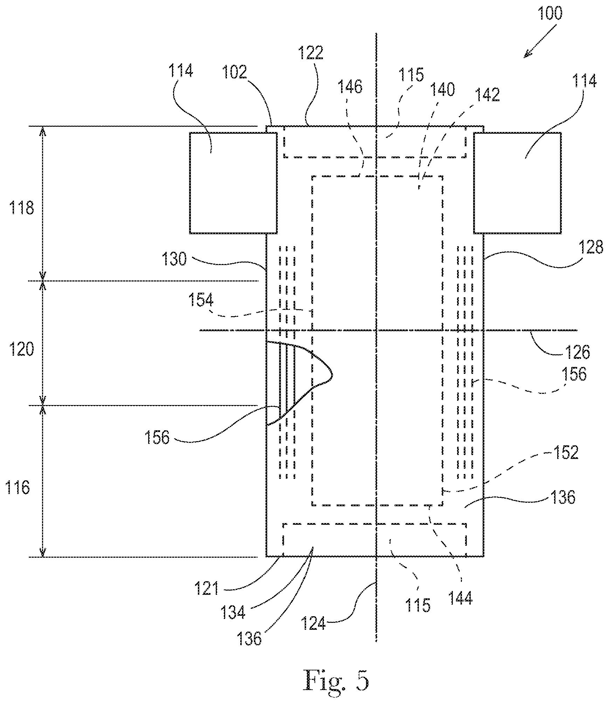

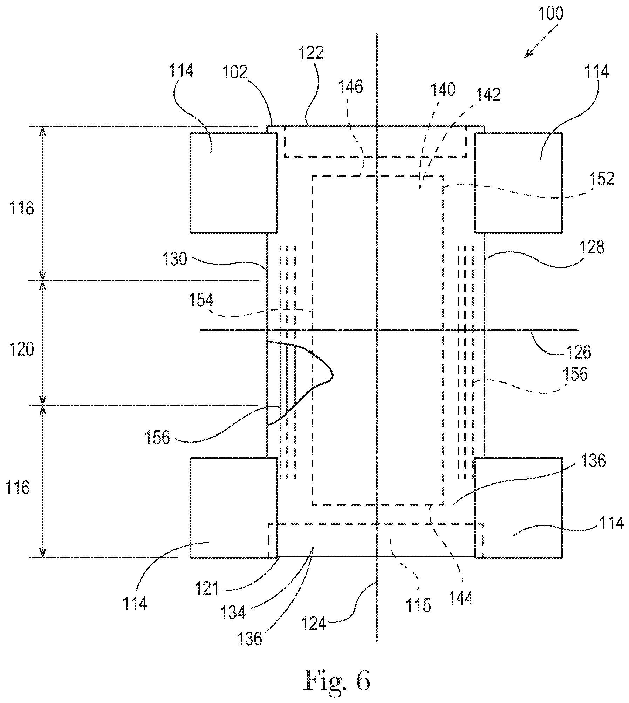

In some embodiment, referring to Figures 5 and 6, the article 100 may comprise an elasticized waistband 115. The elasticized waistband may provide improved fit and containment and may be configured to elastically expand and contract laterally to dynamically fit a wearer's waist. The elasticized waistband may extend longitudinally outwardly from the waist edge of the absorbent article 100 toward the edge of the absorbent core 142. In one embodiment, the absorbent article 100 may have two elasticized waistbands, one positioned in the back waist region 118 and one positioned in the front waist region 116, although other embodiments may be constructed with a single elasticized waistband. The elasticized waistband may be constructed in a number of different configurations including those described in U.S. Pat. Nos. 4,515,595 and 5,151,092. Further, the waistband may be constructed as disclosed in U.S. Publication Nos. 2012/0330262; 2012/0330263; and 2012/0330264 such that the waistband works in combination with the leg cuffs to provide improved fit and containment.

In some embodiments, the elasticized waistbands may comprise materials that have been "prestrained" or "mechanically prestrained" (i.e., subjected to some degree of localized pattern mechanical stretching to permanently elongate the material). In some embodiments, the materials may be prestrained using suitable deep embossing techniques. In other embodiments, the materials may be prestrained by directing the material through an incremental mechanical stretching system as described in U.S. Pat. No. 5,330,458. The materials may then be allowed to return to their substantially untensioned condition, thus forming a zero strain stretch material that is extensible, at least up to the point of initial stretching. Examples of zero strain materials are disclosed in U.S. Pat. Nos. 2,075,189; 3,025,199; 4,107,364; 4,209,563; 4,834,741; and 5,151,092. The waistband may be any shape and size that allows the absorbent article to fit the wearer as desired about the waist region.

In some embodiments, the waistband may be positioned between the side panels 114 and/or the back ears 174 and/or front ears 184. In other embodiments the waistband may be positioned such that a portion of the waistband overlaps a portion of the side panels 114 and/or the back ears 174 and/or the front ears 184.

In some embodiments, the absorbent article 100 may comprise side panels 115. The side panels 115 may be discrete from or integral with the chassis 100. A discrete side panel is formed as a separate element that is joined to the chassis 100. In some embodiments, this includes a plurality of side panels, e.g. Figures 5 or 6 (also referred to as ear panels or side flaps) being joined to the side edges 128, 130 of the chassis in the front and/or rear waist regions 118 and 116. The side panel may be attached to the garment facing surface 132, the body facing surface 132, or between the garment facing surface 132 and the body facing surface 132, such as between the topsheet 138 and the backsheet 136. In some embodiments, the waistbands 112 can overlap the side panels to create a continuous belt-like structure (not shown).

In some embodiments, the side panels in the back waist region may connect with the garment facing surface of the absorbent article in the front waist region to form a waist circumference that may encircle the wearer during wear of the absorbent article. In other embodiments, the side panels disposed in the back waist region may connect with the side panels disposed in the front waist region at a seam, which forms a waist circumference that may encircle the wearer during wear of the absorbent article. The seam may be an overlapping seam or a butt seam. Further, in some embodiments, the seam may be refastenable, such that the side panels may be detached and reattached, or permanent, such that the seam may not be detached and reattached.

The side panels may comprise an inner nonwoven layer and an outer nonwoven layer and elastic elements, such as elastic strands or a film, therebetween. The inner and outer nonwoven layers may be joined using adhesive or thermoplastic bonds. Various suitable side panel configurations can be found in U.S. Pub. No. 2013/0211363.

An integral side panel is a portion, one or more layers, of the chassis that projects laterally outward from the longitudinal edge. The integral flap may be formed by cutting the chassis to include the shape of the flap projection.

While many of the embodiments illustrated in this application having belt-like side flaps are pant articles, taped articles may have belt-like side flaps disposed in one or both waist regions as well. The side panels may be any shape that allows the absorbent article to fit the wearer as desired about the waist region and the leg openings.

The absorbent article may also include a fastening system. When fastened, the fastening system interconnects the front waist region 116 and the rear waist region 118 resulting in a waist circumference that may encircle the wearer during wear of the absorbent article 10. This may be accomplished by ears 174, 184 or side panels 115, for example. The ears 174 or side panels 115 in the back waist region interconnect with ears 184 or side panels 115 in the front waist region or by the flaps or side panels in the back waist region interconnecting with the chassis 100 in the front waist region. The fastening system may comprises a fastener 176 such as tape tabs, hook and loop fastening components, interlocking fasteners such as tabs and slots, buckles, buttons, snaps, and/or hermaphroditic fastening components, although any other known fastening means are generally acceptable. The fasteners may releasably engage with a landing zone 182, which may be a woven or nonwoven. Some exemplary surface fastening systems are disclosed in U.S. Patent Nos. 3,848,594; 4,662,875; 4,846,815; 4,894,060; 4,946,527; 5,151,092; and 5,221,274. An exemplary interlocking fastening system is disclosed in U.S. Patent No. 6,432,098. The fastening system may also provide a means for holding the article in a disposal configuration as disclosed in U.S. Pat. No. 4,963,140. The fastening system may also include primary and secondary fastening systems, as disclosed in U.S. Pat. No. 4,699,622. The fastening system may be constructed to reduce shifting of overlapped portions or to improve fit as disclosed in U.S. Patent Nos. 5,242,436; 5,499,978; 5,507,736; and 5,591,152.

It is to be appreciated that any of the aforementioned components of the absorbent article may include one or more substrates. For example, a substrate having a single layer with a basis weight from about 8 to about 40 g/m² may form a component or portion of a component of the absorbent article. It is also to be appreciated that the substrate may include more than one layer.

Particularly regarding feminine hygiene products, one suitable material for the backsheet can be a liquid impervious thermoplastic film having a thickness of from about 0.012 mm (0.50 mil) to about 0.051 mm (2.0 mils), for example including polyethylene or polypropylene. Typically, the backsheet can have a basis weight of from about 5 g/m² to about 35 g/m². The backsheet can be typically positioned adjacent the outer-facing surface of the absorbent core and can be joined thereto. For example, the backsheet may be secured to the absorbent core by a uniform continuous layer of adhesive, a patterned layer of adhesive, or an array of separate lines, spirals, or spots of adhesive. Illustrative, but nonlimiting adhesives, include adhesives manufactured by H. B. Fuller Company of St. Paul, Minn., U.S.A., and marketed as HL-1358J. An example of a suitable attachment device including an open pattern network of filaments of adhesive is disclosed in U.S. Pat. No. 4,573,986. Another suitable attachment device including several lines of adhesive filaments swirled into a spiral pattern is illustrated by the apparatus and methods discussed in U.S. Pat. Nos. 3,911,173; 4,785,996; and 4,842,666. Alternatively, the attachment device may include heat bonds, pressure bonds, ultrasonic bonds, dynamic mechanical bonds, or any other suitable attachment device or combinations of these attachment devices.

Further, the absorbent article, such as a feminine hygiene product, may comprise "wings" (not shown) intended to wrap the edges of the wearer's undergarments in the crotch region and/or affix the article to the undergarment to avoid poor folding and premature detachment. Exemplary absorbent articles comprising wings are disclosed in U.S. Pat. No. 8,039,685.

It is to be appreciated that the features of the absorbent article described herein may be excluded or combined to form various embodiments of an absorbent article.

As previously mentioned, the methods according to the present disclosure may be utilized to assemble discrete absorbent articles 100 and/or various components of absorbent articles 100, such as for example, chassis 102, elastic belts 106, 108, and/or leg cuffs 156. Although the following methods may be provided in the context of absorbent articles 100, as shown in Figures 1, 2, 4, 5, and 6, it is to be appreciated that the methods and apparatuses herein may be used with various process configurations and/or absorbent articles, such as for example, disclosed in U.S. Patent No. 7,569,039 and 9,072,632; U.S. Patent Publication Nos. 2005/0107764 A1, 2012/0061016 A1, and 2012/0061015 A1; 2013/0255861 A1; 2013/0255862 A1; 2013/0255863 A1; 2013/0255864 A1; and 2013/0255865 A1; and U.S. Patent Application Ser. Nos. 62/136,003 filed on March 20, 2015; 14/996,683 filed on January 15, 2016; and 62/286,662 filed on January 25,2016.

Other materials that may be considered substrates, or include substrates as a part of a final product, with respect to the disclosure include films. Suitable films include water-soluble or water-dispersible films. The films may be thermo-formable and/or vacuum-formable. The film may include polymeric materials. Suitable polymeric materials include polyvinyl alcohols, hydroxypropyl methyl cellulose (HPMC), copolymers thereof, derivatives thereof, or combinations thereof. The film may further include one or more additive ingredients, such as plasticizer, surfactant, cleaning additives, water, or other suitable adjuncts. The films may be obtained by casting, blow-molding, extrusion or blown extrusion of the polymeric material, as known in the art. The film may have a thickness of from about 20 to 150 microns, or from about 50 to 110 microns. Suitable water-soluble films may include those supplied by MonoSol, LLC (Merrillville, Indiana, USA) under the trade references M8630, M8900, M8779, M9467, and M8310, as well as films, such as PVA films, having corresponding solubility, deformability, and/or sealing characteristics. Suitable films are also described in US 6,166,117, US 6,787,512, US 2006/0213801, WO 2010/119022, US 2011/0186468, and US 2011/0188784.

The films may be formed, for example by thermoforming and/or vacuum-forming, into unitized dose pouches, such as single- or multi-compartment pouches. One or more films may be formed into a web of sealed compartments via a continuous or a discontinuous process, and the web may be cut to form individual pouches. The pouches may contain a composition, such as a fabric care or hard surface care composition. Such compositions may be in the form of liquid, gel, solid, granular, or combinations thereof. Suitable pouches and processes for making such pouches are described in WO 2002/42408 and WO 2009/098659. Commercially available pouches include those marketed as TIDE PODS, GAIN FLINGS, and CASCADE ACTIONPACS (each available from The Procter & Gamble Company, Cincinnati, Ohio, USA).

Further, substrates may be used in cleaning products. For example, a duster cleaning article may comprise a nonwoven sheet having tow fibers joined thereto. The cleaning article may have a longitudinal axis. The tow fibers may be joined to the nonwoven sheet in a generally transverse direction and particularly in a direction normal the longitudinal axis, to provide a laminate structure of two layers.

If desired, the cleaning article may comprise additional layers, also referred to herein as laminae. For example, the tow fibers may be disposed intermediate two nonwoven sheets. Plural laminae of tow fibers may be disposed intermediate the nonwoven sheets and/or outboard thereof. Optionally, one or more of the nonwoven sheets may be cut to comprise strips. The strips may be generally normal to the longitudinal axis.

The tow fibers and/or nonwoven sheets may comprise an additive to assist in removal of dust and other debris from the target surface. The additive may comprise wax, such as microcrystalline wax, oil, adhesive and combinations thereof. The cleaning article may be made according to US Patent 6,813,801 and according to commonly assigned US 7,803,726; 8,756,746; 8,763,197 and 8,931,132.

The laminae of the cleaning article may be joined together using adhesive, thermal bonding, ultrasonic welding, etc. If desired, the bonding lines may be generally parallel to the longitudinal axis and may be continuous, or discontinuous as desired. Three longitudinally parallel bonding lines may be utilized to define two sleeves.

The two sleeves may accept one or more complementary fork tines of a handle. The fork tines may be removably inserted into the sleeves of the cleaning article to provide for improved ergonomics. The handle may be plastic and made according to the teachings of US patents 7,219,386; 7,293,317, 7,383,602 and/or commonly assigned 8,578,564. Representative dusters are sold by the instant assignee under the name SWIFFER®.

Further still, substrates may include cleaning sheets. The cleaning sheet may comprise a nonwoven. The nonwoven may be synthetic and/or have cellulosic fibers therein. The synthetic fibers may comprise carded, staple, wet laid, air laid and/or spunbond fibers. The nonwoven cleaning sheet may be made according to a hydro-entangling process to provide a texture and a basis weight of about 20 to about 120 g/m².

Optionally, the cleaning sheet may further comprise an additive, to improve cleaning performance and/or enhance the cleaning experience. The additive may comprise wax, such as microcrystalline wax, oil, adhesive, perfume and combinations thereof. The cleaning sheet according to the present invention may be made according to commonly assigned US patents 6,305,046; 6,484,346; 6,561,354; 6,645,604; 6,651,290; 6,777,064; 6,790,794; 6,797,357; 6,936,330; D409,343; D423,742; D489,537; D498,930; D499,887; D501,609; D511,251 and/or D615,378.

In some embodiments, the cleaning sheet may comprise layers, to provide for absorption and storage of cleaning fluid deposited on the target surface. If desired, the cleaning sheet may comprise absorbent gelling materials to increase the absorbent capacity of the cleaning sheet. The absorbent gelling materials may be distributed within the cleaning sheet in such a manner to avoid rapid absorbency and absorb fluids slowly, to provide for the most effective use of the cleaning sheet.

The cleaning sheet may comprise plural layers disposed in a laminate. The lowest, or downwardly facing outer layer, may comprise apertures to allow for absorption of cleaning solution therethrough and to promote the scrubbing of the target surface. Intermediate layers may provide for storage of the liquids, and may comprise the absorbent gelling materials. The cleaning sheet may have an absorbent capacity of at least 10, 15, or 20 grams of cleaning solution per gram of dry cleaning sheet, as set forth in commonly assigned US Patents 6,003,191 and 6,601,261.

The top or upwardly facing outer layer, maybe liquid impervious in order to minimize loss of absorbed fluids. The top layer may further provide for releasable attachment of the cleaning sheet to a cleaning implement. The top layer may be made of a polyolefinic film, such as LDPE.

As previously mentioned, the apparatuses and methods according to the present disclosure may be used to impart a line of weakness into a substrate, to separate the substrate, and to assemble absorbent articles that include components comprising one or more substrates. Some of these components may require imparting a line of weakness and separating the substrate so that the component is the proper size and/or the proper shape, for example, to be joined to other components.

A laser source is one method used to impart a line of weakness into these components or substrates. The laser source may be used to project a laser beam at a scan head that directs the laser beam, also referred to herein as a laser, at the component part, which may be, for example, an advancing substrate, such as a nonwoven, a film, or a laminate. An example of a scan head is the SCANcube III available from SCANLAB America, Inc. of St. Charles, IL. The laser beam interacts with a portion of the advancing substrate resulting in a line of weakness being imparted to that portion of the advancing substrate. Upon a line of weakness being imparted to the substrate, the advancing substrate may be separated into a first portion and a second portion. Each of the first portion and the second portion has a separation edge. The separation edge is the edge formed from the laser beam causing the ablation or ablation and melting of the substrate and the separating of the substrate along the line of weakness. Generally, the more power used by the laser source, the faster the substrate line of weakness may be imparted to the substrate. Due to relatively high manufacturing speeds, using a laser source to impart a line of weakness requires the laser to traverse relatively quickly, which has traditionally required the laser to operate at a relatively high power output.

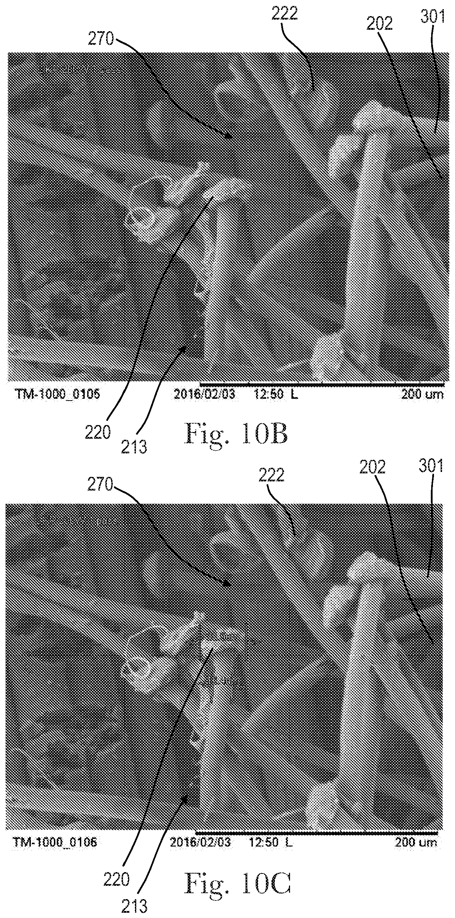

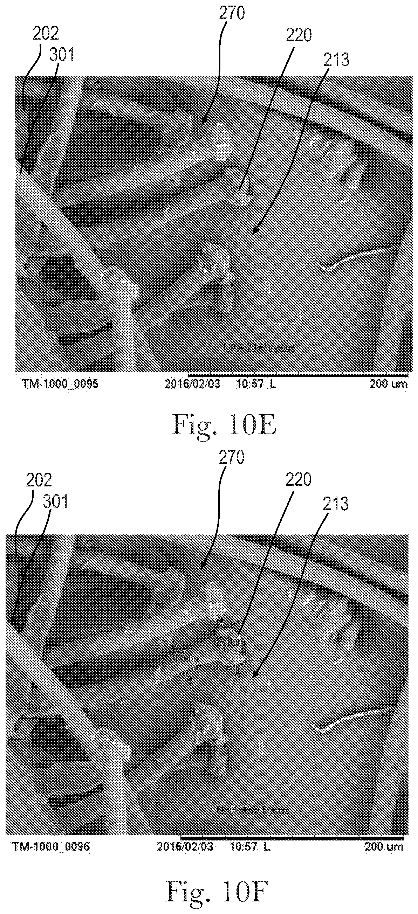

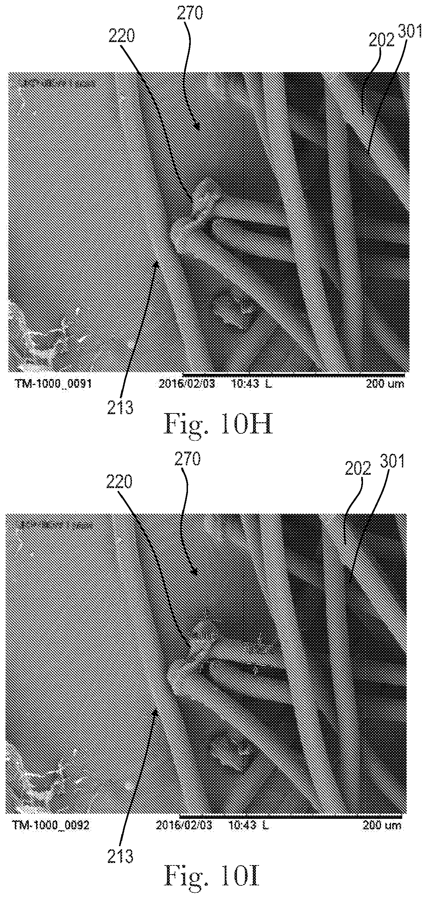

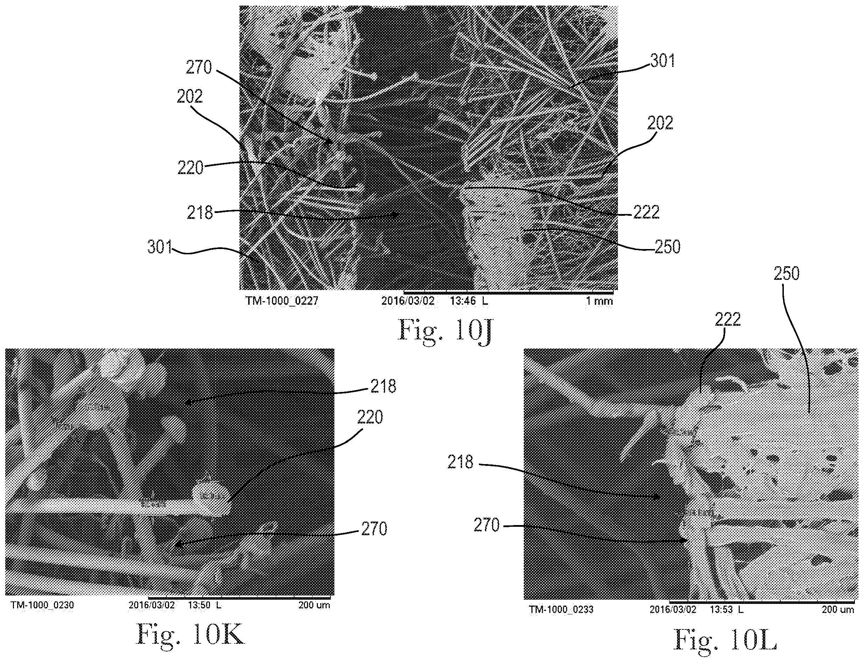

However, increasing the power of the laser source has traditionally resulted in degradation of the final edge. More specifically, cutting substrates with the use of a laser source operating at a relatively high power, which generates a greater amount of heat, may create a relatively rough feeling at the edge of the substrate. For nonwoven substrates, this rough edge is due to the formation of accumulated material. The accumulation of material is due, in part, to the elastic and/or thermal deformation of the substrate during the cutting of the substrate. For example, for a nonwoven substrate, the individual fibers that are in relatively direct contact with the laser beam are ablated. However, the individual fibers of the nonwoven substrate along the edge that do not get ablated undergo melting and/or shrinkage and subsequent cooling. The amount of melting of the nonwoven substrate is greater when the heat generated by the laser beam has a greater time to penetrate the nonwoven substrate. The heat generated from the laser source generally penetrates the nonwoven beginning at the edge and extending perpendicular to the edge toward the central portion of the substrate or parallel to the direction of the laser beam. It is to be appreciated that heat affects the nonwoven in the area surrounding the laser beam. When cutting through the nonwoven, some material is ablated or vaporized to form the edge. In some embodiments, during the subsequent cooling of the separated nonwoven, the fibers along the edge snap-back, which also may be described as roll back, resulting in an accumulation of material at the end portion of the fibers, which will be referred to herein as an accumulation bulb 220 such as illustrated in Figure 10B, 10E, and 10H. Further, one or more fibers may join together to form a cluster of accumulated material. A cluster occurs when one or more fibers join together during the melting of the nonwoven substrate. Generally, the longer time the laser beam dwells on the material, the larger the amount of accumulated bulbs and/or clusters at the edge. This accumulated material, including bulbs and clusters, is particularly undesirable for absorbent articles. Absorbent articles are intended to be worn or used in close contact with an individual's skin. Therefore, it is undesirable to have an absorbent article that is perceived to be rough and/or coarse.

It is also to be appreciated that a portion of the snap-back, also referred to as roll back, may be due to the processes used to form the nonwoven substrate. The individual fibers used to form a nonwoven substrate may be made by an extrusion process, for example. An extruder forces the individual fibers through a tubular structure resulting in the individual fibers being under some tension. As the fibers are laid down to form the nonwoven substrate, the individual fibers are still under a relative amount of tension. However, when the laser source acts on the individual fibers to separate them, the tension in the individual fibers is released causing the individual fiber to want to relax. This release of tension and relaxation of the individual fiber may contribute to the accumulation of material at the end of individual fiber, the accumulation bulb, that has undergone separation by the laser. The tension in the individual fiber may only be one of numerous factors that contribute to the accumulation bulb at the end of the individual fiber.

Further, when cutting through films, some material is ablated or vaporized to form the edge. The material adjacent the edge or in the heat modified zone may deform such that the edge undulates forming a wave-like pattern. The heat transferred to the film may also result in thermal distortion and roughness at the edge. The heat may also affect properties of the film such as its strength and degradation rate. The material in the heat modified zone may also be chemically transformed such that the molecular structure of the film are changed due to the effect of the heat. For example, the degree of crystallinity and polymer chain alignment may be changed. A high degree of crystallinity and/or polymer chain alignment is likely associated with a stiff, high modulus material. Heating the material of a film above its melting point may result in an undesirable change in crystallinity once the material re-solidifies. Also, melting the material may also result in a loss of polymer chain alignment induced by radial expansion, which may adversely affect the material. The greater the heat generated by the laser source, the greater the undesirable impact on the film and the larger the heat modified zone.

As previously discussed, it is desired to produce a substrate that includes minimal or no accumulation bulbs and clusters, or minimal or no changes to the chemical properties of the substrate. Stated another way, it is desirable to minimize or eliminate the heat modified zone. The heat modified zone is the zone of the substrate that is not removed during cutting but is exposed to the energy from the laser beam, either directly or indirectly. Direct exposure may be due to exposure of the substrate from a section of the laser beam with an intensity that is not great enough to remove the substrate material. For example, the portions of the laser beam near its edges may not have an intensity sufficiently high to ablate or remove the substrate material. A substrate may also be exposed to energy indirectly due to heat conduction. For example, a nonwoven material may heat up causing distortion and roughness at the edge, which may be due to clusters and/or accumulation bulbs forming along the edge. The heat may also alter the properties of the substrate material resulting in changes in, for example, chemical properties, strength, degradation rate, stiffness, and edge sharpness.

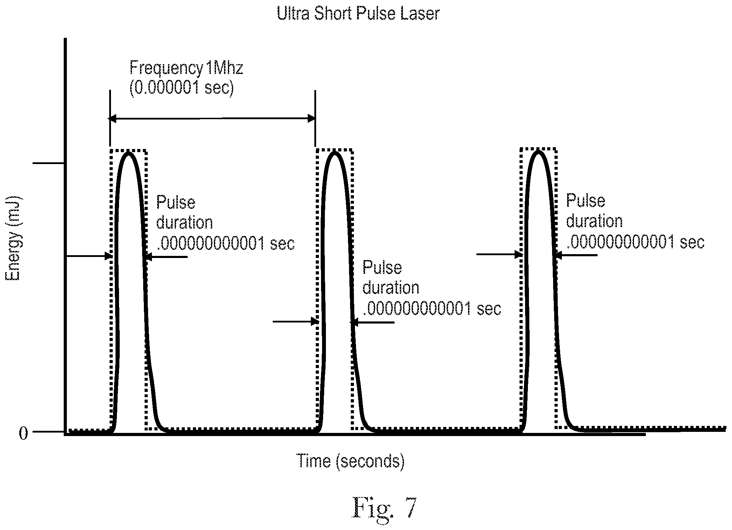

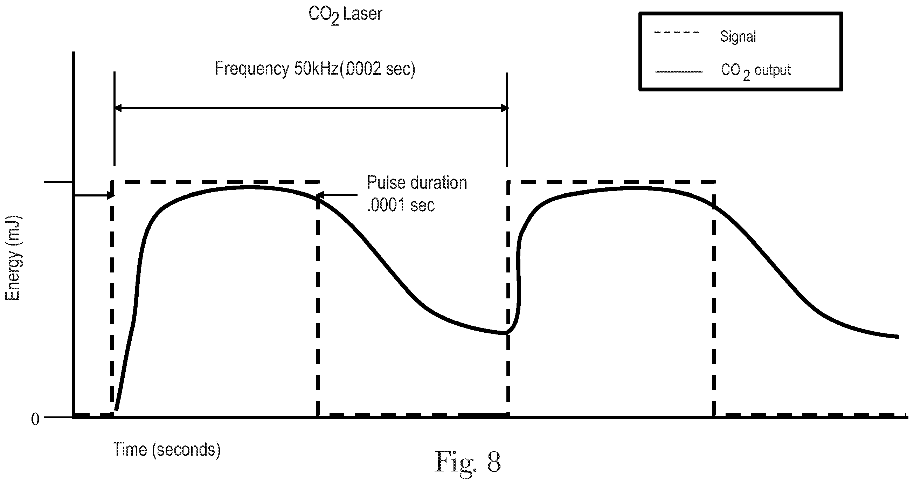

Each laser source operates at a certain pulse duration and frequency. The pulse duration, also referred to herein as pulse, is the period of time over which the laser beam imparts energy to the material. The frequency is the time period starting from the beginning of a first pulse and ending at the beginning of the next, subsequent pulse, as illustrated, for example in Figures 7 and 8. Different types of lasers have different pulse durations. Lasers that have a relatively longer pulse duration, referred to herein as longer-pulse lasers, generally remove material or cut material thermally. By contrast, ultra short pulse lasers have a relatively short pulse duration, as illustrated in Figure 7. The pulse duration and frequency change how the laser beam interacts with the substrate. Generally, the laser energy is absorbed by the material, which may be a substrate, resulting in an increase in temperature at and/or near the area of absorption. As the temperature of that material increases to the melting point, material is removed by conventional melting and vaporization. However, because the longer pulse lasers have a longer pulse duration and longer frequency, longer pulse lasers cause the temperature of the material to increase resulting in melting of the substrate and an undesirable heat modified zone. By contrast, for ultra short pulse lasers, which have a short pulse duration and short frequency, the temperature rise in the area to be affected may be fast and short, resulting in thermal ablation. Generally, an advantage of ultra short pulse lasers over longer-pulse lasers is that the ultra short pulse lasers deposit energy so quickly that the material ablates before having the time to melt the adjacent areas of the material. An ultra short pulse laser converts portions of the material from a solid state to a gaseous state with minimal effect on the area adjacent the cut edge or separation edge. Thus, using a longer-pulse laser results in a greater heat modified zone than using an ultra short pulse laser.

Ultra short pulse lasers refer to lasers having pulse durations less than about 100 picoseconds (10⁻¹²). Ultra short pulse lasers may have pulse durations on the order of femtoseconds (10⁻¹⁵). Ultra short pulse lasers are distinguishable from continuous wave lasers and longer-pulse lasers, which may have a pulse duration of nanoseconds (10⁻⁹). Examples of ultra short pulse lasers include Ti-Sapphire and Dye lasers. Ultra short pulse lasers are available to be supplied, for example, by Coherent, Inc. of Santa Clara, California; TRUMPF Inc. of Farmington, Connecticut; and ROFIN-SINAR Laser GmbH of Hamburg, Germany. Similarly, examples of continuous wave lasers and longer-pulse lasers include CO₂ and Nd:Yag. Figures 7 and 8 graphically depict the difference in the pulse duration and frequency of an ultra short pulse laser, Figure 7, and a CO₂ laser, Figure 8.

Referring to Figure 7, the ultra short pulse laser is able to operate at relatively high frequencies and relatively short pulse durations. For example, the frequency of the ultra short pulse laser may be 1MHz (0.000001 seconds) and the pulse duration may be 1 picosecond (0.000000000001 seconds). It is to be appreciated that the pulse duration is one million times shorter than the period, which is the inverse of the frequency. The ultra short pulse laser is configured to output energy during the pulse duration. Because the energy is output at such a short interval of time, the thermal effects due to the energy imparted to the substrate are minimized. Stated another way, the pulse duration, the time that energy is imparted to the substrate, is short compared to the thermal diffusion time of the material of the substrate. Thus, there is no or very limited time for the heat to diffuse and the material adjacent the laser beam to melt, forming accumulation bulbs and/or clusters and/or altering the properties of the material. Further, the frequency of the ultra short pulse laser is relatively high. The frequency is the time from the start of a first pulse duration to the start of the next, subsequent pulse duration. Frequency is measured as a cycle/second. Thus, during a single cycle, energy is imparted to the substrate during the pulse duration and the remainder of the time, no or minimal energy is imparted to the substrate because the decay time for the ultra short pulse laser is almost instantaneous. This allows for relatively high energy to be imparted to web over repeated, short periods of time. Due to relatively short frequency, short pulse duration, and precision of the ultra short pulse lasers, there is a lower dependence on wavelength, which will be discussed herein, and an ability to machine materials that have a greater heat sensitivity.

By contrast, Figure 8 graphically shows the frequency and pulse duration of a CO₂ laser, which is considered to be a longer pulse laser. As illustrated, the pulse duration is much longer than that of the ultra short pulse laser. In application, when this laser is used quickly the laser is unable to recover such that there are clear, defined pulse durations. As illustrated in Figure 8, after the laser imparts energy to the substrate during the pulse duration, the laser imparts no energy to the substrate during the remainder of the cycle. During the time that the signal passed to the laser source instructs the laser source not to impart energy to the substrate, the laser source tapers off over a period of time, referred to as the decay time, as shown by the output. Generally, the tapering off is longer than the pulse duration. As illustrated by the graphs, the decay time is much faster for the ultra short pulse laser. Further, the CO₂ laser is unable to output energy over as short a period of time as compared to the ultra short pulse laser. Rather, the CO₂ laser imparts energy over a longer pulse duration which allows the material to heat up during cutting or scoring and results in a more undesirable edge as compared with the edge formed by the ultra short pulse laser.

It is also to be appreciated that the wavelength of ultra short pulse lasers and longer pulse lasers are different. The wavelength of the ultra short pulse laser is less than about 1 micron. For example, the wavelength of the ultra short pulse laser may be from about 300 nanometers to about 1080 nanometers and/or from about 700 nanometers to about 1030 nanometers, including all 0.1 nanometer increments therebetween. Further, in some embodiments, the wavelength of the ultra short pulse laser may be from about 1000 nm to about 1080 nm and/or from about 1020 nm to about 1070 nm and/or from about 1030 nm to about 1050 nm, including all 0.1 nm increments therebetween. Generally, each different material has a wavelength or range of wavelengths at which its absorptivity is greatest or optimal. Thus, a laser source may be chosen such that the wavelength emitted by the laser is more readily absorbed by the substrate. It is to be appreciated that materials may be altered to increase their absorptivity even if the laser source is operating outside their optimal range of wavelengths. In some embodiments, the substrate may be chemically altered such that the substrate has an increased rate of energy absorption, or absorptivity. It is believed that due to the pulse duration at which the ultra short pulse laser operates and the great amount of energy imparted to the substrate over such a short period of time, the ultra short pulse laser is able to modify materials that have a greater heat sensitivity, and, thus, are less dependent on the wavelength at which the absorptivity is greatest or optimal.

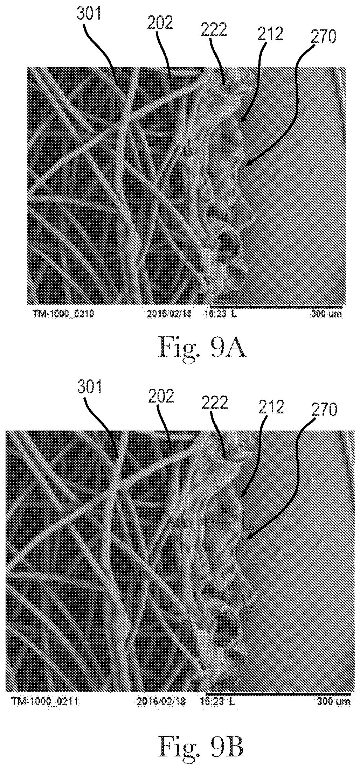

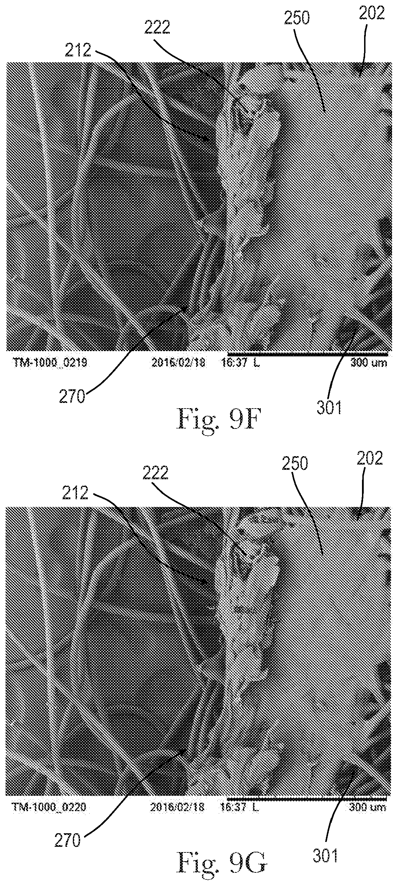

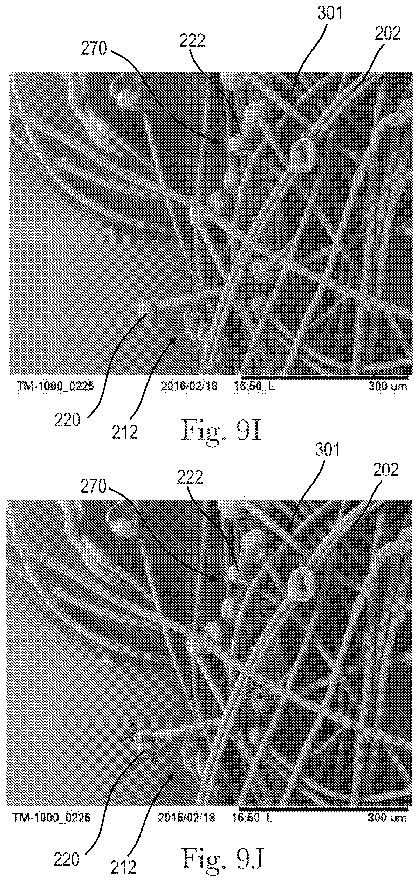

Figures 9A-10I illustrate the difference in the separation edge between a CO₂ laser source and an ultra short pulse laser source. It is to be appreciated that a laser source severs or cuts the substrate when the laser source alone separates the substrate into a first portion and a second portion along a cut edge. A laser source imparts a line of weakness to a substrate when as the laser source imparts the line of weakness fibers remain connected along the line of weakness and an additional step of separating the substrate along the line of weakness is required to separate the substrate into a first portion and a second portion. The type of laser source used to cut, also referred herein as sever, or impart a line of weakness to the nonwoven substrate 202 results in the edge having relatively different characteristics. As described above, a separation edge including less accumulated bulbs and less cluster is more preferable.

Figures 9A-9D illustrate a separation edge of a substrate 301. More specifically, the substrate 301 is a nonwoven substrate 202 including a first layer and a second layer of nonwoven material and each layer having a basis weight from about 10 g/m² to about 17 g/m². The nonwoven substrate 202 was intended to be cut by a 600W CO₂ laser source that was operated at 360W or 60% of the total power. The nonwoven substrate 202 was advanced in a machine direction at 8 m/s while undergoing cutting by the CO₂ laser source.

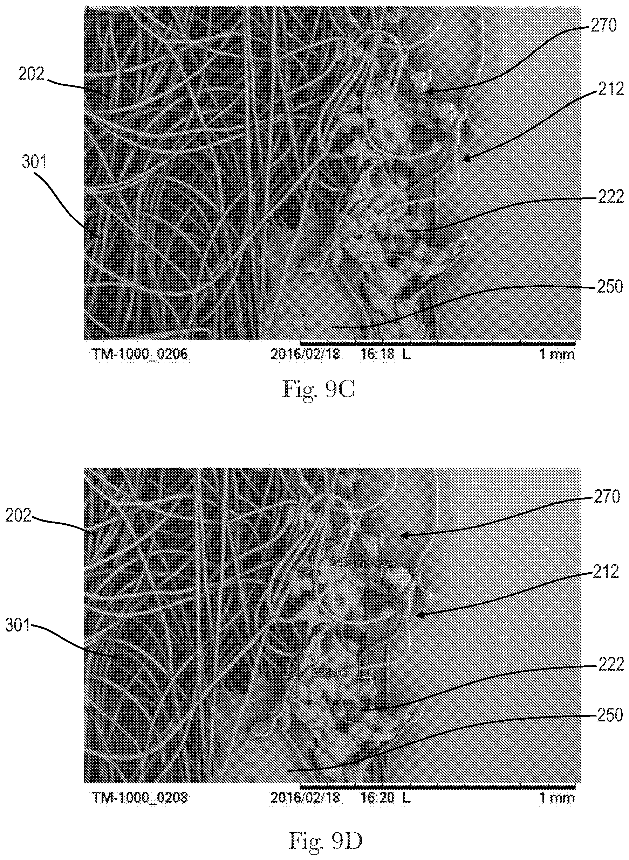

The substrate 301 was cut forming a cut edge 212. Figures 9A-9D illustrate the characteristics of the separation edge 212 after the substrate 301 was cut with a laser source operating at 60% of its total power capacity and the trim was removed. Figures 9A and 9B are a top view of the nonwoven substrate 202 at a first location. As illustrated in Figures 9A and 9B, the separation edge 212 includes a heat modified zone 270 including one or more clusters 222. Figures 9C and 9B are a top view of the nonwoven substrate at a second location. As illustrated in Figures 9C and 9B, the cut edge 212 includes a heat modified zone 270 including one or more clusters 222. These clusters of accumulated material makes that cut edge feel relatively rough and/or coarse. It is to be appreciated that the nonwoven substrate 202 may also include a bond site 250. The bond site 250 is not considered a part of the heat modified zone. However, during cutting, a bond site 250 may be acted upon by the laser source and the heat modified zone may pass through a portion of the bond site. Thus, a bond site 250 that is acted on by the laser source may include a portion of the heat modified zone.

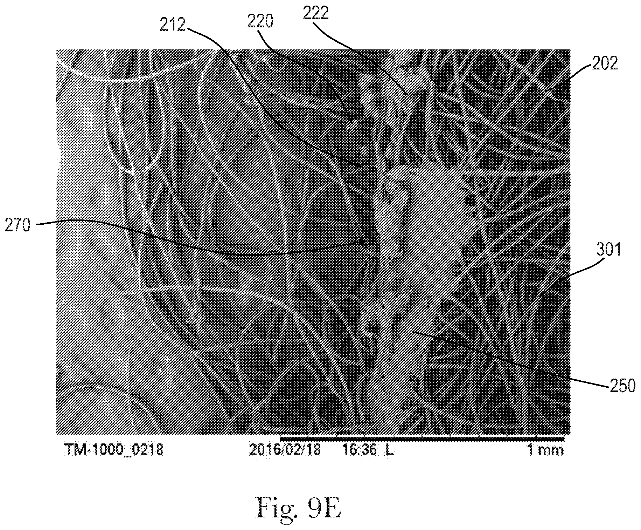

Figures 9E-9G illustrate a separation edge of a substrate 301. More specifically, the substrate 301 is a nonwoven substrate 202 including a first layer and a second layer of nonwoven material and each layer having a basis weight from about 10 g/m² to about 17 g/m². The nonwoven substrate 202 was cut by a 600W CO₂ laser source that was operated at 240W or 40% of the total power and underwent separation by a trim removal member. The nonwoven substrate 202 was advanced in a machine direction at 8 m/s while undergoing cutting by the CO₂ laser source.

The substrate 301 was cut forming a cut edge 212. Figures 9E-9G illustrate the characteristics of the separation edge 212 after the substrate 301 was cut with a laser source operating at 40% of its total power capacity. Figures 9E-9G are a top view of the nonwoven substrate 202 at a first location. As illustrated in Figures 9E-9G, the separation edge 212 includes a heat modified zone 270 including one or more clusters 222 and one or more accumulation bulbs 220. These clusters and accumulation bulbs of material make that cut edge feel relatively rough and/or coarse. It is to be appreciated that the nonwoven substrate 202 may also include a bond site 250. The bond site 250 is not considered a heat modified zone. However, during cutting, a bond site 250 may be acted upon by the laser source and the heat modified zone may pass through a portion of the bond site, such as illustrated in Figure 9E. As illustrated in Figure 9G, the size of the cluster 222 is decreased in comparison to the cluster 222 illustrated in Figure 9D. This is likely due in part to the reduction in the power of the laser source when preforming the cut edge. However, due to the number of displaced fibers illustrated in Figures 9E-9G, the edge may have undergone some tearing during the trim removal process. Thus, the nonwoven substrate 202 may not have been completely separated by the laser source.

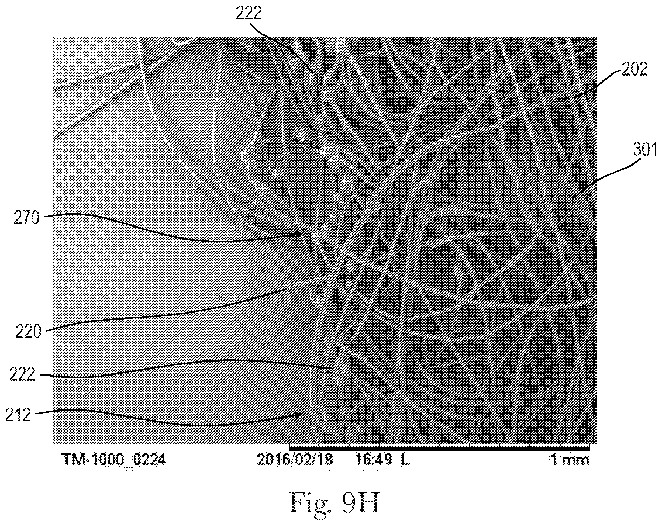

Figures 9H-9J illustrate a separation edge of a substrate 301. More specifically, the substrate 301 is a nonwoven substrate 202 including a first layer and a second layer of nonwoven material and each layer having a basis weight from about 10 g/m² to about 17 g/m². The nonwoven substrate 202 was cut by a 600W CO₂ laser source that was operated at 210W or 30% of the total power and underwent separation of the trim by a trim removal member. The nonwoven substrate 202 was advanced in a machine direction at 8 m/s while undergoing cutting by the CO₂ laser source.

The substrate 301 was cut forming a cut edge. Figures 9H-9J illustrate the characteristics of the separation edge 212 after the substrate 301 was cut with a laser source operating at 30% of its total power capacity. Figures 9H-9J are a top view of the nonwoven substrate 202 at a first location. As illustrated in Figures 9H-9J, the separation edge 212 includes a heat modified zone 270 including one or more clusters 222 and one or more accumulation bulbs 220. These clusters and accumulation bulbs of material make that cut edge feel relatively rough and/or coarse. However, as illustrated in Figure 9J, the size of the accumulation bulb 220 is decreased in comparison to the clusters 222 illustrated in Figures 9G and 9D. This is likely due in part to the reduction in the power of the laser source when preforming the cut edge. However, due to the number of displaced fibers illustrated in Figures 9H-9J, the edge may have undergone some tearing during the trim removal process. Thus, the nonwoven substrate 202 may not have been completely separated by the laser source.

In summary, as illustrated in Figures 9A-9J, the heat modified zone was reduced as the power of the laser source was reduced. The smaller heat modified zone, or, stated another way, the heat modified zone including smaller clusters and/or accumulation bulbs produces a relatively softer, more consumer acceptable edge. However, it is desirable to minimize the heat affected zone. As previously stated, in comparison to the aforementioned, it is desirable to have component parts, such as substrates, that are considered to be soft, smooth, and/or non-irritating for use in absorbent articles and that are easily and efficiently processed. Thus, to solve the aforementioned problems, an ultra short pulse laser source may be used to cut the nonwoven substrate 301.

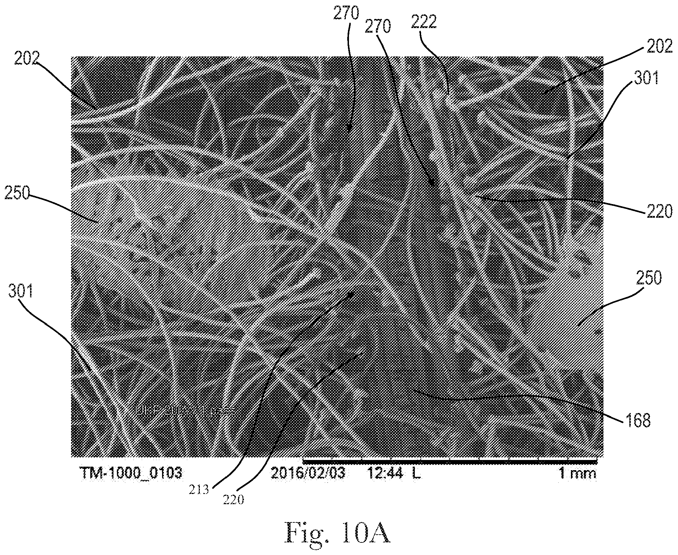

Figures 10A-10I illustrate the characteristics of the cut edge 213 after undergoing cutting by an ultra short pulse laser source. The substrate 301 is a nonwoven substrate 202 including a first layer and a second layer of nonwoven material and each layer having a basis weight from about 10 g/m² to about 17 g/m². The nonwoven substrate 202 was advanced in a machine direction at about 10 m/s while undergoing cutting by the ultra short pulse laser source. It is to be appreciated that the laser source may be operated at various levels of total power output.

Figures 10A-10C illustrate a cut edge 213 imparted by an ultra short pulse laser source operating at 200W. As shown, the nonwoven substrate 202 is severed into a first portion and a second portion, and each portion includes a cut edge 213. The cut edge 213 includes a heat modified zone 270 including one or more accumulation bulbs 220 and one or more clusters 222. Figure 10A illustrates the greater number of accumulation bulbs 220 in comparison to the number of clusters. Further, Figures 10B and 10C illustrate a portion of the cut edge 213 including a detailed view of the accumulation bulbs 220. As illustrated in Figure 10C, the accumulation bulb 220 is less than three times the size of the individual fiber.

It is to be appreciated that the ultra short pulse laser source may not sever the elastic strands 168, as illustrated in Figure 10A. An additional laser source may be used to sever the elastic strands, such as a CO₂ laser source or an ultra short pulse laser source operating at a higher power.

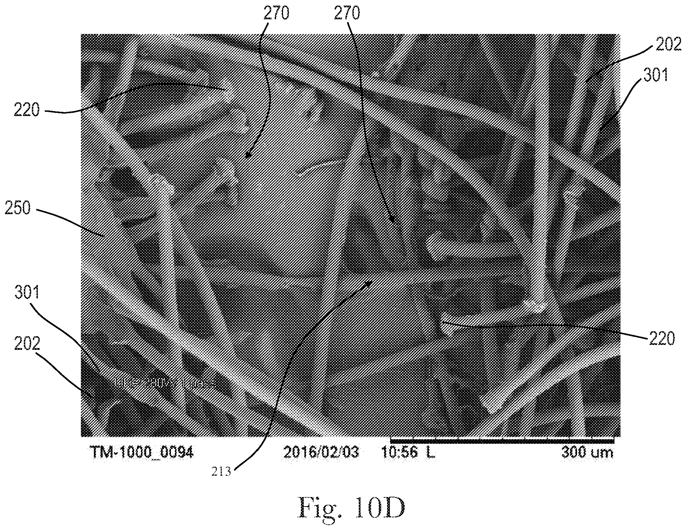

Figures 10D-10F illustrate a cut edge 213 imparted by an ultra short pulse laser source operating at 280W. As shown, the nonwoven substrate 202 is severed into a first portion and a second portion, and each portion includes a cut edge 213. The cut edge 213 includes a heat modified zone 270 including one or more accumulation bulbs 220 and one or more clusters 222. Figures 10E and 10F illustrate a portion of the cute edge 213 including a detailed view of the accumulation bulbs 220. As illustrated in Figure 10F, the accumulation bulb 220 is about two times the size of the individual fiber.

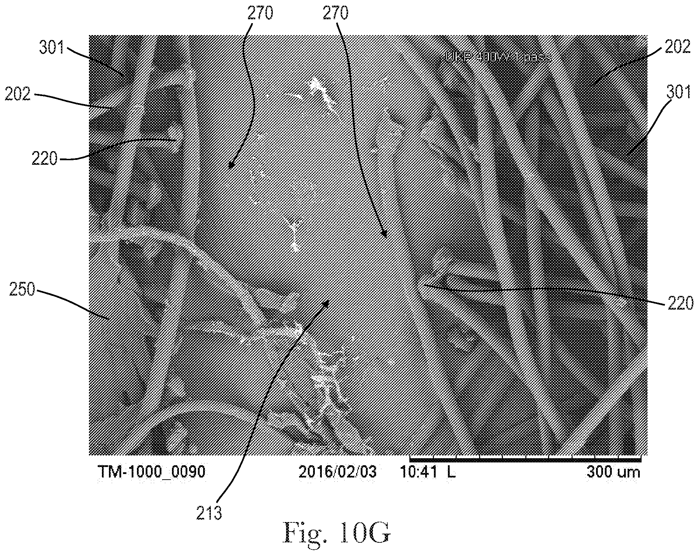

Figures 10G-10I illustrate a cut edge 212 imparted by an ultra short pulse laser source operating at 400W. As shown, the nonwoven substrate 202 is severed into a first portion and a second portion, and each portion includes a cut edge 213. The cut edge 213 includes a heat modified zone 270 including one or more accumulation bulbs 220. Figures 10H and 10I illustrate a portion of the cute edge 213 including a detailed view of the accumulation bulbs 220. As illustrated in Figure 10I, the accumulation bulb 220 is less than about two times the size of the individual fiber.

As evidenced by the Figures, the cut edge 212 formed by the ultra short pulse laser source includes less material accumulation than the cut edge 212 formed by the CO₂ laser source. The reduction in material accumulation leads to the edge being perceived as relatively soft and/or smooth. An edge with less material accumulation is more consumer acceptable. Further, the ultra short pulse laser allows manufacturers to create cut edges having various profiles and shapes and to manufacture various sized products using the same laser source.

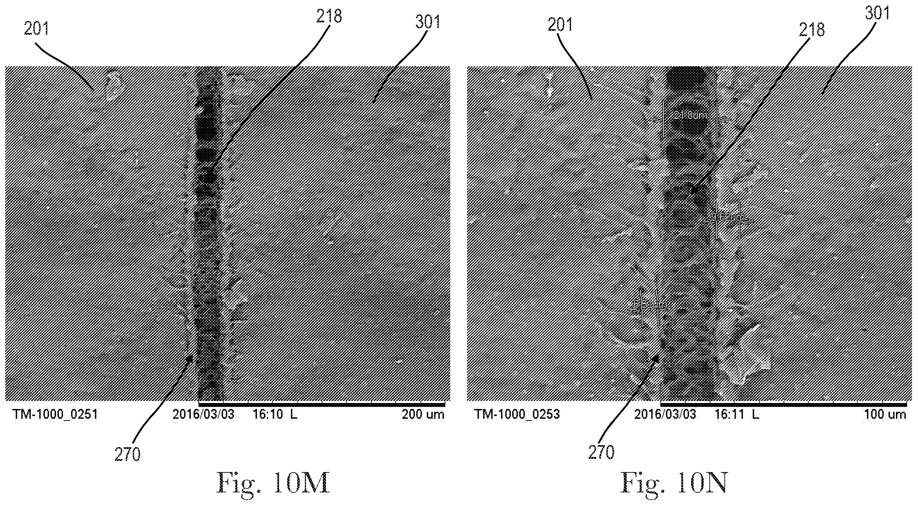

To further reduce the heat modified zone, the ultra short pulse laser source may impart a line of weakness into substrate rather than cutting a continuous line through the entire substrate. It is believed that imparting a line of weakness into the substrate may further improve the edge quality. For example, by imparting a line of weakness and separating the substrate along the line of weakness it is believed that a greater number of fibers will tear and create free fiber ends, which may contribute to the soft feeling of the separation edge 212. Further, by imparting a line of weakness into the substrate, the number of clusters may be reduced or eliminated and/or the change in properties of the substrate may be reduced as compared to cutting which also is believed to contribute to the soft feeling of the separation edge 212. Thus, to create an acceptable edge for consumer products, the ultra short pulse laser source may impart a line of weakness into the substrate and the substrate may be separated as will be described herein. Figures 10J-10L illustrate a nonwoven substrate into which an ultra short pulse laser imparted a line of weakness. Further, Figures 10M and 10N illustrate a film substrate into which an ultra short pulse laser imparted a line of weakness.

The present disclosure relates to a method and apparatus to overcome the aforementioned deficiencies while utilizing an ultra short pulse laser source, and to manufacture a substrate and/or other component parts that are perceived to be softer and/or smoother as compared to a similar substrate and/or other component parts that have undergone cutting by a longer pulse laser source.

It has been found that the heat modified zone may be minimized by using an ultra short pulse laser. The ultra short pulse laser may be pulsed at a frequency from about 100 kHz to about 100 MHz for a pulse duration from about 5 femtoseconds to about 10 picoseconds. Further, the ultra short pulse laser includes a peak energy from about 20 µJ to about 875 µJ. The exact operating parameters depend in part on the material proprieties of the substrate, such as the thickness, density, material makeup (i.e. nonwoven, film, laminate), and chemical additives. In some embodiments, using an ultra short pulse laser to impart a line of weakness into the substrate to form a separation edge may produce a heat modified zone having a maximum width that is less than about 200 microns or less than about 100 microns or less than about 50 microns or less than about 20 microns. The maximum width of the heat modified zone is measured from the edge in a direction perpendicular to the separation edge toward a central region of the substrate. In some embodiments, using an ultra short pulse laser to impart a line of weakness into the substrate to form a separation edge may produce a heat modified zone including a cluster and/or an accumulation bulb. As previously discussed, a cluster results from fibers that have been affected by the laser resulting in one or more fibers melting and joining together. Clusters may be formed from two or more fibers that have melted and joined together. Each cluster and accumulation bulb has a maximum linear length. The maximum linear length is measured according to the Nonwoven Substrate Edge Quality test method disclosed herein. The maximum linear length of a cluster may be less than about 200 µm and/or less than about 150 µm and/or less than about 100 µm and/or less than about 80 µm and/or less than about 50 µm and/or less than about 30 µm. The separation edge may also include a number of clusters per centimeter (cm). An ultra short pulse laser may be used, in part, to produce a separation edge having less than 1 cluster/cm. The separation edge may also include a Free Fiber End value, which may be greater than 1 for a separation edge produced, in part, with an ultra short pulse laser source. Further, as previously discussed, an ultra short pulse laser may produce a separation edge that is less rough than a cut edge produced by a longer pulse laser source. Further, with respect to films, an ultra short pulse laser source may produce a separation edge having a Distortion Ratio that is greater than the Distortion Ratio for a cut edge or separation edge produced by a longer pulse laser source, such as a CO₂ laser source.

The following description relates to processes and apparatuses that use a laser source to impart a line of weakness into a substrate. It is to be appreciated that the laser source used in the following description may be an ultra short pulse laser source.

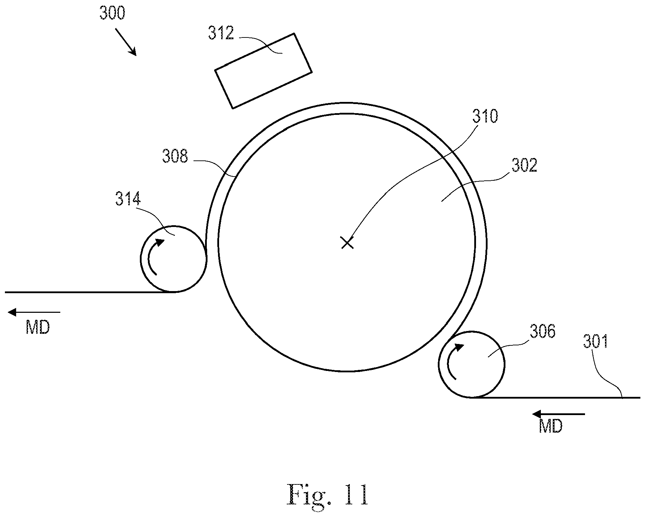

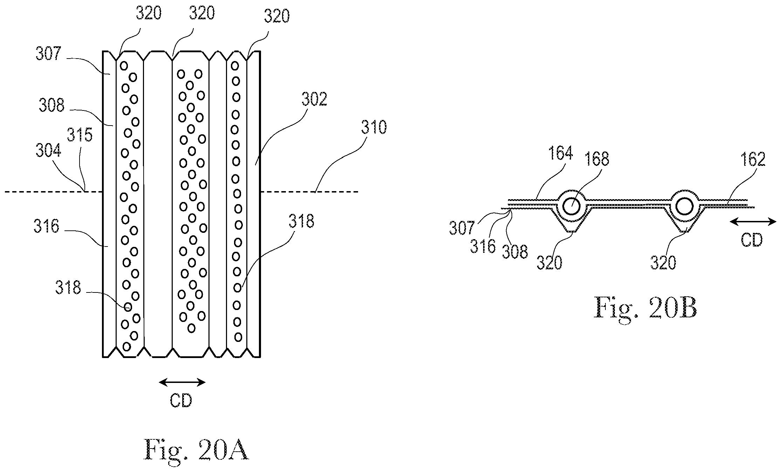

Figures 11, 12 and 13 illustrates an exemplary schematic representation of an apparatus 300 that may be used to impart a line of weakness into a substrate 301. As illustrated in Figure 11, the apparatus 300 may include a process member 302. The process member 302 may rotate about a longitudinal axis of rotation 310. Further, the process member 302 may be configured to receive the substrate 301. It is to be appreciated that substrate may include a nonwoven, film, laminate or other material as discussed herein. The substrate 301 may advance in a machine direction MD toward the process member 302. A first guide roller 306 may aid in the transfer of the substrate 301 onto an outer circumferential surface 308 of the process member 302. The outer circumferential surface 308 of the process member 302 may include one or more apertures, as illustrated in Figure 20A. A vacuum source, not shown, may be in fluid communication with the one or more apertures. The vacuum source allows fluid to be circulated through the one or more apertures toward the longitudinal axis of rotation 310 of the process member 302. The movement of fluid may result in the substrate 301 being forced toward the outer circumferential surface 308 of the process member 302. The process member 302 may rotate about the longitudinal axis of rotation 310 causing the substrate 301 to advance toward an ultra short pulse laser source 312. The ultra short pulse laser source 312 may emit a laser beam that is used to impart a line of weakness into the substrate 301. The substrate 301 including the line of weakness may advance to additional processes such as separating the first substrate portion from the second substrate portion and/or adding additional components to the substrate 301, such as in forming an absorbent article. The process member 302 may include a pressure source (not shown) that transfers a gas and/or fluid through the one or more apertures 318 causing the substrate 301 to be forced away from the outer circumferential surface 308 of the process member 302. A second guide roller 314 may be used to advance the substrate 301 to these subsequent processes and/or to aid in the subsequent processes.

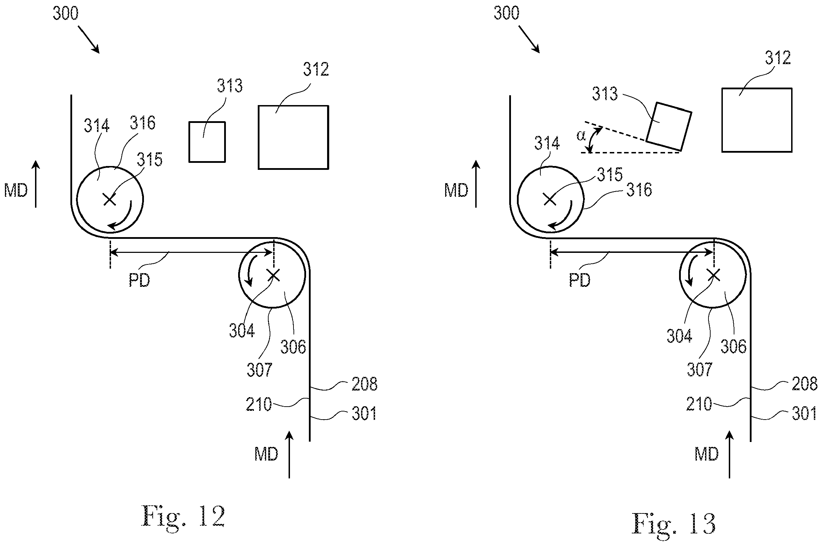

Figure 12 illustrates an exemplary schematic representation of an apparatus 300 that may be used to impart a line of weakness into a substrate 301. The apparatus 300 may include a first guide roller 306. The first guide roller 306 may rotate about a first axis of rotation 304. The first guide roller 306 may be driven by a motor or may rotate freely about the first axis of rotation 304. Further, the first guide roller 306 may be configured to receive the substrate 301. It is to be appreciated that a substrate is used to describe the process and apparatus herein, but any film, laminate, multiple layer substrate, and/or other absorbent article component, as previously discussed, may be used in the process and apparatus discussed herein. The substrate 301 may include a first surface 208 and a second surface 210, opposite the first surface 208. These surfaces may be referred to herein as a garment facing layer 162 and a wearer facing layer 164. The substrate 301 may advance in a machine direction MD toward the first guide roller 306. The substrate 301 may be disposed on a portion of an outer circumferential surface 307 of the first guide roller 306. More specifically, at least one of the first surface 208 or the second surface 210 of the substrate 301 may be disposed on the outer circumferential surface 307 of the first guide roller 306.

The first guide roller 306 may rotate about the first axis of rotation 304 resulting in the substrate 301 advancing toward at an ultra short pulse laser source 312. The first laser source 312 may be used to cut the substrate 301. More specifically, the laser source 312 may transmit a first laser beam to a first scan head 313. The scan head 313 may direct the first laser beam such that the first laser beam engages the substrate. A line of weakness may be imparted to the substrate 301.

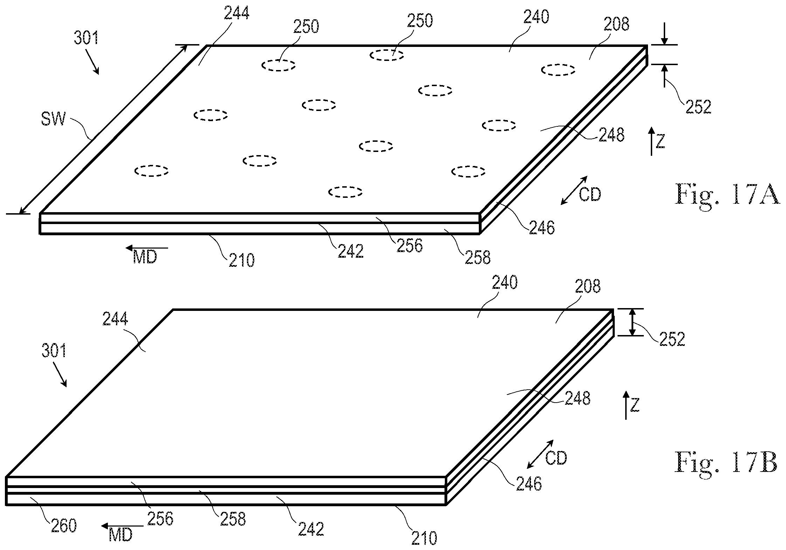

The substrate 301 may be advanced to a second guide roller 314. The portion of the substrate 301 positioned between the first guide roller 302 and the second guide roller 314 is referred to herein as the unsupported portion. The unsupported portion is the area of the substrate 301 on which the laser beam may affect the substrate 301. The distance between the first guide roll 306 and the second guide roll 314 is referred to herein as the process distance PD. The process distance PD may be such that the unsupported portion of the belt assembly remains substantially taut as the cut is imparted by the laser beam. The process distance PD may be any distance that holds the substrate in the desired position and allows the laser beam to impart the line of weakness into the substrate in the desired location. In some embodiments, for example, the process distance PD may be less than about 3 times the substrate width SW and/or less than about 2 times the substrate width SW and/or less than about the substrate width SW and/or less than about 0.5 times the substrate width SW and/or less than about 0.25 times the substrate width SW. The substrate width SW, as illustrated in Figures 17A, is the width extending parallel to the cross direction CD from each outside edge of the belt assembly 204.

The second guide roller 314 may be used to advance the substrate 301 to one or more subsequent processes and/or to maintain the tension and/or position of the substrate. The second guide roller 314 may rotate about a second axis of rotation 315. The second guide roller 314 may be driven by a motor or may rotate freely about the second axis of rotation 315. The substrate 310 may be disposed about an outer circumferential surface 316 of the second guide roller 314. The first surface 208 of the substrate 301 may be in facing relationship with the outer circumferential surface 316 of the second guide roller 314. Facing relationship may include items that are directly next to one another or items that are separated by one or more additional items. For example, the first surface 208 may be positioned in facing relationship with the outer circumferential surface 316 such that the first surface 208 is in contact with the outer circumferential surface 316. The first surface 208 may be positioned in facing relationship with the outer circumferential surface 316 such that an intermediate substrate layer is positioned between the first surface 208 and the outer circumferential surface 316. This process and apparatus will be described in more detail herein. The substrate 301 including the line of weakness may advance to additional processes such as separating the first portion from the second portion and/or adding additional components to the substrate 301.

It is to be appreciated that the substrate may be positioned such that the first surface of the substrate engages the outer circumferential surface of the first guide roller and the second surface of the substrate engages the outer circumferential surface of the second guide roller.

As illustrated in Figure 13, the first scan head 313 may be oriented at an angle. The first scan head 313 may be at a first angle α with respect to the machine direction MD or the first planar surface of the substrate. The first angle may be from 0 degrees to about 20 degrees and/or from about 2 degrees to about 15 degrees and/or from about 5 degrees to about 10 degrees, including all 0.1 increment therebetween. The angle of the scan head may aid in imparting the line of weakness into the substrate 301. However, it is to be appreciated that it is not necessary that the scan head be at an angle. The scan head 313 may be positioned substantially perpendicular to the machine direction MD and/or a surface of the substrate, as illustrated in Figure 12.

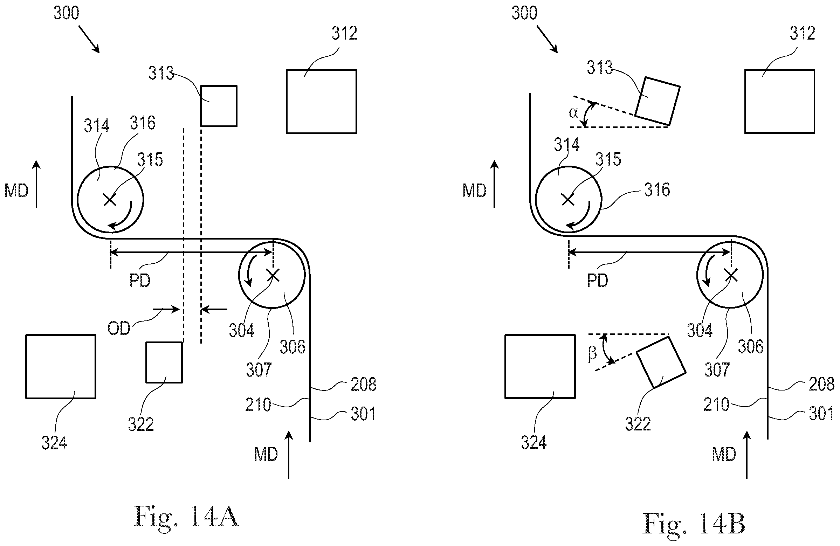

In some embodiments, the substrate 301 may include greater than two layers or may be of such thickness that using two laser sources to emit two laser beams or a single laser source that is configured to operate with a splitter to emit at least two laser beams may be beneficial to producing a consumer acceptable separation edge. Using two laser sources or more than one laser beam may aid in reducing the heat modified zone of the substrate upon imparting the line of weakness. Referring to Figures 14A and 14B, the first guide roller 306 may rotate about the first axis of rotation 304 resulting in the substrate 301 advancing toward at least one of a first laser source 312 and a second laser source 324. The first laser source 312 and the second laser source 324 may be used to impart lines of weakness into the substrate 301. More specifically, the first laser source 312 may transmit a first laser beam to a first scan head 313. The first scan head 313 may direct the first laser beam such that the first laser beam engages the substrate. Similarly, the second laser source 324 may transmit a second laser beam to a second scan head 322. The second scan head 322 may direct the second laser beam such that the second laser beam engages the substrate. It is to be appreciated that a single laser source may be used to emit both the first laser beam and the second laser beam.

The first scan head 313 may be offset from the second scan head 322 by an offset distance OD. The offset distance OD may be any distance such that the first laser beam does not interfere with the second scan head 322 and/or laser source 324 and the second laser beam does not interfere with the first scan head 313 and/or laser source 312. The offset of the first scan head 313 and the second scan head 322 may prevent the opposing laser beam from potentially being directed back to the laser source through the scan head and causing damage to the laser source and/or the scan head. However, it is to be appreciated that the first scan head 313 and the second scan head 322 need not be offset from one another. In some embodiments, the first scan head 313 may be positioned opposite to the second scan head 322 and each laser source and scan head may be controlled such that there is no effect on the opposite laser source and/or scan head.

The first laser source 312 may supply a first laser beam to the first scan head 313. The first scan head 313 directs the first laser beam such that the first laser beam imparts of line of weakness into the first surface 208 of the substrate 301. The second laser source 324 may supply a second laser beam to the second scan head 322. The second scan head 322 directs the second laser beam such that the second laser beam imparts a line of weakness into the second surface 210 of the substrate 301. The first line of weakness may be coincident with the second line of weakness. The first line of weakness is coincident with the second line of weakness when the first line of weakness and the second line of weakness are separated by a distance less than about 2 mm and/or less than about 1.5 mm and/or less than about 1 mm and/or less than about 0.5 mm, including all 0.1 increments.

As illustrated in Figure 14B, the first scan head 313 and the second scan head 322 may each be at an angle. The first scan head 313 may be at a first angle α with respect to the machine direction MD or the surface of the substrate 301. The first angle may be from 0 degrees to about 20 degrees and/or from about 2 degrees to about 15 degrees and/or from about 5 degrees to about 10 degrees, including all 0.1 increment therebetween. The second scan head 322 may be at a second angle β with respect to the machine direction MD or the surface of the substrate 301. The first angle may be from about 0 degrees to about 20 degrees and/or from about 2 degrees to about 15 degrees and/or from about 5 degrees to about 10 degrees, including all 0.1 increments therebetween. The first scan head 313 and the second scan head 322 may each be positioned at an angle so that the first laser beam does not interfere with the second scan head 322 and/or the second laser source 324 and the second laser beam does not interfere with the first scan head 313 and/or the first laser source 312. It is to be appreciated that it is not necessary that either the first scan head or the second scan head be at an angle. Each of the first scan head 313 and the second scan head 322 may be positioned substantially perpendicular to the machine direction MD and/or a surface of the substrate, as illustrated in Figure 14A, and each laser source and scan head may be controlled such that there is no effect on the opposite laser source and/or scan head.

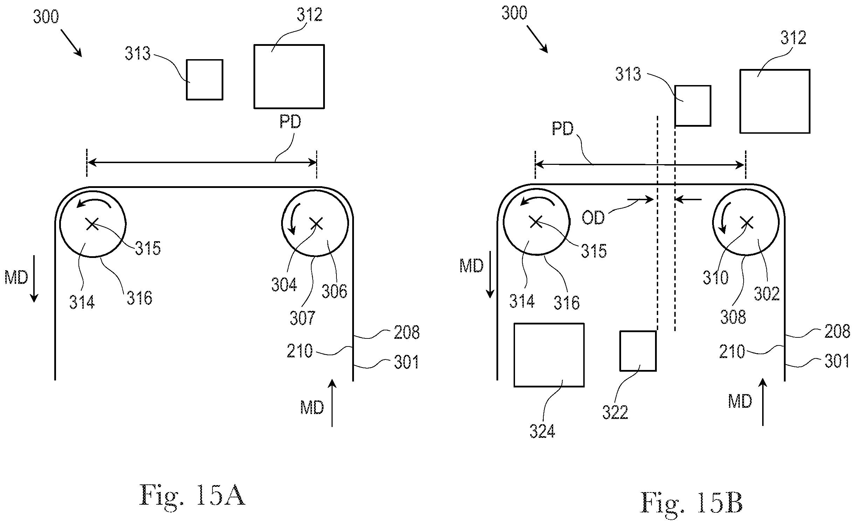

Figures 15A and 15B illustrate another configuration of the guide rollers and their interaction with the substrate 301; the laser sources and their respective scan heads may operate as discussed with respect to Figures 11 through 14B. Thus, a single laser beam or more than one laser beam may machine the substrate 301. As illustrated in Figures 15A and 15B, both the first roller and second roller may interact with a single surface of the substrate. For example, the second surface 210 of the substrate 301 engages a portion of the outer circumferential surface of the first roller and the second roller. The configuration illustrated in Figures 15A and 15B may be beneficial for those substrates which include a substance, such as adhesives, or other material that is desired to remain untouched by process members and/or rollers.

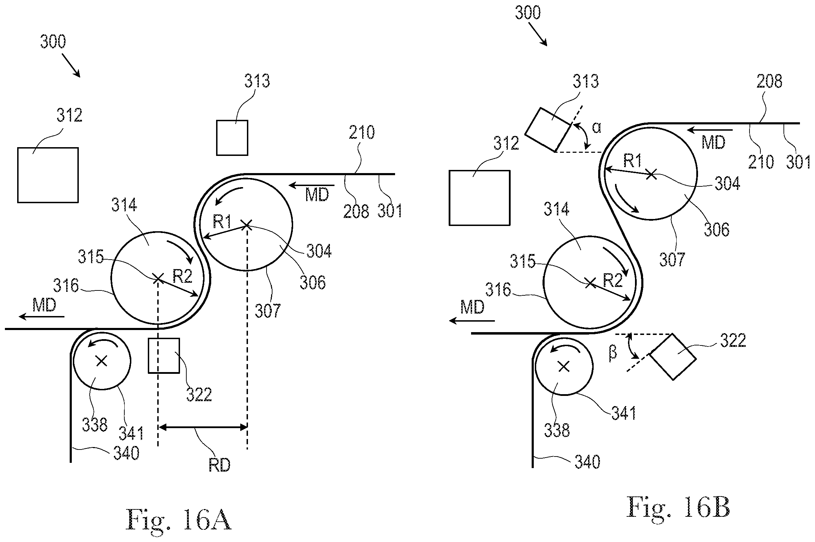

In some embodiments, during processing of the belt assembly 204, the first guide roller 306 and the second guide roller 314 may be positioned adjacent one another, as illustrated in Figures 16A and 16B. This configuration may be referred to herein as an s-wrap configuration. The first guide roller 306 and the second guide roller 314 may be positioned such that each roller is vertically and horizontally offset. The first guide roller 306 may be horizontally offset such that the outer circumferential surface 307 of the first guide roller 306 is parallel to or in an overlapping relationship with a portion of the outer circumferential surface 316 of the second guide roller 314 as illustrated in Figures 16A and 16B. Stated another way, the roller distance RD measured parallel to the machine direction MD between the first axis of rotation 304 of the first guide roller 306 and the second axis of rotation 315 of the second guide roller 314 may be equal to or less than the sum of the radius R1 of the first guide roller 306 and the radius R2 of the second guide roller 314. Either the first surface 208 or the second surface 210 of the substrate 301 may be disposed about each of the first guide roller 306 and the second guide roller 314.

For example, as illustrated in Figure 16A, the substrate 301 may be configured such that the first surface 208 is disposed about the outer circumferential surface 307 of the first guide roller 306 and the second surface 210 is disposed about the outer circumferential surface 316 of the second guide roller 314. Thus, as the first surface 208 is disposed on the outer circumferential surface 307 of the first guide roller 306, the first laser source 312 may emit a laser beam such that the laser beam operatively engages a first scan head 313. The first scan head 313 directs the laser beam at the second surface 210 and may impart a first line of weakness into the second surface 210 of the substrate 301. The substrate 301 may continue to advance in the machine direction MD such that the second surface 210 is disposed on the outer circumferential surface 316 of the second guide roller 314. Thus, as the second surface 210 is disposed on the outer circumferential surface 316 of the second guide roller 314, the first laser source 312 may emit a laser beam such that the laser beam operatively engages a second scan head 322. The second scan head 322 directs the laser beam at the first surface 208 and may impart a second line of weakness to the first surface 208 of the substrate 301. The first line of weakness and the second line of weakness may be coincident or offset. It is to be appreciated that a single laser source and a single laser beam may be used as the substrate traverses through the s-wrap configuration. For example, if the substrate 301 comprises one or more layer that may be adequately processed by a single laser beam and the separation edge exhibits the desired edge quality after the line of weakness is separated, it is not necessary to have a first and second laser beam acting on the substrate 301. However, if the substrate 301 comprises multiple layers or is of relatively great thickness that the edge quality is diminished by using a single laser beam, multiple laser beams may be used to impart a line of weakness into portions of the substrate to form the final separation edge.

As illustrated in Figure 16B, the first guide roller 306 and the second guide roller 314 may be positioned in an s-wrap configuration and the substrate 301 may traverse this configuration such that there is an unsupported portion of the substrate 301 as the substrate 301 advances from the first guide roller 306 to the second guide roller 314. Thus, the laser beam may affect the portion of the substrate disposed on the outer circumferential surface of the guide roller and/or the unsupported portion of the substrate.

Further, as the substrate 310 is transferred from the second guide roller 314, the substrate 301 may engage a trim removal member 338, as illustrated in Figures 16A and 16B. The trim removal member 338 may be configured to engage the trim 340. The trim 340 is either the first substrate portion or the second substrate portion that is desired to be discarded prior to the remainder of the substrate advancing to downstream processes. The trim 340 may be disposed about a portion of the outer circumferential surface 341 of the trim removal member 338 while the remainder of the substrate 310 is advanced to other downstream processes. The trim 340 may be removed immediately after the substrate 310 is cut into a first substrate portion and a second substrate portion or the first and second substrate portions may traverse together to and during subsequent processing. The trim 340 may be removed after several other processes have been performed on either the first or second portion of the substrate.

It is also to be appreciated that one or more additional devices such as a cutting device including a blade, an additional laser source, or another roller may be used to aid in the removal of the trim 340 from the substrate 301.

As the substrate traverses through the processes previously described, the substrate 301 may be placed under tension in at least one of the machine direction MD and the cross direction CD. More specifically, referring to Figure 11, the first roller 306 may rotate such that as the substrate 301 is advanced onto the outer circumferential surface 308 of process member 302, a machine direction tension of at least about 0.5% strain of the material in the machine direction MD is applied to the substrate 301. A machine direction tension of at least about 0.25% strain to about 4% strain of the material may be applied to the substrate. Generally, the tension placed on the material is not greater than the tension that would cause permanent deformation of the material, and the material may be able to recover after the tension is removed from the material. Similarly, as illustrated in Figures 12-16B, the first guide roller 306 and the second guide roller 314 may each be configured to rotate about their respective axes such that a machine direction tension of at least about 0.5% is applied to the substrate 301.

The tension on the substrate 301 may aid in the separation of the substrate 301 once the substrate has been acted on by the laser beam. As described above, as the line of weakness is imparted to the substrate 301, the portion of the substrate adjacent to the line of weakness may undergo some melting. By placing the substrate 301 under tension, the substrate 301 wants to contract once it has been affected by the line of weakness. Thus, the tension aids in separating the substrate into a first substrate portion and a second substrate portion. Providing tension on the substrate 301 prevents the first substrate portion from joining the second substrate portion along the line of weakness while the line of weakness is in a tacky state. It is to be appreciated that either cross direction tension or machine direction tension may aid in separation of the first and second substrate portions along the line of weakness.

In some embodiments, by placing only machine direction tension on the substrate 301, the substrate 301 may be stretched in the machine direction but experience neck down in the cross direction. Stated another way, because the substrate 301 is being stretched in the machine direction, the substrate 301 may contract in the cross direction CD due to the tension in the machine direction MD. Thus, when the substrate 301 undergoes processing, such as imparting a line of weakness, the material may contract in the cross direction, which may result in re-weld of the cut substrate 301. To prevent re-weld due to neck down in the cross direction, cross machine direction tension may be applied to the substrate upon being cut. This cross machine direction tension may be applied through spreading rolls, cross direction grippers, bar, or canted idlers, for example. In some other embodiments, a crowned roll or a roll that has a radius that varies from the center of the cylindrical roll to the ends of the cylindrical roll may be used to prevent re-weld. For example, the radius at the end of the cylindrical roll may be less than the radius at the center of the cylindrical roll. The crowned roll aids in the separation of the first substrate portion from the second substrate portion in the cross machine direction upon being acted on by the laser beam.

The substrate 301 is a material which has a thickness (in a Z direction) that is relatively small in comparison to its length (in an X direction or Machine Direction MD) and width (in a Y direction or cross direction CD). Substrates may include a web, layer or layers of fibrous materials, nonwovens, films, and foils such as polymeric films or metallic foils. These materials may be used alone or may comprise two or more layers joined together. The substrate 301 may be a single layer of fibrous material, nonwoven, film, and/or foil. The substrate 301 may also include multiple layers, as illustrated in Figures 17A and 17B.

As illustrated in Figure 17A, the substrate 301 includes a first edge portion 240 extending in the machine direction MD and a second edge portion 242 opposite the first end portion 240 and extending in the machine direction MD. Between the first edge portion 240 and the second edge portion 242 is a central portion 248. The substrate 301 may also include a leading edge portion 244 that extends in the cross direction CD and is first to advance to the laser as the substrate traverses in the machine direction MD. The substrate 301 may also include a trailing edge portion 246 opposite the leading edge portion 244. The trailing edge portion 246 follows the leading edge portion 244 as the substrate 301 traverses in the machine direction MD. It is to be appreciated that a physical edge may not define the trailing edge portion and the leading edge portion. For example, for absorbent articles, a product pitch may delineate a leading edge portion and a trailing edge portion. A product pitch is the length in a direction parallel to the machine direction MD of a single article or product defined on the substrate 301. Further, as illustrated in Figure 17A, the substrate may include a first substrate layer 256 and a second substrate layer 258. The first and second substrate layers 256, 258 may be any material as previously specified. Further, each of the first substrate layer 256 and the second substrate layer 258 may have a layer thickness. However, the substrate thickness 252 is the thickness of the combined layers of the substrate 301.

A substrate 301 having one or more layers may be bonded. For example, as illustrated in Figure 17A, the first substrate layer and the second substrate layer may be bonded at bond sites 250. The number and type of bonds may be determined based on the materials to be bonded. It is also to be appreciated that a substrate 301 having more than one layer need not be bonded. One substrate layer may be disposed on the other substrate layer without attaching the two substrate layers. It is also to be appreciated that a single substrate layer may also include bond sites. For example, for nonwoven substrate, it may be necessary to use bond sites to hold the individual fibers of the nonwoven together, which makes the nonwoven a web that can be processed. A substrate may be joined using several bonding methods which include, for example, heat, ultrasonic, or pressure bonds.

As illustrated in Figure 17B, the substrate 301 may include a first substrate layer 256, a second substrate layer 258, and a third substrate layer 260. The second substrate layer 258 may be disposed intermediate the first substrate layer 256 and the third substrate layer 260. In some embodiments, the first substrate layer 256 and the third substrate layer 260 may be nonwovens and the second substrate layer 258 may be a film. It is also to be appreciated that the substrate 301 may include a first substrate layer which is a film and a second substrate layer which is a nonwoven. For example, a backsheet, used in absorbent articles, may include a film layer and a nonwoven layer.

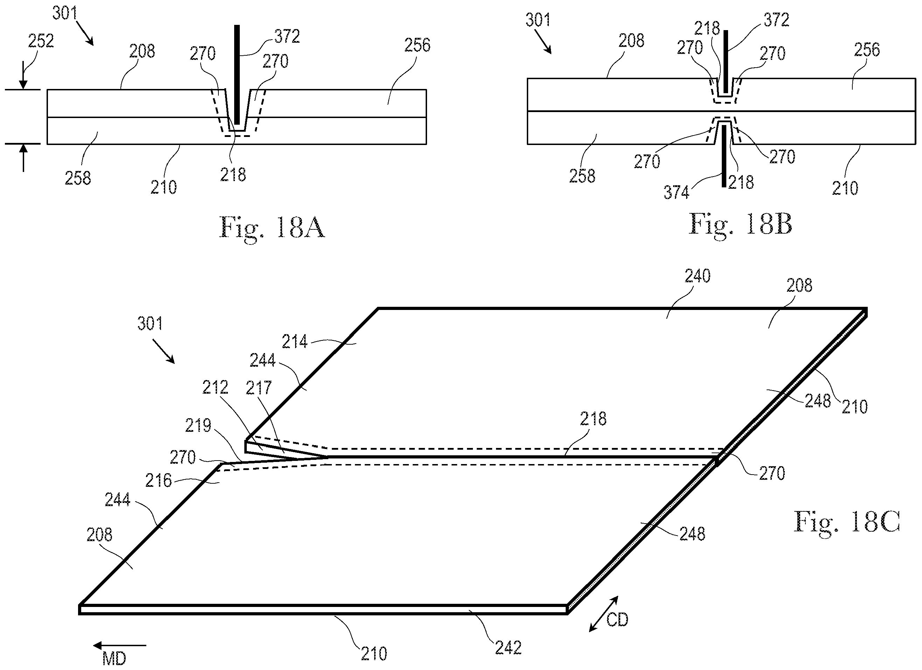

Figures 18A and 18B illustrate example schematic representations of a laser beam or laser beams interacting with a substrate 301. As previously described, a single laser source may be used to emit a single laser beam that imparts a line of weakness to the substrate 301. Similarly, a single laser source or at least two laser sources may be used such that more than a single laser beam acts on the substrate 301 to impart one or more lines of weakness into the substrate 301. For example, a first and second laser beam may be used to impart a first and second line of weakness into the substrate 301. Using more than one laser source on the substrate may result in a smaller heat modified zone, due to the use of less power to weaken the substrate, and a relatively softer separation edge. As illustrated in Figure 18A, a single laser beam 372 may be used to impart a line of weakness into the substrate 301. The single laser source 372 may be used to affect the entire substrate thickness 252, which is illustrated as a first substrate layer 256 and a second substrate layer 258. When a single laser beam is used to affect the substrate 301, the laser beam 372 may first engage the first surface 208, or the surface of the substrate in facing relationship with the laser beam. The laser beam 372 imparts a line of weakness into the first substrate layer 256 and subsequently through the second substrate layer 258. The heat modified zone occurs perpendicular to the direction of the laser beam. Further, the heat modified zone may vary throughout the thickness of the substrate 301. For example, as illustrated in Figure 18A, the heat modified zone may have a diminishing profile. The heat modified zone may be greatest at the first surface 208 of the substrate 301 because this is the portion of the substrate that may be affected by the laser the longest and, thus, gives the heat generated by the laser beam the greatest time to penetrate the substrate. However, as the laser beam imparts the line of weakness into the substrate, the time the laser beam interacts with the substrate decreases, which results in a decreased amount of time for the heat to penetrate through the substrate. It is to be appreciated that for an ultra short pulse laser the time the laser beam dwells on the material is very short and, thus, the heat has minimal time to dissipate. Thus, the diminishing profile of the heat modified zone may be unidentifiable and appear as though it is parallel to the direction of the laser beam.

As illustrated in Figure 18B, a first laser beam 372 and a second laser beam 374 may be used to impart a first line of weakness and a second line of weakness into the substrate 301. The first laser beam 372 may first engage the first layer 256 and the first surface 208 of the substrate 301 and the second laser source 374 may first engage the second layer 258 and the second surface 210 of the substrate 301. The first laser beam 372 may weaken a portion of the first substrate 256 and the second laser beam 374 may weaken a portion of the second substrate 258. For a multiple layer substrate, the first laser beam 372 may weaken a portion of the first substrate 256 or cut through the first substrate 256 and the second laser beam 374 may weaken a portion of the second substrate 256 such that fibers of the second substrate remain attached. Stated another way, the first laser beam 372 may cut through the first substrate layer 256 only and the second laser beam 374 may impart a line of weakness into the second substrate layer 258. In some other embodiments, the first laser beam 372 may impart a line of weakness into the first substrate layer 256 and the second laser source 374 may cut through the second substrate layer 258. The first laser beam 372 and the second laser beam 374 work together to affect the substrate 301. Thus, the first laser beam 372 and the second laser beam 374 may each weaken a certain amount of the substrate 301 so long as fibers of the substrate 301 remain attached after the lasers have acted on the substrate 301. The line of weakness imparted by the first laser beam 372 may be coincident with the line of weakness imparted by the second laser beam 374, as illustrated in Figure 18B. The line of weakness imparted by the first laser beam 372 may also be offset from the line of weakness imparted by the second laser beam 374.

It is also to be appreciated that the line of weakness imparted by the first laser beam and the line of weakness imparted by the second laser beam may be imparted to the substrate at two different points in time or at the same time. For example, in some embodiments, the first laser beam may impart a first line of weakness into the substrate and, subsequently, the second laser beam may impart a second line of weakness into the substrate. By using two laser sources to weaken the substrate layer(s), the heat modified zone produced at the separation edge may be minimized and, thus, the substrate edge may be perceived as softer feeling and more appealing to consumers. The heat modified zone may be smaller when using two laser beams as compared to a single laser beam to weaken the substrate. As previously described, the heat modified zone may be proportional to the time the laser beam acts on the substrate and, thus, the time the heat has to dissipate into the material. Since using two laser sources may allow each laser beam to interact for a short period of time with the material, the heat modified zone may be smaller and, thus, a more desirable separation edge may be produced. It is also to be appreciated that by using two laser sources, each laser source may operate at a lower power which may also contribute to minimizing the heat modified zone.

The substrate 301 includes one of more lines of weakness 218. The substrate 301 including the line of weakness may undergo one or more processes, such as a trim removal process, to separate the substrate 301 along a separation edge 212, as illustrated in Figure 18C. The substrate 301 separates along a separation edge 212 into one or more portions. For example, a substrate 301 is separated along a separation edge 212 into a first substrate portion 214 and a second substrate portion 216. Each of the first substrate portion 214 and the second substrate portion 216 may include a first surface 208 and a second surface 210 opposite the first surface 208. Further, the first substrate portion 214 may include a first separation edge 217. Similarly, the second substrate portion 216 may include a second separation edge 219. The first and second separation edges 217, 219 are formed by the separation edge 212 upon separating the substrate along the line of weakness. Each separation edge 212 may be linear, non-linear, or any desired shape. For example, each of the first and second separation edges may form a straight line, a curved line, a circle, an oval, a square, a rectangle, a parallelogram, a hexagon, or other shape. The line of weakness and, thus, the separation edge 212 may be continuous or discrete. A continuous line of weakness is one where the line of weakness is perceived to be continuous, without end, over at least two product pitches. A discrete line of weakness is one where the line of weakness begins and ends within no more than two product pitches. Examples of these types of lines of weakness will be discussed in greater detail herein.

It is to be appreciated that a substrate may include both discrete and continuous lines of weakness. For example, a substrate may include a first product pitch extending parallel to the machine direction MD and a second product pitch adjacent to the first product pitch and extending parallel to the machine direction. The laser beam acts on the substrate to form a line of weakness. The line of weakness may include a first portion and a second portion. The first portion may be disposed within the first product pitch and the second portion may be disposed within the second product pitch. The first portion may be formed from a discrete or continuous line of weakness. Similarly, the second portion may be formed from a discrete or continuous line of weakness. Due to the versatility of the laser beam, a substrate may include numerous product pitches each including a different line of weakness pattern. Product pitches which are positioned adjacent one another may include different line of weakness patterns. However, for large scale manufacturing the line of weakness may be the same or similar for a span of product pitches.

As illustrated in Figure 17A, the substrate 301 may include one or more bond sites. It is to be appreciated that the laser may impart a line of weakness through the one or more bond sites. A bond site should not to be considered a cluster of material. A cluster of material results from the laser beam acting on the substrate and is considered part of a heat modified zone. A cluster may form along a portion of the bond site that has been affected by the laser source.

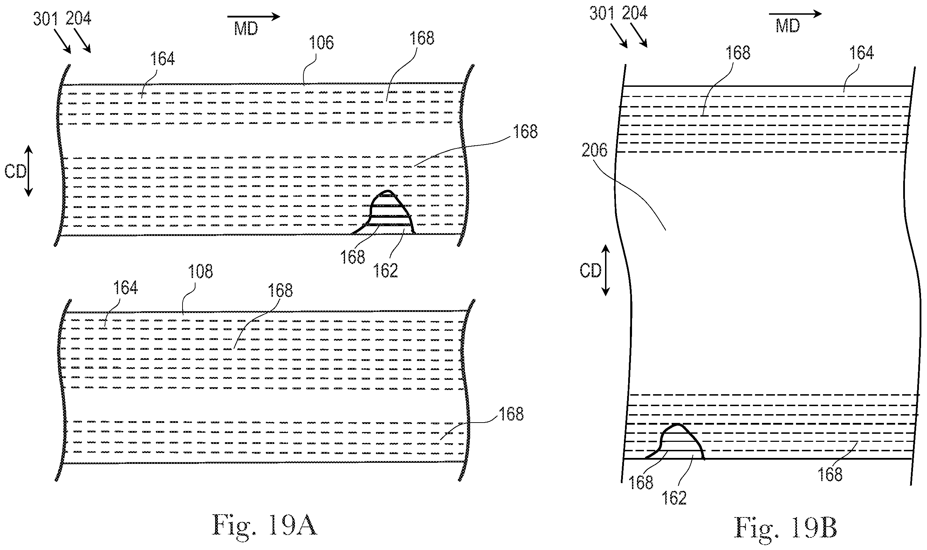

The substrate 301 may be any material as discussed. The following is an example of a substrate 301 used in absorbent articles. The substrate 301 may be a belt assembly 204, as illustrated in Figures 19A and 19B. The belt assembly 204 may include a first belt 106 and a second belt 108, as illustrated in Figure 19A. The first belt 106 and the second belt 108 may be spaced such that an absorbent core or other discrete component may be disposed across a portion of the first belt 106 and the second belt 108. The first belt 106 and the second belt 108 may each include an outer layer 162, an inner layer 164 disposed in facing relationship with the outer layer 162, and elastic strands 168 disposed between the outer layer 162 and the inner layer 164. The elastic strands 168 may be stretched in the machine direction MD and bonded with the first substrate layer 162 and/or the second substrate layer 164. More particularly, the elastic strands 168 may be continuously bonded with the first substrate layer 164 and/or the second substrate layer 162 with adhesive along the machine direction MD and/or the elastic strands 168 may be intermittently bonded with the first substrate layer 162 and/or the second substrate layer 164 with adhesive along the machine direction MD. Thus, the elastic strands 168 may include non-bonded regions along the machine direction MD. The elastic strands are not bonded to either of the first substrate 162 or the second substrate 164 in the non-bonded region. It is to be appreciated that the first and second substrates 162, 164 may also be joined, such as by bonding, between the elastic strands 168.

In some embodiments, as illustrated in Figure 19B, the belt assembly 204 may include a unitary, body substrate 206. The body substrate 206 may include an outer layer 162, an inner layer 164 disposed in facing relationship with the outer layer 162, and one or more elastics 168 disposed between the outer layer 162 and the inner layer 164.

The belt assembly 204 may advance on to the outer circumferential surface 308 of the process member 302, as illustrated in Figure 11, or the first and second guide rollers 306,314, as illustrated in Figures 12-16B. The belt assembly 204 may be disposed on the outer circumferential surface of process member 302 or the guide rollers such that either the outer layer 162 or the inner layer 164 of the belt assembly 204 is disposed on the outer circumferential surface. More specifically, at least one of the outer layer 162 and the inner layer 164 may be disposed on the outer circumferential surface. It is to be appreciated that the outer layer 162 and the inner layer 164 may each be made up of one or more layers that have different properties, such as the type of fiber, additives, and density. The properties of the outer layer 162 and the properties of the inner layer 164 may make it advantageous to have one layer or the other layer in closer proximity to, or facing relationship with, the laser beam.

It is also to be appreciated that the characteristics of the separation edge may make it advantageous to have either the outer layer 162 or the inner layer 164 in facing relationship with the laser beam. As described herein, the line of weakness of a substrate may include a heat modified zone. Within the heat modified zone may be accumulation bulbs and clusters, such as for nonwoven substrates, and/or modification of properties, such as crystallinity, strength, degradation rate, and polymer chain, such as for films. The type and properties of the substrate may be one variable that affects severity of the heat modified zone that forms along the line of weakness. The heat modified zone may be greater on the layer positioned in facing relationship with the laser source or, stated differently, the layer that the laser beam first encounters when acting on the substrate may include a greater number of accumulation bulbs and clusters. The layer having a greater heat modified zone may be positioned on the absorbent article such that when the absorbent article is worn it reduces or eliminates contact with the wearer's skin, and the layer having a smaller heat modified zone may be positioned on the absorbent article such that when the absorbent article is worn there may be some contact with the wearer's skin. Minimizing the heat modified zone on the inner layer 164 or outer layer 162 may aid in the perceived softness of the layers. The process and apparatus described herein may act on either the inner layer 164 or the outer layer 162 of a substrate, such as the belt assembly.

In some embodiments, the elastic strands 168 may be positioned in a certain location on the outer circumferential surface of the process member or the guide rollers. Thus, the outer circumferential surface may include one or more grooves into which the elastic stands 168 may be disposed, as illustrated in Figures 20A and 20B.

Figure 20A illustrates an outer circumferential surface 308, 307, 316 of one of the process member 302, the first guide roller 306, and/or the second guide roller 314. The outer circumferential surface may include one or more apertures 318 configured to transfer air toward or away from the longitudinal axis of rotation 310, 304, 315. The one or more apertures 318 may aid in transferring the belt assembly 204 onto the outer circumferential surface and keeping the belt assembly 204 in place during rotation and subsequent processing.

Further, the outer circumferential surface may include one or more grooves 320. The one or more grooves may surround the outer circumferential surface such that the groove extends about the axis of rotation 310, 304, 315. In some embodiments, all or some of the grooves may extend only partially around the axis of rotation 310, 304, 315. The grooves 320 may be placed such that there are ungrooved portions between groove portions. Further, the grooves may be spaced in the cross direction such that there is a uniform distance between each groove 320. It is also to be appreciated that the grooves 320 may be spaced in the cross direction such that there is a non-uniform distance between each groove 320, as illustrated in Figure 20A. The grooves may be spaced in the cross direction CD such that each groove corresponds to the desired spacing of the elastic strands 168. The outer circumferential surface 308 may include any number of grooves 320 that allow the belt assembly 204 to remain in a desired position during advancement of the belt assembly 204 and/or to locate one or more of the elastic strands 168 in the belt assembly 204. For example, to locate the elastic strands 168, the outer circumferential surface 308 may include a number of grooves 320 into which the elastic strands 168 are positioned as the belt assembly 204 is transferred onto the process member 302. Figure 20B illustrates a portion of the outer circumferential surface 308, 307, 316 including one or more grooves 320 into which the elastic strands 168 are positioned. It is to be appreciated that the grooves may be any shape such as semi-circular, triangular, hexagonal, trapezoidal, or any other shape that inhibits movement of the elastic strands and/or maintains the location of the elastic strands 168 about the outer circumferential surface 308.

It is to be appreciated that the outer circumferential surface 308, 307, 316 may include grooves that extend parallel to the longitudinal axis or orthogonal to the longitudinal axis. The outer circumferential surface 308, 307, 316 may be machined in a number of configurations to aid in maintaining the position of and transferring the belt assembly 204.

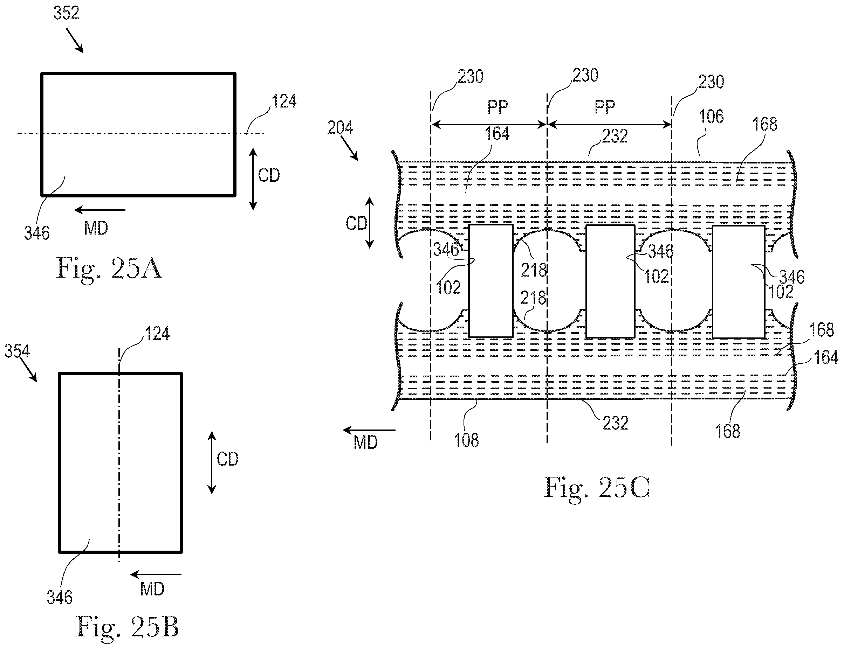

As illustrated in Figures 11 and 12-16B, the substrate 301, which may be a belt assembly 204, is advanced to a laser source, which emits a laser beam. The laser source may be used to impart a line of weakness into the belt assembly 204. A line of weakness may include linear and non-linear patterns, such as curvilinear patters, or other shapes, such as circles, rectangles, or triangles. A laser source forms a line of weakness by causing portions of material to separate while other portions of the material remain attached. A line of weakness may be a discrete line of weakness or a continuous line of weakness. A discrete line of weakness may be a line that includes a first end point and a second end point within the length of two product pitches. It is to be appreciated that the first and second end points may not be identifiable, such as in the case where the laser imparts a circular line of weakness into the substrate. A continuous line of weakness may be a line that continues over the length of two or more product pitches. A product pitch PP is the desired length of a discrete product or the length between the beginning and end of a single product extending in the machine direction MD. It is to be appreciated that the product pitch changes based on the size and type of the product that is being produced with the substrate. An example of a product pitch PP is illustrated in Figure 25C. The product pitch is measured parallel to the machine direction MD and between a first cut line and an adjacent, second cut line, as illustrated in Figure 25C. It is to be appreciated that the product pitch PP is to be measured prior to the separation of the product from the belt assembly because the belt assembly is placed under machine direction tension as it is produced.

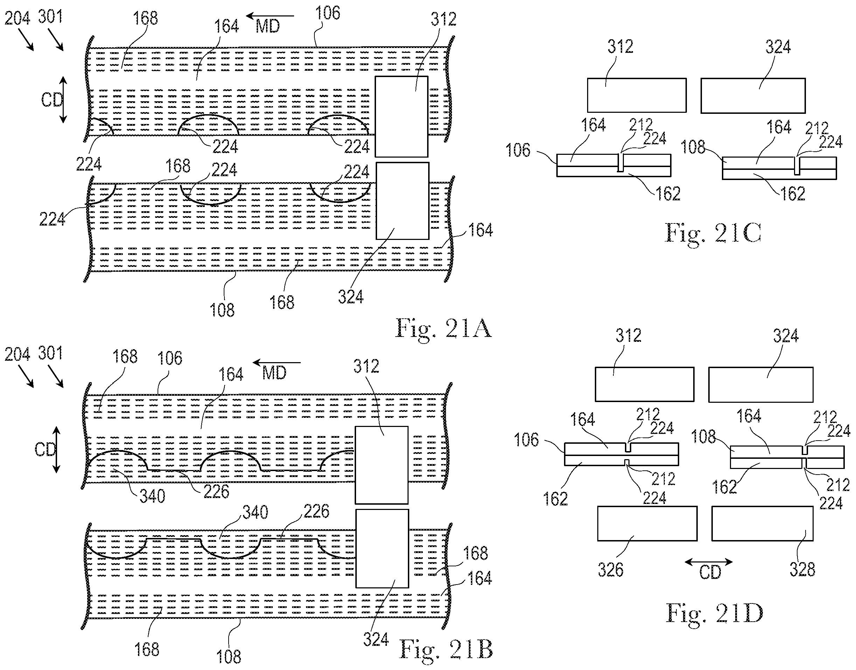

As illustrated in Figures 21A and 21C, one or more laser sources may be used to impart a line of weakness into the belt assembly 204. More specifically, for example, a first laser source 312, which emits a first laser beam, may be positioned to interact with the first belt 106 and a second laser source 324, which emits a second laser beam, may be positioned to interact with the second belt 108. The first laser source 312 may be used to impart a line of weakness 218, which may be a discrete line of weakness 224, into the first belt 106. The second laser source 324 may be used to impart a line of weakness 218, which may be a discrete line of weakness 224, into the second belt 108. The first laser source 312 emits a first laser beam to engage the wearer facing layer 164 of the first belt 106 forming a discrete line of weakness 224 in the wearer facing layer 162, and the line of weakness may extend through a portion of the garment facing layer 162. The second laser source 324 emits a second laser beam to engage the wearer facing layer 162 of the second belt 108 forming a discrete line of weakness 224 in the wearer facing layer 162, and the line of weakness may extend through a portion the garment facing layer 164. Each of the first laser source 312 and the second laser source 324 may be sequenced to create each discrete line of weakness. Each sequence includes a dwell period, during which time the laser beam is not emitted, and an active period, during which time the laser beam is emitted. As the belt assembly 204 advances in the machine direction MD, each laser source may be sequenced such that during the active period the laser source imparts the discrete line of weakness and subsequently may be sequenced such that during the dwell period the laser source does not affect the belt assembly. The dwell period may be any time. For example, the dwell period may begin at the end of a first discrete line of weakness and end when the belt assembly has advanced to a positioned where the laser source is to begin a second discrete line of weakness. It is to be appreciated that the laser source is not powered off during the dwell time but rather just fails to emit a laser beam during the dwell time. Each discrete line of weakness 224 may have characteristics such as described with respect to Figures 10A-10N.

Referring to Figures 21A and 21D, in some embodiments, additional laser sources may be used. For example, as illustrated in Figure 21D, a first laser source 312 may be positioned adjacent the first belt 106 and a second laser source 324 may be positioned adjacent the second belt 108. The first laser source 312 may be used to impart a line of weakness 218 into the first belt 106. The second laser source 324 may be used to impart a line of weakness 218 into the second belt 108. The first laser source 312 emits a first laser beam to engage the wearer facing layer 164 of the first belt 106 forming discrete line of weakness 224 in the wearer facing layer 164. The second laser source emits a second laser beam to engage the wearer facing layer 164 of the second belt 108 forming discrete line of weakness 224 in the wearer facing layer 164. It is to be appreciated that a third laser source 326 and a fourth laser source 328 may each be used to impart a line of weakness on the garment facing surface 162 of each of the first belt 106 and the second belt 108 as described above. It is also to be appreciated that the discrete line of weakness defined by the wearer facing layer 164 may coincide with the discrete line of weakness defined by the garment facing layer 162. Each of the first laser source 312, the second laser source 324, the third laser source 326, and the fourth laser source 328 may be sequenced to create each discrete line of weakness. As the belt assembly 204 advances in the machine direction, each laser source may be sequenced such that the laser source emits a laser beam that imparts the discrete line of weakness and subsequently, sequenced such that the laser source does not emit a laser beam until the belt assembly 204 advances to a position where a second discrete line of weakness needs to be imparted onto the belt assembly. Each discrete line of weakness 224 may have characteristics such as that described with respect to Figure 10A-10N.

It is also to be appreciated that a discrete line of weakness may also be imparted to the substrate by a laser source that continually emits a laser beam. For example, the laser beam may impart the line of weakness into the substrate along the discrete line and, subsequently, may be diverted to a position adjacent the edge of the substrate once the discrete line of weakness is complete. The laser source may remain in an active state, meaning a laser beam is being emitted, while the substrate advances in the machine direction. As the substrate advances in the machine direction, the laser beam may be directed such that it imparts a line of weakness and, subsequently, the laser beam may be diverted such that the laser beam is positioned adjacent the edge of the substrate or, state another way, does not act on the substrate.

Further, the power output of the laser source may be adjusted while the laser source is being sequenced. For example, the energy output of the laser source may be adjusted during a dwell period. It is to be appreciated that sequencing the laser source, as previously discussed, is not the same as the pulse duration or frequency of the laser. Additionally, the pulse duration and frequency may be adjusted. The specifications of the particular laser source selected may limit how much the pulse duration and frequency may be adjusted. The average power is calculated by multiplying the pulse duration by the frequency. For example, for a laser source having an average power of 200 Watts, the following combinations of pulse duration and frequency may be used: 200 µJ at 1 MHz; 20 µJ at 10 MHz; 50 µJ at 4 MHz. The laser source may also be adjusted such that it operates at less power, such as 150 Watts or 100 Watts. When the power of the laser source is adjusted, the pulse duration and frequency are each adjusted accordingly. It is to be appreciated that the operating parameters of the laser source may be selected and subsequently adjusted based on the type of material and the desired characteristics of the final separation edge.

For example, a first discrete line of weakness may be imparted to the belt assembly at a first power output and a second discrete line of weakness may be imparted to the belt assembly at a second power output, wherein the first power output is greater than or less than the second power output. The power output of the laser source may also be adjusted while imparting a single, discrete line of weakness. More specifically, the laser source may impart a portion of the discrete line of weakness at a first power output and impart another portion of that discrete line of weakness at a second power output, which is greater than or less than the first power output. In some embodiments, for example, a belt assembly may include a first portion including a single substrate layer and a second portion including more than one substrate layer, such as two or three substrate layers. A discrete or continuous line of weakness may be required to be imparted over both the first portion and the second portion of the belt assembly. Thus, the laser source may operate at a first power output as it imparts the line of weakness over the first portion including only a single substrate layer and the laser source may operate at a second power output, which is different than the first power output, as it imparts the line of weakness over the second portion including several substrate layers. The laser source may be adjusted from the first output power to the second output power while it is powered on and imparting the line of weakness into the belt assembly. The power output of the laser source may increase as the number of layers of substrate into which the line of weakness needs to be imparted increases. It is to be appreciated that there may be one or more laser sources depending on the type of line of weakness that needs to be imparted to the belt assembly 204 and the material properties of the belt assembly. Once a given power is selected the pulse duration and frequency may also be adjusted to impart a line of weakness that ultimately obtains a desired separation edge.

It is to be appreciated that the speed at which the laser beam moves about the substrate may be varied in addition to the power output or independent of the power output. For example, the laser beam may move such that the laser beam traverses over the substrate at a speed of at least about 8 m/s or from about 8 m/s to about 11 m/s. The speed at which the substrate is moving and the speed at which the laser source cuts the substrate also impact the final quality of the separation edge. For example, the greater time the laser beam acts on a particular portion of the substrate the more likely a greater heat modified zone will be present along the separation edge.

A laser source may emit many hundreds of watts, which can be concentrated over a relatively small spot, referred to as the spot size, which is a measure of the diameter of the spot. For example, the spot size may be from about 5 µm to about 300 µm, and/or from about 50 µm to about 200 µm, and/or from about 100 µm to about 150 µm, including all 0.1 µm therebetween. An ultra short pulse laser, for example, may have a spot size of at least about 10 µm or from about 40 µm to about 60 µm. A CO₂ laser may have a spot size of at least about 100 µm. It is to be appreciated that spot size is also dependent on the wavelength of the laser beam. The spot size of the laser beam may be varied. For example, the scan head may be configured such that the laser beams spot size on the substrate may be widened and narrowed. When the spot size of the laser beam is widened such that the laser beam covers a greater area of the substrate, the laser beam affects the substrate less. As the spot size of the laser beam is narrowed, becoming concentrated over a relatively smaller area of the substrate, the more likely that the laser beam may affect the substrate. Thus, the spot size of the laser beam may be widened over portions of the substrate that are desired to be unaffected or minimally affected by the laser beam, and the spot size of the laser beam may be narrowed over portions of the substrate that are desired to be affected by the laser beam. The spot size allows the laser beam to precisely cut the substrate. The smaller the spot size the greater the precision of the cut line and/or line of weakness. For example, the spot size of the ultra short pulse laser source is smaller than that of a CO₂ laser source. Thus, the ultra short pulse laser source is able to affect the substrate with greater precision than a CO₂ laser source.

The speed of the laser beam, the power output of the laser source, and the spot size of the laser beam are all variables that may be altered to impart a line of weakness into a surface of a substrate, which may be a belt assembly, to obtain a desired separation edge. Altering any one of these variables may change the characteristics of the separation edge. Ultra short pulse lasers provide optimal operating parameters that may be adjusted to impart a line of weakness that produces a separation edge having a relatively smaller heat modified zone and a desirable separation edge quality.

The line of weakness may be a continuous line of weakness, as illustrated in Figure 21B. A first laser source 312 may be positioned to interact with the first belt 106 and a second laser source 324 may be positioned to interact with the second belt 108. The first laser source 312 may be used to impart a continuous line of weakness 226 into the first belt 106. The second laser source 324 may be used to impart a continuous line of weakness 226 into the second belt 108. The continuous lines of weakness 226 may be separated and have characteristics such as the separation edge 212 described with respect to Figures 10A-10N. It is to be appreciated that the power output of the laser source 312, 324 may be adjusted while the laser source is imparting the continuous line of weakness onto the belt assembly, as previously described. In addition, in order to ensure separation of the trim portion of the line of weakness, the power output of the laser source may need to be increased around curved or non-linear portions of the continuous line of weakness. Further, as described above with respect to Figure 21A, the configuration in Figure 21B may also include a third laser source 326 and a fourth laser source 328 configured to interact with the opposite surface of the belt assembly as the first and second laser sources and configured to impart a second continuous line of weakness that may be coincident or offset with the first line of weakness.

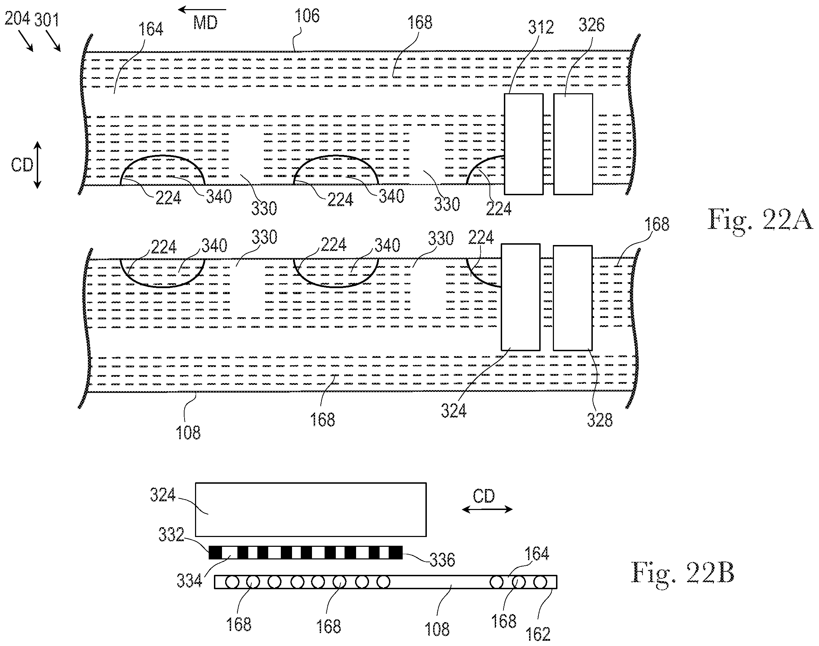

In some embodiments, a series of laser sources may be used, as illustrated in Figure 22A. For example, a first laser source 312 may be used to impart a line of weakness 118 into a first belt 106 and a second laser source 324 may be used to impart a line of weakness 218 into a second belt 108. Further, a third laser source 326 and a fourth laser source 328 may be used to sever one or more elastic strands 168 in the belt assembly 204. More specifically, the third laser source 326 may be used to sever one or more elastic strands 168 in the first belt 106 and the fourth laser source 328 may be used to sever one or more elastic strands 168 in the second belt 108. The elastic strands 168 to be severed may be located in the portion of the belt assembly that is to overlap a discrete component or sub-assembly, such as an absorbent core, as described with reference to Figures 2 and 4, which may be disposed on the belt assembly 204 in a subsequent process. The elastic strands 168 may be severed in this region to prevent the belt assembly from gathering in the region of the absorbent core and/or the chassis, which are examples of component parts that may be added to the belt assembly 204. It is to be appreciated that other portions of the elastic strands may be severed for purposes of providing a better fit about the waist and/or leg openings of the wearer.

The laser source may be configured to sever any number of elastics 168. Thus, the size of the gap 330 in the elastic stands 168 may differ across a belt assembly 204. For example, if the elastic strands 168 have been continuously bonded to the substrate, there may be no gap 330 or minimal gap 330 between severed elastic strands 168. Alternatively, if the elastic strands 168 have been intermittently bonded to the substrate, the severed elastic strands 168 may snap back to a portion of the elastic strand that has been bonded to the substrate forming a gap 330. Thus, the gap 330 may be of a uniform width or a non-uniform width.

It is to be appreciated that a configuration of laser sources and their associated scan heads may also be present adjacent the opposite side of the substrate as set forth in Figure 21D.

In some embodiments, the laser source may be operated in the cross direction as the belt assembly advances in the machine direction to sever the one or more elastic strands. More specifically, the laser source and/or the laser beam emitted by the laser source may be operated such that it imparts a cut line across the portion of the elastic strands that are desired to be severed. It is to be appreciated that the laser source 312 and/or laser beam may also move in a direction at an angle to the cross direction CD. For example, the laser source 312 or laser beam may move in a substantially diagonal direction due to the movement of the belt assembly 204 in the machine direction MD. Thus, the movement of the belt assembly in the machine direction may be accounted for in the movement of the laser such that the laser source and/or laser beam moves in a diagonal direction so that the elastic strands are severed in a direction extending parallel to the cross direction.

It is to be appreciated that the laser source that imparts the line of weakness may be the same type of laser source that cuts the elastic strands or a differently type of laser source. For example, the laser source used to impart a line of weakness may be an ultra short pulse laser source and the laser source used to sever the elastic strands may be a longer pulse laser source, such as a CO₂ laser source.

In some embodiments, a mask 332 may be used to prevent the laser beam from engaging the substrate at certain locations. For example, a mask 332 may be used to prevent those portions of the nonwoven that do not overlap an elastic strand 168 from being affected by the laser source. The mask 332 may be positioned between the laser source 324 and the belt assembly 204, as illustrated in Figure 22B. The mask 332 may include transfer portions 334, which allows the laser source to interact with the substrate and/or the elastic strand(s), and preventative portions 336, which stops the laser source from acting on the substrate and the elastic strand(s). In some embodiments, the mask 332 may be positioned such that the transfer portions 334 coincide with the elastic strands and the preventative portions 336 coincide with the portions of the elastic belt that do not have elastic strands. Stated another way, a laser source may continuously operate as it moves in the cross direction to sever the elastic strands 168. The mask allows the laser source to affect only certain portions of the substrate(s), for example those portions overlapping the elastic strands 168. The mask 332 may be configured with any number of transfer portions and preventative portions. The number and design of these portions will depend, in part, on which portions of the substrate(s) it is desirable for the laser source to affect. The mask may be moveable in the machine direction MD and the cross direction CD. The mask may be continually adjusted in one or more directions so that it may maintain alignment with the portion of the substrate that is desired to be acted on by the laser source.

It is to be appreciated that to use the laser source to sever one or more elastics, the location of the elastics should be known or detected. As previously described, the outer circumferential surface of the process member and guide rollers may include one or more grooves. Thus, each elastic strand may be disposed within a groove, or those elastic strands that are to be severed may be disposed within one or more grooves. The location of the grooves may be predetermined and, therefore, the location of the elastic strands may be known. Alternatively or in addition to the aforementioned, a high speed camera may be used to detect the position of the elastic strands. The position of the elastic strands may then be communicated to the laser source and the laser source may be operated accordingly.

In some embodiments, the laser source may be sequenced so that certain portions of the substrate, which may be a belt assembly, remain unaffected by the laser source. For example, the laser source may be controlled such that the laser source emits a laser beam for a certain period of time, active period, and the laser source fails to emit a laser beam for a certain period of time, dwell period. The duration of the dwell period and active period may depend, in part, on the speed of the belt assembly advancing in the machine direction and the desired characteristics of the separation edge. The duration of the dwell period and the active period may be changed each time the laser source completes a sequence. Thus, the time of a first active period, when the laser source emits a laser beam, may be longer than a second, subsequent active period. This may apply to the dwell period as well.

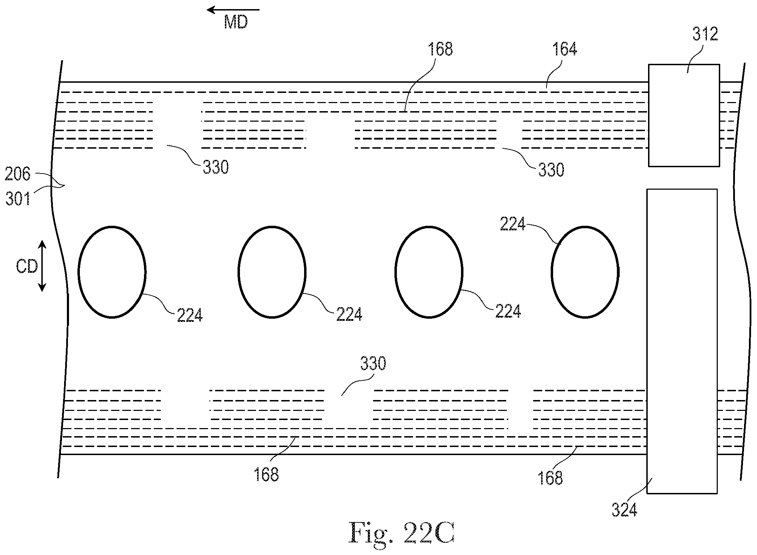

Figure 22C illustrates a first laser source 312 and a second laser source 324 adjacent the wearer facing surface 164 of a belt assembly 204 including a body substrate 206. In some embodiments, the first laser source 312 may be used to sever one or more elastic strands 168 to form a gap 330 in the elastic strands. To sever the one or more elastic strands 168, the laser source may be adjusted, so that the laser source emits a laser beam to impart energy to the substrate while it is disposed over an elastic strand and, subsequently, imparts no or minimal energy while it is not disposed over an elastic strand. Alternatively, or in addition to sequencing the laser source and/or adjusting the pulse duration or frequency the laser source, a mask may be used to control which portions of the substrate the laser source may affect. A second laser source 324 may be used to impart a line of weakness. As illustrated in Figure 22C, the second laser source 324 may impart a discrete line of weakness 224 into the body substrate 206. The second laser source 324 may traverse in the cross direction CD to a second position or the laser beam may be directed to a second position. In the second position, the laser beam may also be used to sever one or more elastic strands to form a gap 330 in the elastic strands. The one or more elastic strands 168 may be severed in any manner as previously discussed. It is to be appreciated that an additional configuration of laser source(s) and/or scan head(s) may be positioned adjacent the opposite, garment facing surface of the belt assembly to engage the garment facing surface as previously described with respect to the wearer facing surface.

It is to be appreciated that when severing the elastic strands, it is desirable to minimize the destruction of the substrate by controlling the exposure of the substrate layers to the laser source and to ensure that the elastic strands are separated by the laser. Stated another way, the intent is to sever the elastic strand prior to separating all nonwoven fibers. Generally, the nonwoven substrate that is disposed between the laser source and the elastic strand will degrade, such as by melting and/or ablating, prior to the elastic strand due to the properties of the nonwoven substrate and the elastic strand. More specifically, the nonwoven belts and the elastic strands each have a wavelength or range of wavelengths at which its absorptivity is greatest or optimal. Thus, a laser source may be chosen such that the wavelength emitted by the laser beam is more readily absorbed by the elastic strands than the nonwoven substrate. In this case, the elastic strands may break prior to all the fibers of the nonwoven substrate separating. It is to be appreciated that the elastic strands may be under tension when they are acted on by the laser source. Elastics under tension want to relax. This property of the elastic strands may also aid in cutting the elastic strand prior to breaking or separating all the fibers of the nonwoven substrate.

Materials may be altered to increase their absorptivity even if the laser source is operating outside the optimal range of wavelengths that coincide with the optimum absorptivity. In some embodiments, the elastic strands may be chemically altered such that the elastic strands have an increased rate of energy absorption, or absorptivity at a certain wavelength. These chemical additives may be added to the material that forms each elastic strand prior to the elastic strand being formed, such as by extrusion or other known methods. These chemicals additives may also be added to the elastic strand after formation. For example, these chemicals may be applied topically to each elastic strand. These chemical additives may also be added to the adhesive that attaches the elastic strand to the nonwoven substrate. Such chemical additives are available from Clearweld, Binghamton, NY. These chemical additives may be added to ensure that the elastic strands 168 are severed or can be severed by a relatively low force upon separation of the trim from the belt assembly 204, and to ensure that the elastic strands 168 present in the region of the belt assembly 204 are severed while not destroying the substrate layers.

It is also to be appreciated that any number of laser sources and/or laser beams may be used to either weaken or sever the elastic strands and/or to impart a continuous or discontinuous line of weakness into the belt assembly. For example, a single laser source may be used to impart a continuous or discontinuous line of weakness into the first and second belts 106, 108 and another laser source may be used to sever the elastic strands in both the first and second belts 106, 108.

In some embodiments, for example, an ultra short pulse laser may be used to impart the line of weakness into the nonwoven portions of the belt assembly and a longer-pulse laser, such as a CO₂ laser, may be used to cut the elastic strands. The ultra short pulse laser operates at a wavelength that coincides with the nonwoven substrate layers of the belt assembly. However, the ultra short pulse laser may not be ideal for cutting elastic strands. It has been found that using a CO₂ laser with a wavelength from about 9.4 µm to about 10.6 µm results in optimum absorptivity of the elastic strand material. Thus, an ultra short pulse laser may be used to cut the nonwoven substrate layers of the belt assembly and a CO₂ laser may be used to cut the elastic strands. To act only on areas that include the elastic strands, the CO₂ laser may be sequenced, such that the laser source oscillates between dwell periods and active periods, or the pulse duration may be adjusted such that the laser beam imparts energy to the substrate during the pulse duration and fails to impart energy for the remainder of the period, which is constrained by the frequency. Referring to Figure 22A, the first laser source 312 may be a CO₂ laser and the third laser source 326 may be an ultra short pulse laser. It is also to be appreciated that the ultra short pulse laser and the CO₂ laser may be placed in other orientations. For example, ultra short pulse laser may be positioned such that the laser beam engages the garment facing surface 164 and the CO₂ laser may be positioned such that the laser beam engages that wearer-facing surface 162. Similarly, the CO₂ laser may be positioned such that laser beam engages the garment facing surface 164 and the ultra short pulse laser may be positioned such that the laser beam engages the wearer facing surface 162.

It is also to be appreciated that the elastic strands may be treated with an additive that allows the ultra short pulse laser to sever or weaken the elastic strands. Thus, for example, referring to Figure 22C, the first laser source 312 and the second laser source 324 may both be ultra short pulse lasers. If the laser source is able to weaken the elastic strands such that they may be readily separated by a trim removal process, this may aid in processing by holding the elastics together while the belt assembly undergoes additional processing.

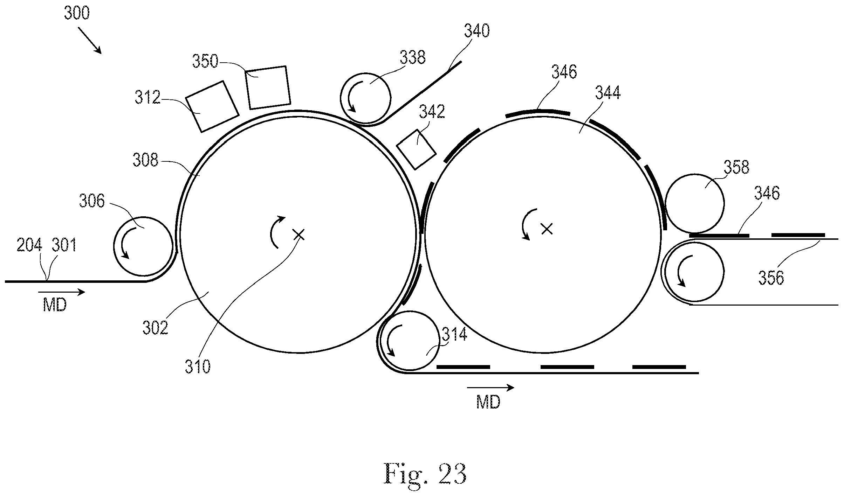

As previously discussed, the belt assembly 204 may undergo one or more processes. Figure 23 illustrates an example embodiment of an apparatus 300 that may be used to manufacture an absorbent article 100. The process member 302 may rotate about a longitudinal axis of rotation 310 and be configured to receive a belt assembly 204, as previously discussed.

The belt assembly 204 may advance in a machine direction MD toward the process member 302. A first guide roller 306 may aid in the transfer of the belt assembly 204 onto an outer circumferential surface 308 of the process member 302. The outer circumferential surface 308 of the process member 302 may include one or more apertures. A vacuum source, not shown, may be in fluid communication with the one or more apertures. The vacuum source allows a fluid to be circulated through the one or more apertures toward the longitudinal axis of rotation 310. The movement of fluid may result in the belt assembly 204 being forced toward the outer circumferential surface 308 of the process member 302. The process member 302 may rotate about the longitudinal axis of rotation 310 causing the belt assembly 204 to advance toward a laser source 312, which may include one or more laser sources, as previously discussed. The laser source 312 may be used to impart a line of weakness into the belt assembly 204.

In some embodiments, the process member 304 may then advance the belt assembly 204 to a cutting member 350. The cutting member 350 may be used to sever one or more elastic strands 168. The cutting member 350 may be an apparatus such as disclosed in U.S. Pat. No. 8,440,043. It is also to be appreciated that a second laser source may be used in place of the cutting member to sever or weaken the elastic strands as previously discussed. It is also to be appreciated that the laser source 312 may be used to both impart a line of weakness and sever the one or more elastic strands.

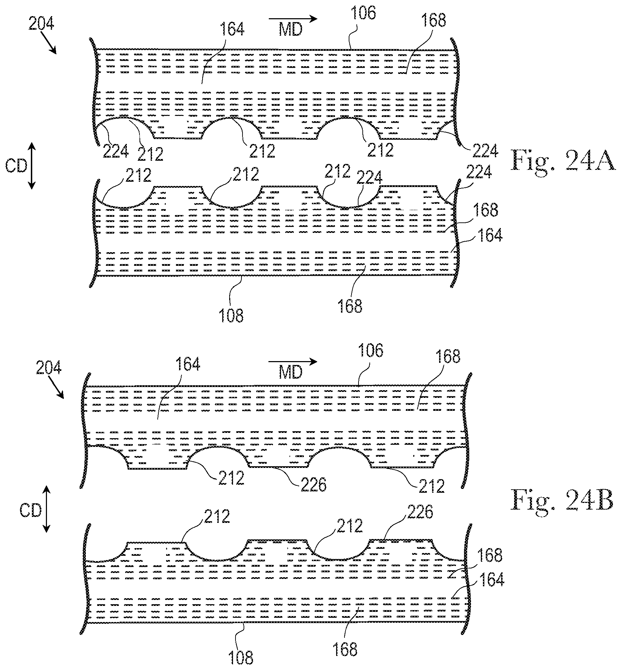

The belt assembly 204 including one or more lines of weakness may advance to a trim removal member 338. The trim removal member 338 may remove the trim 340, which may include a discrete portion and/or a continuous portion of the belt assembly, as illustrated in Figures 21A and 21B. A trim removal member 338 may apply a force to the discrete or continuous line of weakness to remove the continuous lengths of trim as well as the discrete pieces of trim that have been produced by the laser source 312. More particularly, as the first belt 106 and the second belt 108 advance in the machine direction, the trim removal member 338 may be used to separate and remove trim from and/or along either or both opposing side edges of the first belt 106 and the second belt 108. It is also to be appreciated that the trim removal member may impart enough force on the belt assembly that the weakened elastic strands are severed. The trim removal member 338 may include an apparatus such as disclosed in U.S. Publ. No. 2012/0079926. Other devices that may be suitable as a trim removal member 338 include a vacuum head including one or more vacuum nozzles, which may be used to separate the trim from the belt assembly, and a duct system, which may be used to transport the trim from the process member to a disposal location. Figures 24A-24B illustrate the belt assembly 204 upon removal of the trim 340. Upon removal of the trim 340 a separation edge 212, is formed.

The belt assembly 204 may then be advanced to an adhesive applicator 342. The adhesive applicator may apply adhesive, such as glue, to the belt assembly 204. The adhesive may be applied to a portion of the first belt 106 and a portion of the second belt 108. The adhesive may be applied to portions of the first belt 106 and the second belt 108 where additional components for the absorbent article are to be added. In some embodiments, for example, the adhesive may be applied to the portion of the belt assembly 204 having severed elastic strands 168.

Upon applying adhesive to the belt assembly 204, the belt assembly 204 may be advanced to operatively engage with a transfer apparatus 344. The transfer apparatus 344 may be used to transfer and/or rotate a discrete component 346 of the absorbent article. An example of a discrete component 346 is a chassis 102, such as discussed with reference to Figures 2 and 4. In some embodiments, the transfer apparatus 344 may receive a discrete component 346 positioned in a first orientation 352, as illustrated in Figure 25A. More specifically, the discrete component 346 may be orientated in a first orientation 352 when the longitudinal axis 124 of the discrete component 346 is substantially parallel to the machine direction MD and/or substantially perpendicular to the cross direction CD. However, to be disposed on the belt assembly 204, the discrete component 346 may need to be rotated. In the embodiments where the discrete component 346 is a chassis 102, the chassis 102 may need to be rotated so that a first portion of the chassis 102 is disposed on the first belt 106 and a second portion of the chassis 102 is disposed on the second belt 108. Thus, the transfer apparatus 344 may be configured to transfer the discrete component 346 from a first carrier member 356, which may include a conveyor belt supported by one or more guide rollers. More specifically, a third guide roller 358 may be used to aid in transferring the discrete component 346 onto the outer circumferential surface 360 of the transfer member 344. The transfer member 344 may advance the discrete component 346 to a position that allows the discrete component 346 to be disposed on the belt assembly 204. In some embodiments, the transfer member 344 may also rotate the discrete component to a second orientation 354, as illustrated in Figure 25B. More specifically, the discrete component 346 may be orientated in a second orientation 352 when the longitudinal axis 124 of the discrete component 346 is substantially perpendicular to the machine direction MD and/or substantially parallel to the cross direction CD. It is to be appreciated that the discrete component 346 may not be rotated or may be rotated in any position that allows the discrete component 346 to be orientated in a desired position. The transfer member 344 may be an apparatus such as that disclosed in U.S. Pat. No. 8,820,513.

As illustrated in Figure 23, the transfer member 344 may be operatively engaged with the process member 302. More specifically, as the belt assembly 204 rotates about the longitudinal axis of rotation 310, the transfer member 344 may transfer a discrete component 346 onto at least a portion of the belt assembly 204. In some embodiments, as illustrated in Figure 25C, the transfer member 344 may transfer a chassis 102 onto a portion of the first belt 106 and a portion of the second belt 108. Stated another way, a first portion of the chassis 102 may be disposed on a portion of the first belt 106 and a second portion of the chassis 102 may be disposed on the portion of the second belt 108. As was previously discussed, an adhesive may be applied to the belt assembly 204. The adhesive may allow the chassis 102 to be adhered to the belt assembly 304.

Still referring to Figure 23, the belt assembly 204 including the discrete component 346, such as a chassis 346, may be advanced by the process member 302 to a second guide roller 314. The second guide roll 314 may be used to transfer the belt assembly 204 including the discrete component 346 to additional downstream processes. In some embodiments, the second guide roller 314 may also act as a bonding roll. The second guide roller 314 may be positioned such that pressure is applied to the belt assembly 204 and the discrete component 346 as the combination passes between the second guide roller 314 and the process member 302. The second guide roller 314 may be used to bond the discrete component 346 to the belt assembly 204.

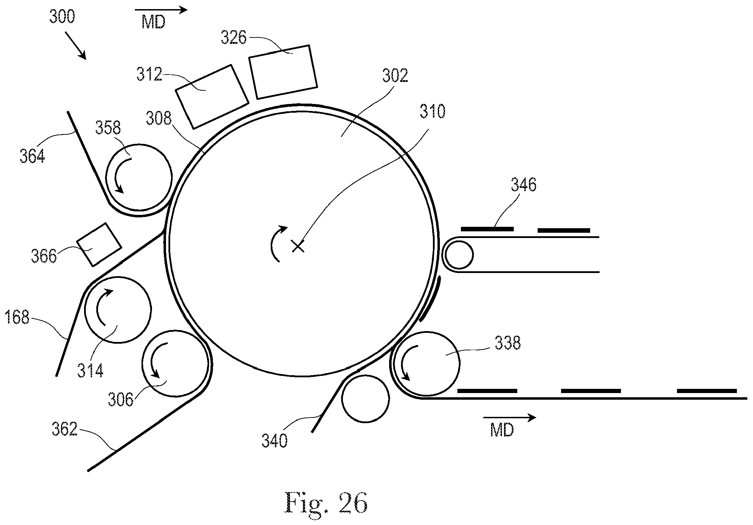

As previously discussed, the belt assembly 204 may include a garment facing layer, also referred to as an outer layer, of nonwoven 162 and a wearer facing layer, also referred to as an inner layer, of nonwoven 164 and one or more elastic strands 168 disposed between the outer layer 162 and the inner layer 164. In some embodiments, the belt assembly 204 may be assembled on the outer circumferential surface 308 of the process member 302, as illustrated in Figure 26. A first continuous substrate layer may correspond with the outer layer 162. A second continuous substrate layer 164 may correspond to an inner layer 164.

As illustrated in Figure 26, a first continuous substrate layer 362 may be advanced toward the process member 304. The first continuous substrate layer 362 may surround a portion of a first guide roller 306. The first guide roller may aid in advancing and transferring the first continuous substrate layer 362. The first continuous substrate layer 362 may be disposed on the outer circumferential surface 308 of the process member 302. As previously discussed, one or more apertures may be in fluid communication with a vacuum source, which may cause the first continuous substrate layer 362 to be forced against the outer circumferential surface 308.

Still referring to Figure 26, one or more elastic strands 168 may be advanced toward the process member 302. The one or more elastic strands 168 may be stretched in the machine direction MD prior to being disposed on the first continuous substrate layer 362. Further, the one or more elastic strands may be adhered to the first continuous substrate layer 362. Thus, a second guide roller 314 may be used to advance the one or more elastic strands 168 to an adhesive applicator 366. The adhesive applicator 366 may apply adhesive to the one or more strands 168. The adhesive may be applied continuously over the one or more elastic strands or the adhesive may be applied in discrete sections, or intermittently, over the elastic strands. It is also to be appreciated that the discrete sections of adhesive may extend over the same length or discrete sections may have different lengths. For example, a first discrete section of adhesive may be longer than or shorter than a second discrete section of adhesive. It is also to be appreciated that there may be sections without adhesive, these sections are non-bonded sections. There may be a non-bonded section where the elastic strands are to be severed. The elastic strands including portions having adhesive applied thereto may be disposed on and bonded to the first continuous substrate 362.

It is also to be appreciated that the elastic strands may be disposed on the first continuous substrate layer 362 prior to adhesive being disposed on the elastic strands. Stated another way, the elastic strands 168 may be disposed on the first continuous substrate layer 362. The first continuous substrate 362 including the elastic strands 168 may be advance to an adhesive applicator 366. The adhesive applicator 366 may apply the adhesive to the one or more strands 168, which are disposed on the first continuous substrate 362. The elastic strands 168 are bonded to the first continuous substrate layer 362.

Still referring to Figure 26, a second continuous substrate 364 may be advanced toward the process member 302. A third guide roller 358 may aid in advancing and transferring the second continuous substrate 364 onto the process member 302. The second continuous substrate 364 may be disposed on the elastic strands 168 and the first continuous substrate layer 362.

It is to be appreciated that the aforementioned may apply to the formation of both the first belt 106, the second belt 108, and the body substrate 206. With respect to the belt assembly 204, the first belt 106 and the second belt 108 may be assembled adjacent one another in the cross direction CD on the outer circumferential surface 308 of the process member 302.

It is also to be appreciated that assembling the first belt 106, the second belt 108, and/or the body substrate 206 on the outer circumferential surface 308 of the process member 302 may aid in locating the one or more elastic strands 168 for severing. Assembling the belt or substrate on the process member 302 may lead to better control of how and where each component is disposed on the outer circumferential surface. Further, a portion of the adhesive may transfer through the first continuous layer causing some adhesion to the outer circumferential surface 308, which may cause the elastic strands to remain in relatively the same location for subsequent processing. Once the belt assembly 204 has been assembled, the belt assembly 204 may proceed to additional processes, as previously discussed.

Figure 26 also illustrates embodiments wherein a discrete component 346 may be disposed on the belt assembly 204 prior to the trim 340 being removed from the belt assembly 204. More specifically, a laser source 312, 326 may impart a line of weakness into the belt assembly 204. Further, one or more discrete components 346 may be disposed on the belt assembly 204. Subsequently, the belt assembly 204 may be advanced such that a trim removal member 338 engages the belt assembly 204 causing the discrete and/or continuous trim 340 to remain engaged with the outer circumferential surface 308 of the process member 302 and for the remainder of the belt assembly 204 including the discrete component 346 to diverge from the outer circumferential surface 308 of the process member 304 and advance in a machine direction MD away from the process member 304. As previously discussed, the trim removal member 338 may be an apparatus such as disclosed in U.S. Publ. No. 2012/0079926.

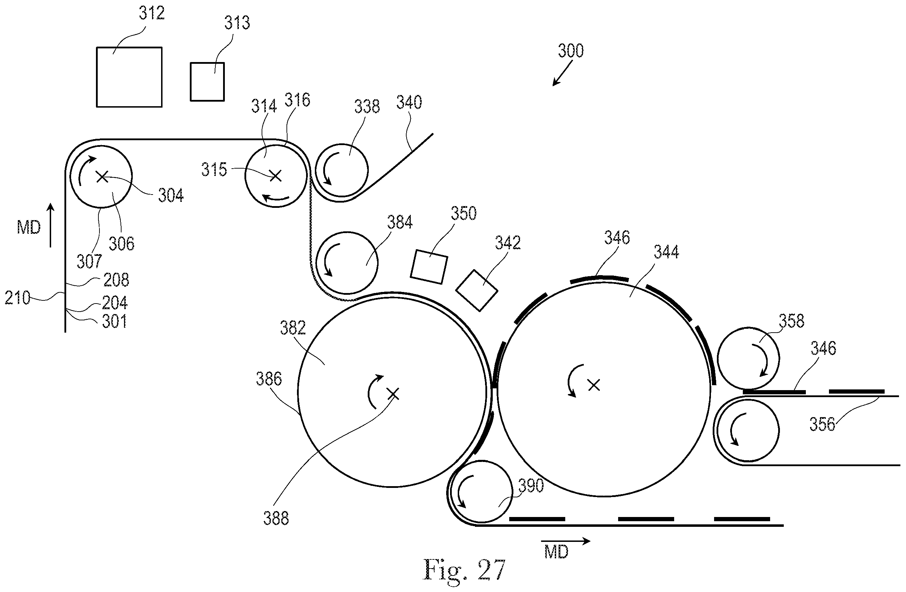

In some embodiments, an absorbent article may be manufactured by an apparatus 300 as illustrated in Figure 27. The belt assembly 204 may advance such that belt assembly 204 is disposed on a portion of a first roller guide 302 and a second roller guide 314 and the belt assembly is acted on by one or more laser beams to impart lines of weakness, as previously discussed.

The belt assembly 204 having a line of weakness may advance to a trim removal member 338. The trim removal member 338 may remove the trim 340, which may include a discrete portion and/or a continuous portion along the line of weakness, as illustrated in Figures 21A and 21B. A trim removal member 338 may apply a force to the discrete or continuous line of weakness to remove continuous lengths of trim as well as discrete pieces of trim that have been cut by the laser beam. More particularly, as the first belt 106 and the second belt 108 advances in the machine direction MD, the trim removal member 338 may be used to separate and remove trim from and/or along either or both opposing side edges of the first belt 106 and the second belt 108. The trim removal member 338 may include an apparatus such as disclosed in U.S. Publ. No. 2012/0079926. Other devices that may be suitable as a trim removal member 338 include a vacuum head including one or more vacuum nozzles, which may be used to separate the trim 340 from the belt assembly, and a duct system, which may be used to transport the trim from the process member to a disposal location. Figures 24A-24B illustrate the belt assembly 204 upon removal of the trim 340. Upon removal of the trim 340 a separation edge 212, is formed. It is to be appreciated that another device may be used in the removal of trim 340 such as a cutting device including a blade, a pressure roller, a hot air supply device, and/or another laser source.

The belt assembly 204 may advance in a machine direction MD toward a process drum 382. A fourth guide roller 384 may aid in the transfer of the belt assembly 204 onto an outer circumferential surface 386 of the process drum 382. The outer circumferential surface 386 of the process drum 382 may include one or more apertures. A vacuum source, not shown, may be in fluid communication with the one or more apertures. The vacuum source allows a fluid to be circulated through the one or more apertures toward the longitudinal axis of rotation 388. The movement of fluid may result in the belt assembly 204 being forced toward the outer circumferential surface 386 of the process device 382. The process device 382 may rotate about the longitudinal axis of rotation 388 causing the belt assembly 204 to advance toward one or more processes. For example, the process device 382 may then advance the belt assembly 204 to a cutting member 350. The cutting member 350 may be used to sever one or more elastic strands 168 or any other portion of the belt assembly 204. The cutting member 350 may be an apparatus such as disclosed in U.S. Pat. No. 8,440,043. It is to be appreciated that the cutting member may be substituted with a second laser source, which emits a second laser beam, configured to sever the elastic strands or another portion of the substrate.

The belt assembly 204 may then be advanced to an adhesive applicator 342. The adhesive applicator may apply adhesive, such as glue, to the belt assembly 204. The adhesive may be applied to a portion of the first belt 106 and a portion of the second belt 108. The adhesive may be applied to portions of the first belt 106 and the second belt 108 where additional components of the absorbent article are to be added. For example, the adhesive may be applied to the portion of the belt assembly 204 having severed elastic strands 168.

Upon applying adhesive to the belt assembly 204, the belt assembly 204 may be advanced to operatively engage with a transfer member 344. The transfer member 344 may be used to transfer and/or rotate a discrete component 346 of the absorbent article as previously described with reference to Figure 23. The transfer member 344 may be an apparatus such as that disclosed in U.S. Pat. No. 8,820,513.

As illustrated in Figure 27, the transfer member 344 may be operatively engaged with the process member 382. More specifically, as the belt assembly 204 rotates about the longitudinal axis of rotation 388, the transfer member 344 may transfer a discrete component 346 onto at least a portion of the belt assembly 204. In some embodiments, as illustrated in Figure 25C, the transfer member 344 may transfer a chassis 102 onto a portion of the first belt 106 and a portion of the second belt 108. Stated another way, a first portion of the chassis 102 may be disposed on a portion of the first belt 106 and a second portion of the chassis 102 may be disposed on the portion of the second belt 108. As was previously discussed, an adhesive may be applied to the belt assembly 204. The adhesive may allow the chassis 102 to be adhered to the belt assembly 304.

Still referring to Figure 27, the belt assembly 204 including the discrete component 346, such as a chassis 346, may be advanced by the process member 302 to a fifth guide roller 390. The fifth guide roll 390 may be used to transfer the belt assembly 204 including the discrete component 346 to additional downstream processes. In some embodiments, the fifth guide roller 390 may also act as a bonding roll.

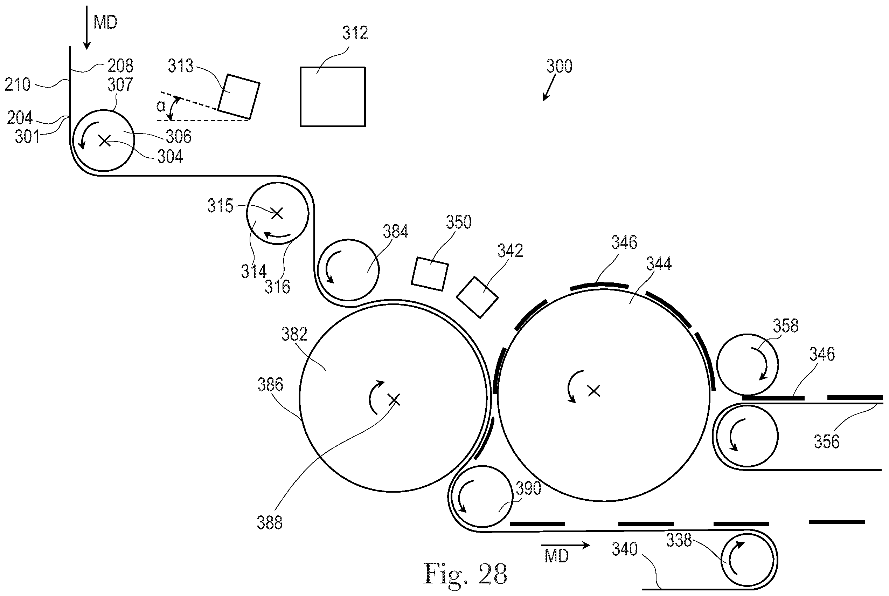

Figure 28 illustrates embodiments wherein a discrete component 346 may be disposed on the belt assembly 204 prior to the trim 340 being removed from the belt assembly 204. More specifically, the belt assembly 204 may be acted upon by a laser beam to impart a line of weakness to the belt assembly 204. The belt assembly 204 including the portion that is desired to be removed may traverse about the process drum 382 and undergo one or more processes. Further, as the belt assembly 204 traverses about the longitudinal axis of rotation 388, one or more discrete components 346 may be disposed on the belt assembly 204. Subsequently, the belt assembly 204 may be advanced such that a trim removal member 338 engages the belt assembly 204 causing the discrete and/or continuous trim 340 to engage with the outer circumferential surface of the trim member 338 and for the remainder of the belt assembly 204 including the discrete component 346 to advance in a machine direction MD away from the trim member 338. As previously discussed, the trim removal member 338 may be an apparatus such as disclosed in U.S. Publ. No. 2012/0079926.

Although much of the present disclosure is provided in the context of manufacturing absorbent articles, it is to be appreciated that the apparatuses and methods disclosed herein may be applied to the manufacture of other types of articles and products manufactured from continuous substrates. In addition to that which was previously discussed, examples of other products include absorbent articles for inanimate surfaces such as consumer products whose primary function is to absorb and retain soils and wastes that may be solid or liquid and which are removed from inanimate surfaces such as floors, objects, furniture and the like. Non-limiting examples of absorbent articles for inanimate surfaces include dusting sheets, pre-moistened wipes or pads, pre-moistened cloths, paper towels, dryer sheets and dry-cleaning clothes such. Additional examples of products include absorbent articles for animate surfaces whose primary function is to absorb and contain body exudates and, more specifically, devices which are placed against or in proximity to the body of the user to absorb and contain the various exudates discharged from the body. Non-limiting examples of incontinent absorbent articles include diapers, training and pull-on pants, adult incontinence briefs and undergarments, feminine hygiene garments such as panty liners, absorbent inserts, and the like, toilet paper, tissue paper, facial wipes or clothes, and toilet training wipes. Still other examples of products may include packaging components and substrates and/or containers for laundry detergent, which may be produced in pellets or pouches and may be manufactured in a converting or web process or even discreet products produced at high speed such as high-speed bottling lines, cosmetics, razor blade cartridges, and disposable consumer batteries. Still other examples of products include medical bandages, medical wraps, medical gowns, medical pads made of nonwoven materials, and wipes.

### Test Methods:

### Free Fiber End Measurement Method

Free Fiber End measurements are performed on images generated using a flatbed scanner capable of scanning in reflectance mode at a resolution of 6400 dpi and 8 bit grayscale (a suitable scanner is the Epson Perfection V750 Pro, Epson, USA). The scanner is interfaced with a computer running image analysis software (suitable image analysis software is ImageJ v. 1.46, National Institute of Health, USA). The sample is scanned with a black glass tile (P/N 11-0050-30, available from HunterLab, Reston, VA) as the background. The free fiber ends along the undisturbed laser separation edge in the scanned sample image are measured using the image analysis software. All testing is performed in a conditioned room maintained at about 23 ± 2 °C and about 50 ± 2 % relative humidity.

### Sample Preparation

Obtain a 3.0 x 3.0 cm square sample from a laser cut substrate, with one of the 3.0 cm sides being an undisturbed laser separation edge of the sheet or laminate. If present, carefully remove any elastic strands from within the sample. A cryogenic spray (such as Cyto-Freeze, Control Company, Houston TX) can be used, if necessary. Five substantially similar replicate samples are prepared for analysis. Precondition the samples at 23°C ± 2°C and 50 % ± 2 % relative humidity for 2 hours prior to testing.

### Image Acquisition

Lay the sample flat onto the center of the scanner bed, and place the black glass tile on top of the sample covering it completely. Orient the sample so that the undisturbed laser separation edge is aligned parallel with and perpendicular to the sides of the scanner's glass surface. Close the lid and acquire a 20.0 mm by 20.0 mm scanned image in reflectance mode at a resolution of 6400 dpi (∼4 µm/pixel) and 8 bit grayscale. The resultant image will have the undisturbed laser separation edge centered across the entire field of view. Save the image as an uncompressed TIFF format file. In like fashion, scan the remaining four replicate samples.

### Image Analysis

Open the sample image in the image analysis program. Threshold the image at an appropriate graylevel (GL) value to generate a binary image. The appropriate threshold value will segregate the sample region, with its free fibers along the undisturbed laser separation edge, from the black background, while maintaining the original dimensions of the free fibers. Initially, the binary image will display the regions containing the sample, those with graylevels above the threshold value as white (GL value of 0), and the regions containing the black background, those with graylevels below the threshold value as black (GL value of 255). Use the fill holes operation to fill in any voids within the black background region. Invert the image so that the sample region above the threshold value will now appear as black (GL value of 255), and those of the background as white (GL value of 0). Create a duplicate copy of this image. Next, two morphological operations are performed on the duplicate binary image to virtually remove the free fibers from the undisturbed laser separation edge in the binary image. First, perform an erosion operation, which will remove a single boundary pixel during each iteration. Perform a sufficient number of erosion operation iterations to remove all of the free fibers along the undisturbed laser separation edge. Second, perform an equivalent number of dilation operations, which will add back a single boundary pixel during each iteration.

Using the image analysis software, measure the perimeter around the sample region in both the image containing the free fibers, and the duplicate image where the free fibers have been removed. Calculate the ratio of the sample perimeter with the free fibers to the perimeter of the sample without the free fibers, and record this Free Fiber End value to the nearest 0.01 units. In like fashion, analyze the remaining four sample images. Calculate and report the average Free Fiber End values to the nearest 0.01 units for the five replicates.

### Roughness Measurement Method

The roughness of a single sheet or laminate substrate separation edge is measured by dragging the substrate across an x-ray photographic film then measuring the surface roughness using a 3D Laser Scanning Confocal Microscope.

Obtain a 2.0 x 4.0 cm rectangular sample from a laser cut substrate, with one of the 2.0 cm edges being the undisturbed laser separation edge, also referred to herein as a separation edge, of the substrate. If present, carefully remove any elastic strands from within the sample. A cryogenic spray (such as Cyto-Freeze, Control Company, Houston TX) can be used, if necessary. Five replicate samples are prepared for analysis. Precondition the samples at 23°C ± 2°C and 50 % ± 2 % relative humidity for 2 hours prior to testing.

Cut a 4.0 x 6.0 cm rectangular piece of x-ray photographic film (Kodak scientific imaging film X-OMAT, obtainable from Care Stream Health. Inc. Rochester, N.Y.). Submerge the piece of x-ray film in distilled water for 5 seconds. Carefully remove the x-ray film from the water, gently shake off any excess water, and lay the film piece on a flat rigid surface. Once wetted, avoid any contact with the testing surface of the x-ray film, to maintain a smooth testing surface. Without undue delay, lay the sample flat on the wetted x-ray film testing surface with the undisturbed laser separation edge 1.0 cm from, and parallel to, one of the short edges of the x-ray film. Place a 2.0 x 2.0 x 0.5 cm rigid rectangular solid Plexiglas (PMMA) support piece on top of the sample, so that it covers a 2.0 x 1.0 cm testing area of the sample, which includes and places the undisturbed laser separation edge in the middle of the Plexiglas support piece. Place an approximate 200 g weight on the rigid Plexiglas support piece. The sample is then pulled horizontally by the remaining 3.0 cm of uncovered sample along the wetted x-ray film testing surface, parallel to the long edge of the film, at constant rate of 2.0 cm/sec for 2 cm. As the trailing edge is pulled across testing surface it will leave a scratch trail on the surface of the film. The sample is then removed and the x-ray film is allowed to fully dry undisturbed prior to analysis at 23°C ± 2°C and 50 % ± 2 % relative humidity. Repeat this procedure for all five of the replicate samples.

### Characterization of the Surface

The surface roughness is determined via an instrument capable of measuring surface profile between 0 and 45 µm with a precision of 0.01 µm, e.g. via the use of an appropriate calibrated standard. A suitable instrument is 3D Laser Scanning Confocal Microscope (suitable 3D Laser Scanning Confocal Microscope is the Keyence VK-X210, commercially available from Keyence Corporation of America, Itasca, IL, USA)

Using a 20X objective lens, a 1.0X zoom level and a 0.50 µm pitch (Z-step size), the microscope is programmed to collect a surface height image with a field of view of at least 500 µm x 700 µm with an x-y pixel resolution of approximately 0.7 microns (µm)/pixel. The scan area is 2.0 x 0.5 mm. For this larger field of view, multiple scans, maintaining the x-y resolution, over the surface are collected with approximately 10% overlap between adjacent images and stitched together prior to analysis. The scan is conducted in the middle of the 2.0 x 2.0 cm scratch area, with the long axis of the scan area perpendicular to the scratch pattern and parallel to the initial laser separation edge of the sample. The height resolution is set at 0.1 nm/digit, over a sufficient height range to capture all peaks and valleys within the field of view. Calibrate the instrument according to the manufacturer's specifications.

Place the sample on the stage beneath the objective lens. Collect a surface height image (z-direction) of the specimen by following the instrument manufacturer's recommended measurement procedures, which may include using the following settings to minimize noise and maximize the quality of the surface data: Real Peak Detection, single/double scan, surface profile mode, standard area, high-accuracy quality; laser intensity (Brightness and ND filter) set using auto gain. Save the surface height image.

Open the surface height image in the surface texture analysis software. Surface texture parameters are calculated for each image separately. Images are prepared for analysis by applying the following filtering procedure to each image according to ISO 25178-2:2012: 1) a Gaussian low pass S-filter with a nesting index (cut-off) of 2.0 µm; and 2) an F-operation of plane tilt (auto) correction. The Gaussian filter is run utilizing end effect correction. This filtering procedure produces the SF surface from which the areal surface texture parameters will be calculated. The surface texture parameters Sq, is described in ISO 25178-2:2012. Sq is the root mean square of the profile heights of the roughness surface. The units of Sq are µm.

Scan and analyze the surface textures of five replicates. Average together the five Sq values and report to the nearest 0.01 µm.

### Nonwoven Substrate Edge Quality

A heat modified zone along the separation edge, also referred to herein as a separation edge, is assessed using Scanning Electron Microscopy (SEM) with a bench top unit (a suitable SEM is the Hitachi TM-1000). A 1.00 cm x 1.00 cm specimen is excised from the substrate along a laser separation edge. The specimen is harvested such that the laser separation edge is the primary edge with the two sides perpendicular and the distal edge parallel to that laser separation edge. As needed, remove any elastic strands from the specimen. A cryogenic spray (e.g. Cyto-Freeze) can be used as necessary. The specimen is mounted on a metal support (e.g. a razor blade cut in half at its lateral midpoint) using double-sided adhesive, copper conductive tape. A 1.0 cm square piece of this tape is adhered to the support. The specimen is then placed on a bench and the support pressed gently onto the specimen such that the laser separation edge is parallel to, but slightly protrudes past, the edge of the support. The support with the mounted specimen is placed into a vacuum gold sputter coater in preparation for analysis.

SEM images are collected normal to the plane of the mounted specimen at a magnification of 100X. Using software that is capable of quantifying liner distances, measure and record the diameter of the nonwoven fibers and the diameter of the accumulation bulbs at the ends of those fibers, across the field of view. Measure the maximum linear length of the longest axis of the individual accumulation bulbs and individual clusters within the field of view and report the individual maximum linear lengths to the nearest .01 µm. Measure the fiber diameter to the nearest .01 µm. Count the number of accumulation bulbs over 1 cm and report as count/cm to the nearest 0.1 units. Count the number of clusters over 1 cm and report as count/cm to the nearest 0.1 units. Calculate the ratio of the maximum linear length of the longest axis (µm) of the accumulation bulb to the fiber diameter (µm) of which the accumulation bulb is attached for 5 individual accumulation bulb and attached fiber pairs and report the arithmetic mean to the nearest 0.01 units.

A total of three (3) replicate analyses should be performed on equivalent sites from three replicate products. Report as the average for the individual maximum linear lengths for each of the accumulation bulbs and clusters, the count/cm and accumulation bulb diameter/fiber diameter.

### Film Substrate Edge Distortion

Distortion Ratio is measured using light microscopy and subsequent image analysis. A benchtop stereo-microscope fitted with a CCD camera with computer interface capable of providing an image at approximately 12X magnification. Appropriate software is used to collect the digital image from the camera and make a calibrated distance measurements along an irregular or linear path.

A 2.0 cm x 2.0 cm specimen is excised from a film substrate having a laser separation edge, also referred to herein as a separation edge. The specimen is harvested such that the laser separation edge is the primary edge with the two sides perpendicular and the distal edge parallel to that laser separation edge. As needed, remove any elastic strands from the specimen so that it can lay flat. A cryogenic spray (e.g. Cyto-Freeze) can be used as necessary.

Place the specimen flat on the microscope stage. Acquire and image at 12X magnification at a resolution of about 600 x 600 pixels. A NIST traceable ruler is included within the image for calibration of the software. Trace along the edge of the laser separation edge across the field of view and calculate the length and record to the nearest 0.001 mm. This is the edge length. Next measure the linear distance between the start and end points of the traced edge and record to the nearest 0.001 mm. This is the linear length. The Distortion Ratio is calculated as the ratio of the edge length/linear length and is recorded to the nearest 0.001 units. A total of five specimens, each harvested from the equivalent position on five replicate samples are measured and the arithmetic mean is calculated and reported to the nearest 0.001 units.

## Claims

1. A method of separating a component of an absorbent article comprising:
advancing a substrate (301) in a machine direction (MD);
providing an ultra-short pulse laser source (312);
emitting a laser beam with the ultra-short pulse laser source (312);
directing the laser beam at a portion of the substrate (301);
imparting a line of weakness (218) into a portion of the substrate (301) using the laser beam, wherein the laser beam is pulsed at a frequency from 100 kHz to 100 MHz, wherein the laser beam has a pulse duration from 5 femtoseconds to 10 picoseconds, and wherein the laser beam has a level of peak energy from 20 µJ to 875 µJ;
forming a heat modified zone (270) along the line of weakness (218); and
separating the substrate (301) along the line of weakness (218) to form a separation edge (212),
wherein the heat modified zone (270) comprises a maximum width that is less than 200 microns, wherein the maximum width is measured from the separation edge (212) in a direction perpendicular to the separation edge (212) toward a central region of the substrate (301).

2. The method of claim 1, wherein the maximum width of the heat modified zone (270) is less than 100 microns.

3. The method according to any of the preceding claims, applying a machine direction tension of at least 0.5% to the substrate (301) prior to the cutting step.

4. The method according to any of the preceding claims, wherein the laser beam has a wavelength from 300 nanometers to 1080 nanometers.

5. The method according to any of the preceding claims, comprising:
rotating a process member (302) about a longitudinal axis of rotation (310);
accepting the substrate (301) on an outer circumferential surface (308) of the process member (302); and
applying a vacuum force on the substrate (301) by circulating a fluid through one or apertures defined by the outer circumferential surface (308) of the process member (302) toward the longitudinal axis of rotation (310) of the process member (302).

6. The method of claim 5, comprising advancing a discrete component (346) toward the process member (302); orienting the discrete component (346); and positioning the discrete component (346) on a portion of the substrate (301).

7. The method according to any of the preceding claims, comprising positioning a nonwoven substrate on the substrate (301) and bonding the nonwoven substrate and the substrate (301).

8. The method according to any of the preceding claims, comprising forming a component of an absorbent article (100) with the substrate (301), wherein the component comprises a belt (204), a side panel (114, 115), a topsheet (138), or a backsheet (136).

## Patentansprüche

1. Verfahren zum Trennen eines Bestandteils eines Absorptionsartikels, umfassend:
das Vorwärtsbewegen eines Substrats (301) in einer Maschinenlaufrichtung (MD);
das Bereitstellen einer Ultrakurzpulslaserquelle (312);
das Emittieren eines Laserstrahls mit der Ultrakurzpulslaserquelle (312);
das Richten des Laserstrahls auf einen Abschnitt des Substrats (301);
das Aufbringen einer Schwächungslinie (218) in einem Abschnitt des Substrats (301) unter Verwendung des Laserstrahls, wobei der Laserstrahl auf einer Frequenz von 100 kHz bis 100 MHz gepulst wird, wobei der Laserstrahl eine Pulsdauer von 5 Femtosekunden bis 10 Pikosekunden aufweist und wobei der Laserstrahl einen Spitzenenergiepegel von 20 µJ bis 875 µJ aufweist;
das Bilden eines wärmemodifizierten Bereichs (270) entlang der Schwächungslinie (218); und
das Trennen des Substrats (301) entlang der Schwächungslinie (218), um eine Trennkante (212) zu bilden,
wobei der wärmemodifizierte Bereich (270) eine maximale Breite umfasst, die geringer als 200 Mikrometer ist, wobei die maximale Breite von der Trennkante (212) in eine Richtung, die senkrecht zu der Trennkante (212) ist, zu einer mittleren Region des Substrats (301) gemessen wird.

2. Verfahren nach Anspruch 1, wobei die maximale Breite des wärmemodifizierten Bereichs (270) geringer als 100 Mikrometer ist.

3. Verfahren nach einem der vorstehenden Ansprüche, wobei eine Spannung in Maschinenrichtung von mindestens 0,5 % auf das Substrat (301) vor dem Schneideschritt angelegt wird.

4. Verfahren nach einem der vorstehenden Ansprüche, wobei der Laserstrahl eine Wellenlänge von 300 Nanometer bis 1080 Nanometer aufweist.

5. Verfahren nach einem der vorstehenden Ansprüche, umfassend:
das Drehen eines Prozesselements (302) um eine Längsdrehachse (310);
das Annehmen des Substrats (301) auf einer äußeren Umfangsoberfläche (308) des Prozesselements (302); und
das Anlegen einer Vakuumkraft auf das Substrat (301) durch Zirkulieren eines Fluids durch eines oder Öffnungen, die durch die äußere Umfangsoberfläche (308) des Prozesselements (302) definiert sind, hin zu der Längsdrehachse (310) des Prozesselements (302).

6. Verfahren nach Anspruch 5, umfassend das Vorwärtsbewegen eines separaten Bestandteils (346) zu dem Prozesselement (302); das Ausrichten des diskreten Bestandteils (346); und das Positionieren des separaten Bestandteils (346) auf einem Abschnitt des Substrats (301).

7. Verfahren nach einem der vorstehenden Ansprüche, umfassend das Positionieren eines Vliessubstrats auf dem Substrat (301) und das Verbinden des Vliessubstrats und des Substrats (301).

8. Verfahren nach einem der vorstehenden Ansprüche, umfassend das Bilden eines Bestandteils eines Absorptionsartikels (100) mit dem Substrat (301), wobei der Bestandteil ein Band (204), ein Seitenfeld (114, 115), eine Oberschicht (138) oder eine Unterschicht (136) umfasst.

## Revendications

1. Procédé de séparation d'un composant d'un article absorbant comprenant :
l'avancement d'un substrat (301) dans une direction machine (MD) ;
la fourniture d'une source de laser à impulsions ultracourtes (312) ;
l'émission d'un faisceau laser avec la source de laser à impulsions ultracourtes (312) ;
l'orientation du faisceau laser au niveau d'une partie du substrat (301) ;
la transmission d'une ligne d'affaiblissement (218) dans une partie du substrat (301) en utilisant le faisceau laser, dans lequel le faisceau laser est pulsé à une fréquence allant de 100 kHz à 100 MHz, dans lequel le faisceau laser a une durée d'impulsion allant de 5 femtosecondes à 10 picosecondes, et dans lequel le faisceau laser a un niveau d'énergie de crête allant de 20 µJ à 875 µJ ;
la formation d'une zone thermiquement modifiée (270) le long de la ligne d'affaiblissement (218) ; et
la séparation du substrat (301) le long de la ligne d'affaiblissement (218) pour former un bord de séparation (212),
dans lequel la zone thermiquement modifiée (270) comprend une largeur maximale qui est inférieure à 200 micromètres, dans lequel la largeur maximale est mesurée à partir du bord de séparation (212) dans une direction perpendiculaire au bord de séparation (212) en direction d'une région centrale du substrat (301).

2. Procédé selon la revendication 1, dans lequel la largeur maximale de la zone thermiquement modifiée (270) est inférieure à 100 micromètres.

3. Procédé selon l'une quelconque des revendications précédentes, appliquant une tension dans la direction machine d'au moins 0,5 % au substrat (301) avant l'étape de découpe.

4. Procédé selon l'une quelconque des revendications précédentes, dans lequel le faisceau laser a une longueur d'onde allant de 300 nanomètres à 1080 nanomètres.

5. Procédé selon l'une quelconque des revendications précédentes, comprenant :
la rotation d'un élément de traitement (302) autour d'un axe longitudinal de rotation (310) ;
la réception du substrat (301) sur une surface circonférentielle externe (308) de l'élément de traitement (302) ; et
l'application d'une force de vide sur le substrat (301) en faisant circuler un fluide à travers une ou des ouvertures définies par la surface circonférentielle externe (308) de l'élément de traitement (302) en direction de l'axe longitudinal de rotation (310) de l'élément de traitement (302).

6. Procédé selon la revendication 5, comprenant l'avancement d'un composant discret (346) en direction de l'élément de traitement (302) ; l'orientation du composant discret (346) ; et le positionnement du composant discret (346) sur une partie du substrat (301).

7. Procédé selon l'une quelconque des revendications précédentes, comprenant le positionnement d'un substrat non tissé sur le substrat (301) et la liaison du substrat non tissé et du substrat (301).

8. Procédé selon l'une quelconque des revendications précédentes, comprenant la formation d'un composant d'un article absorbant (100) avec le substrat (301), dans lequel le composant comprend une ceinture (204), un pan latéral (114, 115), une feuille de dessus (138) ou une feuille de fond (136).
